# EUROPEAN PATENT APPLICATION

(11) **EP 3 992 182 A1**
(43) Date of publication of application: **04.05.2022**
(21) Application number: 20306296.3
(22) Date of filing: 28.10.2020
(51) Int. Cl.: C07D 213/36, C07F 5/00, C09K 11/06, G01N 33/58

(54) **EUROPIUM(III) COMPLEXES AS PH SENSORS**

(71) Applicant: Cisbio Bioassays, 30200 Codolet (FR)
(72) Inventor: PARKER, David, Durham (GB); FRADGLEY, Jack, Nettleham (GB); STARCK, Matthieu, Soisy sur Seine (FR); ZWIER, Jurriaan, Rochefort du Gard (FR); LAMARQUE, Laurent, Sainte Victor La Coste (FR)
(74) Representative: Cabinet Beau de Loménie

(57) **Abstract**

The invention relates to a compound of formula (I): 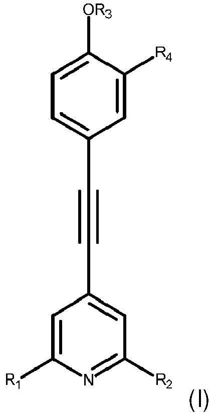 wherein R₁, R₂, R₃ and R₄ are as defined in the description.

The invention also relates to europium (III) complexes obtained from a compound of formula (I), or from a complexing agent including said compound, and to the use of such complexes to label organic or biological molecules.

## Description

### Field of the invention

The invention is based on a series of Eu(III) complexes incorporating a new chromophore based on the structure 4-O-alkyl-3-N,N-dialkyl-arylethynylpyridine whose luminescence emission is switched on by over two orders of magnitude during acidification, following excitation in the range 330 to 370 nm. Such complexes have utility in monitoring acidification in living cells or in any chemical-biological event generating pH modification.

### Background of the invention

Intracellular pH (pHi) is a key parameter to study biological phenomena. In fact, intracellular pH plays numerous essential roles in cellular, enzymatic and tissue activities, including proliferation and apoptosis, multidrug resistance, ion transport, endocytosis and muscle contraction. The study of pH changes within living cells is also crucial for studying the pathways of cell internalization, such as phagocytosis, endocytosis and ligand internalization of a given receptor. Changes in pHi also affect the nervous system, influencing synaptic transmission, neuronal excitability, cell-cell coupling *via* gap junctions and signal cascades. Abnormal pHi values are associated with inappropriate cell function, growth and division and are seen in some types of common diseases such as cancer and Alzheimer's disease. In cell biology, low intracompartmental pH can be used to denature proteins or to activate enzymatic and protein functions that would be too slow around pH 7. For example, the acidic environments of lysosomes (pH 4.5-5.5) can facilitate the degradation of proteins. Thus, cellular dysfunction is often associated with abnormal pH values in cell compartments.

The processes of internalization and endosomal uptake can therefore be followed in time, if the species being internalized (receptor or substrate) is labelled with a luminescent pH sensitive dye whose emission intensity or lifetime varies with pH. Ideally, the observed change should be as large as possible; in the limit, this is akin to the idea of a 'light switch', although this term has been frequently misused in the literature, and rather too many modest emission intensity variations have been unduly celebrated as 'switch-on' sensors (Chem. Rev. 1997, 97, 1515 and Chem. Rev. 2010, 110, 2709).

For many decades, academics and industrialists have developed pH sensors in order to measure pHi. A fluorescein derivative was introduced by Roger Tsien in 1982 to assess the cytoplasmic pH (Cell Biol. 1982, 95, 189). Several improvements have been made by chemically modifying fluorescein in order to target cellular compartments or to improve solubility in the biological media. However, these fluorescein pH sensor derivatives are subject to photobleaching due to singlet oxygen generation that may also damage the cell (Am. J. Physiol. 1989, 256, F957).

In fact, the majority of pH sensors reported in the literature involve an adaptation of the molecular structure of common fluorophores (organic dyes), such as rhodamine, BODIPY^{™} and various cyanine dyes, in order to tune the p*K*ₐ value and permit conjugation.

Indeed, pHrodo^{™} Green and -Red are commercially available from the company Thermofisher. These dyes belong to the rhodamines family and for example some researchers have made some modifications on the skeleton in order to target some biological applications. Thus, the N,N'-trifluoroethyl rhodamine derivative (RH-PEF) has been used to monitor antibody internalisation; its core structure has a p*K*ₐ around 5.1 and the intensity increase is reported to be a factor of 58 between pH 7.4 and 5.0 (Angew. Chem. Int. Ed. 2014, 53, 6085).

Goryo Chemicals provides AcidiFluor^{™} ORANGE which possesses a Rosamine skeleton. AcidiFluor^{™} ORANGE is a fluorescence imaging probe which enhances fluorescence significantly in acidic environments such as lysosomes, late endosomes and granules.

CypHer5 is now commercially available from GE (Amersham) and belongs to the class of cyanine dyes. Related studies have used fluorescent cyanine dye labels and an assay was developed to track the internalisation of the HER-2 receptor (ACS Chem. Biol. 2014, 9, 2237), while Grover has examined GPCR labelling with cyanine dyes, giving modest intensity enhancements of a factor of 5, between pH 5 and 8 (Angew. Chem. Int. Ed. 2012, 51, 4838).

Arguably the most promising approach reported so far uses a modified BODIPY^{™} core, and Nagano reported switching factors of up to 300 using these systems. Here, the p*K*ₐ is easily tuned by variation of the aniline substituents, the p*K*ₐ increases from 3.8 to 6.0 in the sequence H<Me<Et, as lone pair conjugation is increasingly disfavoured and the conjugate acid is less strongly stabilized by solvation. Such systems were used to examine breast cancer tissue samples and permitted the internalisation of the HER-2 antibody receptor to be followed by microscopy in mice, by labelling the immunotherapeutic agent Herceptin^{™} (Trastuzumab) with a BODIPY^{™} dye (Nat. Med. 2009, 15, 104).

These fluorescent organic dyes (fluoresceins, rhodamines, rosamines, cyanines and BODIPYs^{™}) offer neither significant lifetime modulation, nor a ratiometric response and have the inherent drawbacks associated with autofluorescence and photobleaching. To circumvent the photobleaching, autofluorescence and small Stokes' shifts of the organics dyes (fluoresceins, rhodamines, rosamines, BODIPYs^{™}, cyanines etc), others have therefore sought to use luminescent lanthanide labels, and benefit from their well established advantages (e.g., large Stokes' shifts, long emission lifetimes), sometimes associated with time-resolved spectroscopy or microscopy methods.

Yuan has reported monitoring the change in the red/green Eu³⁺ to Tb³⁺ emission intensity ratio in a modified acyclic chelate based on a terpyridine ligand, leading to a system with a ratiometric ratio variation of a factor of 7 (Anal. Chim. Acta 2013, 761, 149). Since the system is based on two complexes (europium and terbium), the chemical tool is difficult to use and complicates the experimental procedure. Furthermore, these complexes are not functionalized which precludes any bioconjugation reaction.

Papkovsky et al has described a rather weakly emissive Eu(III) DTPA complex based on a carbostyril sensitizer, in which the lifetime and emission intensity at 614 nm changed by a factor of 7 between pH 7.5 and 6.5 (p*K*ₐ 6.5, *λ*_{exc} 370 nm), allowing extracellular acidification to be monitored for a series of high throughput assays (Anal. Biochem. 2009, 390, 21 and US 2002/0058793). The europium complex was designed to monitor only extracellular pH variations induced by glycolysis events. Therefore, the system cannot be used for pHi measurement.

Smith has described methods that allow the real-time monitoring of lysosomal pH, using confocal microscopy, based on analysis of the Eu/Tb emission intensity ratio, that varied from 1.3 at pH 4.6 to 2.9 at pH 6.5 (*λ_{exc}* 355 nm) (Chem. Commun. 2012, 48, 8520). The system works with two different complexes (based on europium and terbium) of a common ligand complicating the system somewhat, from an industrial point of view. The system behaves in an inverse manner compared to the existing organic dye pHi sensors, since the fluorescence emission is higher at neutral pH and decreases when the pH decreases. From a microscopy point of view, this is a significant drawback.

Using kinetically stable macrocyclic ligand systems based on triazacyclononane, McMahon (Chem. Commun. 2013, 49, 5363) devised Eu(III) complexes that permitted monitoring of the pH of the endoplasmic reticulum in live cells, based on emission lifetime variation; a 75% change in the europium lifetime was observed over the pH range 6.5 to 7.5. The authors improved the brightness of the complexes compared to the one described by Smith *et al.* However, the system does not allow for any changes of the chelating moieties around the europium centre since one of the pendant arm (sulfonamide moiety) is mandatory in the pH sensor. Also, the complex is not designed for bioconjugation. Finally, the europium complex emission occurs when pH increases from pH 4 to 8 which makes the system not well suited to microscopy purposes.

Very recently, Patra *et al* developed a luminescent pH-sensitive, lysosome targeting, europium probe (New J. Chem. 2020, 44, 3570). The absorption wavelength is 445 nm which is not compatible with the majority of the excitation sources commercially available (flash lamp excitation is between 300 and 360 nm or laser at 337 nm). Furthermore the complex is still emissive at pH 7 which is an undesirable property for biological and microscopy applications. Again this lanthanide system does not permit the conjugation of the emissive probe to a targeting vector or to a protein.

Takalo has studied the photophysical properties of arylethynyl pyridines complexes (Helv. Chim. Acta 1993, 76, 877), and Latva et al. gathered results in one article (J. Lumin. 1997-75-149) discussing europium chelate properties. Neither article mentions that the fluorescence of such systems was pH sensitive.

There is therefore a strong need for a lanthanide based fluorescent pH sensitive probe which is compatible with biological media allowing biological events involving pH variation to be monitored. Excitation of the probes should be possible between 320 and 365 nm, wavelengths which are widely used in plate readers (flash lamp and laser).

Therefore, the inventors set out to create a new family of compounds that display a 100% change in excited state lifetime over the pH range 8 to 4, and a change in the long-lived emission intensity of two orders of magnitude. These desirable properties have been thus far shown impossible to obtain. It has been found that in order to obtain these desirable properties over the pH range 8 to 4, the p*K*ₐ of the emissive species undergoing protonation needs to be between 4.5 and 6.5. Achieving this result was made possible, according to the invention, by designing a chromophore consisting of an arylethynylpyridinyl moiety where the aryl is disubstituted, wherein one of the substituents is a 4-O-alkyl group and the second substituent is a 3-N,N-dialkylamine moiety (e.g. a 3-N,N-dialkylaniline). Such a chromophore is capable of forming kinetically stable complexes with europium(III), when incorporated into macrocycles such as triazacyclononane (TACN), 1,4,7,10-tetraazacyclododecane-1,4,7,10-tetraacetic acid (DOTA) or related chelate systems.

### Summary of the invention

The present invention relates to a compound of formula (I): 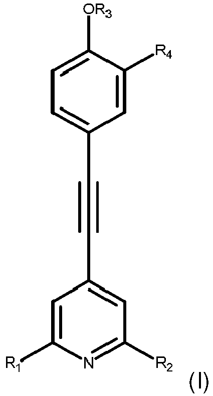 wherein R₁, R₂, R₃ and R₄ are as defined in the detailed description which follows.

The invention also relates to a complexing agent of formula (II) or (III): 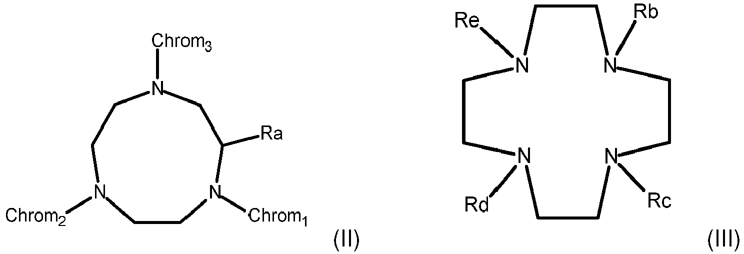 wherein Rₐ, R_{b}, R_{c}, R_{d}, Rₑ, Chrom₁, Chrom₂ and Chrom₃ are as defined in the detailed description which follows.

The present invention also relates to an europium complex comprising a compound of formula (I), or a complexing agent of formula (II) or (III), and an europium(III) ion.

The present invention further relates to a conjugate obtained by reaction between an europium complex and a molecule of interest.

### Brief description of the figures

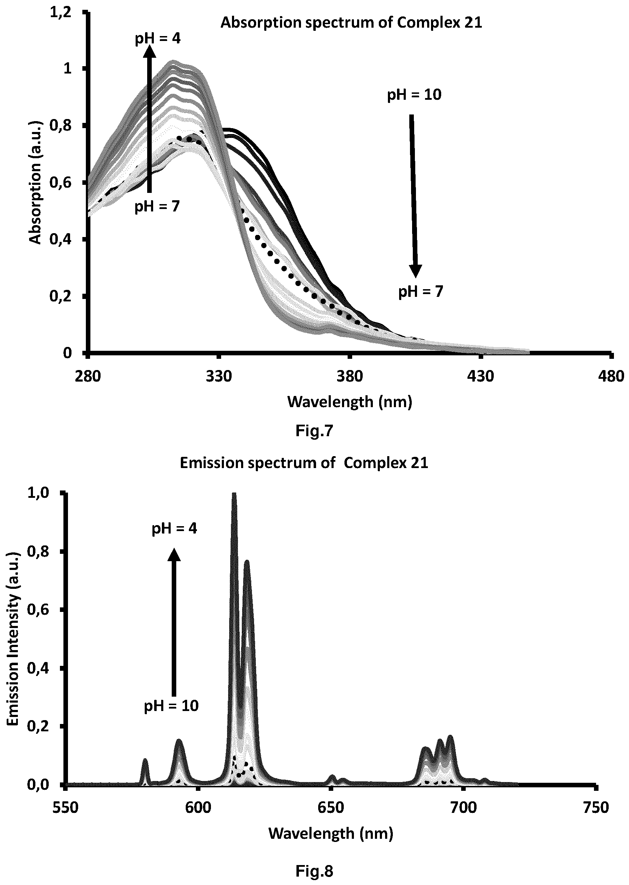

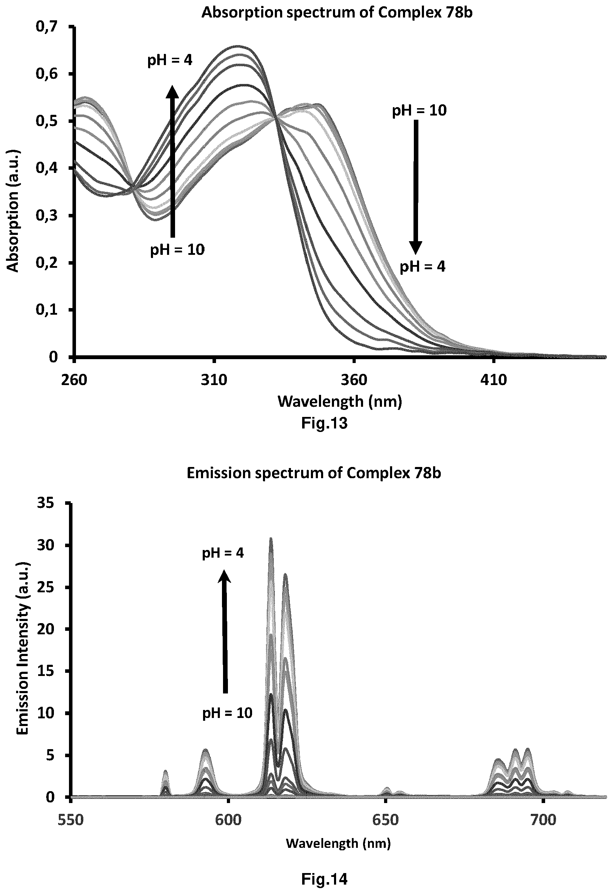

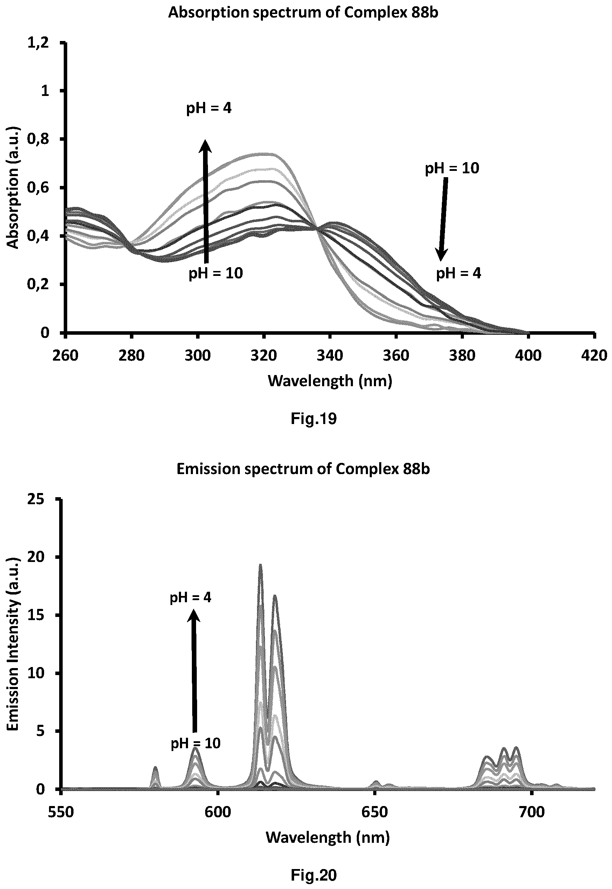

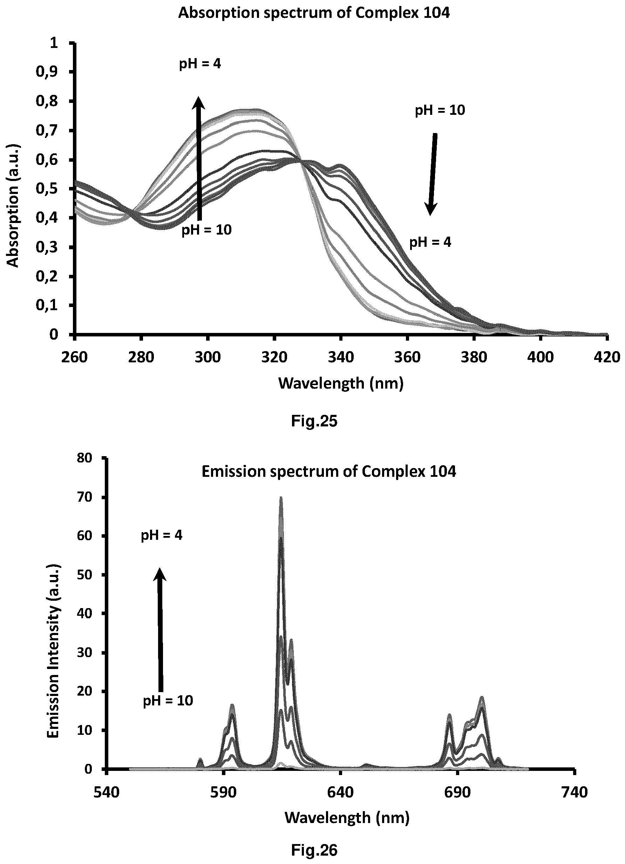

Figures 1, 4, 7, 10, 13, 16, 19, 22 and 25 describe the absorption spectra from pH 4 to 10 for complexes **14**, **18, 21, 78a, 78b, 88a, 88b; 93** and **104.**
Figures 2, 5, 8, 11, 14, 17, 20, 23 and 26 describe the emission spectra from pH 4 to 10 for complexes **14**, **18, 21, 78a, 78b, 88a, 88b; 93** and **104.**
Figures 3, 6, 9, 12, 15, 18, 21, 24 and 27 describe the emission lifetime variation versus pH allowing the determination of the p*K*ₐ value for complexes **14, 18, 21, 78a, 78b, 88a, 88b; 93** and **104.**
Figure 28 (top) shows the emission intensity of Complex **18** measured at various pH values (295 K) with different time delays (red = 60 µs, orange = 460 µs, purple = 1000 µs). Data was normalised for a 60 µs delay at pH 4.

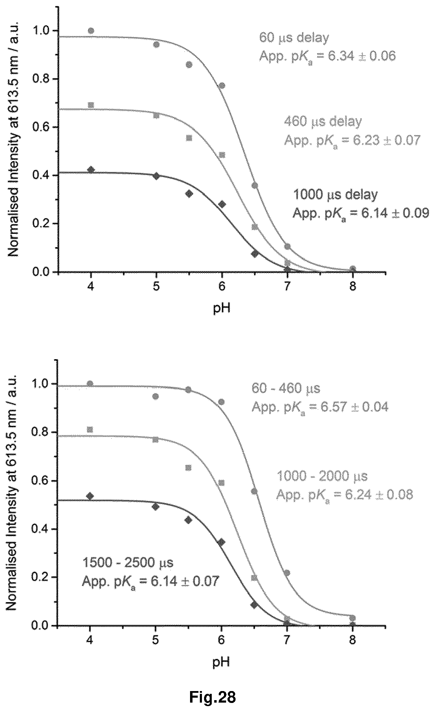

Figure 28 (bottom) shows the emission intensity of Complex 18 measured at various pH values with different time windows (red = 60 - 460 µs, orange = 1000 - 2000 µs, purple = 1500 - 2500 µs). Data were normalised for a 60 - 460 µs time window at pH 4. Measurements were taken in NH₄CH₃CO₂ (pH 4 and 5), MES (pH 5.5, 6 and 6.5), HEPES (pH 7) and NH₄HCO₃ (pH 8) buffers (all 0.1 M with 0.1 M NaCl).

### Detailed description of the invention

In one aspect, the present invention relates to a compound of formula (I): 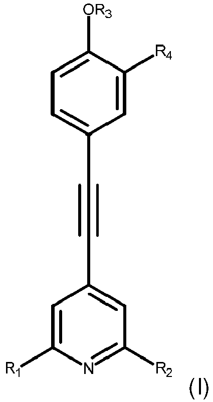 wherein:
R₁ is -CO₂H, -PO(OH)R₅ or -CH₂N(CH₂CO₂H)₂;
R₂ is -CH₂OH, -CH₂OSO₂CH₃, Br, Cl or -CH₂N(CH₂CO₂H)₂;
R₃ is a (C₁-C₆)alkyl optionally substituted with a group -L₁-E or with a group G;
R₄ is -(CH₂)ₘ-NR₆R₇;
R₅ is a (C₁-C₄)alkyl, preferably a methyl; a phenyl optionally substituted with a group -SO₃⁻, the latter preferably being in the meta or para position; or a benzyl;
R₆ is H or a (C₁-C₄)alkyl;
R₇ is a (C₁-C₆)alkyl optionally substituted with a group -L₁-E;
or R₆ and R₇, together with the nitrogen atom to which they are bonded, form a piperidine, a morpholine, or a piperazine optionally N-protected by a *tert*-butoxycarbonyl group or optionally N-substituted with R₈;
R₈ is a (C₁-C₆)alkyl optionally substituted with a group -L₁-E;
L₁ is a direct bond, -CONH-(CH₂)ₙ- or -NHCO-(CH₂)ₙ-;
E is -SO₃H, -SO₂(OCH₂CF₃), -N⁺Alk₁Alk₂Alk₃, a carbohydrate residue, or a sulfo-betaine;
G is a carboxyl group optionally protected in the form of an ester (for example a group -CO₂Me or -CO₂^{t}Bu), an amino group optionally protected by a *tert*-butoxycarbonyl group, a succinimidyl ester, a haloacetamido group, a hydrazine group, an isothiocyanato group, or a maleimido group;
Alk₁, Alk₂, and Alk₃ each independently represent a (C₁-C₆)alkyl, preferably a (C₁-C₄)alkyl; m is 0, 1, 2 or 3;
n is 0, 1, 2 or 3.

In the context of the present disclosure, a carbohydrate residue is understood to mean a glucose residue in cyclic or linear form, or else a group of formula -(CHOH)ₖ-CH₂OH where k is an integer from 3 to 12, preferably k is 3 or 4.

In the context of the present disclosure, a sulfo-betaine is understood to mean a group having one of the following formulae: 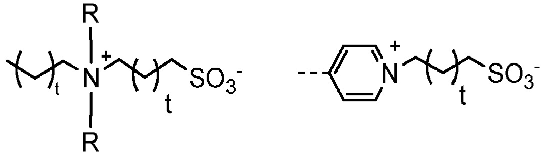 where R represents a (C₁-C₆)alkyl, preferably methyl or ethyl, and t is an integer from 1 to 6, preferably t is 1 or 2. In one embodiment the sulfo-betaine is a group of formula -(CH₂)₂N⁺(CH₃)₂-(CH₂)₃-SO₃⁻.

In the context of the present disclosure, the -SO₃H, -CO₂H and -PO(OH)₂ groups are in deprotonated form or not depending on the pH. These groups therefore also refer in the following description and appended claims to the groups -SO₃⁻, -CO₂⁻ and -PO(OH)O⁻, respectively, and vice versa.

In the context of the present disclosure, the embodiments described herein can be combined.

In one embodiment, R₁ is -CO₂H or -PO(OH)R₅ where R₅ is a (C₁-C₄)alkyl, preferably a methyl, and R₂ is -CH₂OH or -CH₂OSO₂CH₃. In another embodiment, R₁ and R₂ are each -CH₂N(CH₂COOH)₂.

In one embodiment, R₃ is a (C₁-C₄)alkyl optionally substituted with a group -L₁-E or with a group G.

In one embodiment, R₄ is -NR₆R₇ where R₆ and R₇ are each independently a (C₁-C₄)alkyl, or R₆ and R₇, together with the nitrogen atom to which they are bonded, form a piperazine optionally N-protected by a *tert*-butoxycarbonyl group or optionally N-substituted with a group R₈.

In one embodiment, R₈ is a (C₁-C₄)alkyl optionally substituted with a group -L₁-E.

In one embodiment, L₁ is a direct bond or -CONH-(CH₂)ₙ-.

In one embodiment, E is -SO₃H or -SO₂(OCH₂CF₃).

In one embodiment, G is a carboxyl group optionally protected in the form of an ester (for example a group -CO₂Me or -CO₂^{t}Bu) or an amino group optionally protected by a *tert-*butoxycarbonyl group.

In one embodiment, m is 0. In another embodiment m is 1. In yet another embodiment m is 2. In yet another embodiment m is 3.

In one embodiment, n is 0. In another embodiment n is 1. In yet another embodiment n is 2. In yet another embodiment n is 3.

In another aspect, the invention relates to a complexing agent of formula (II) or (III): 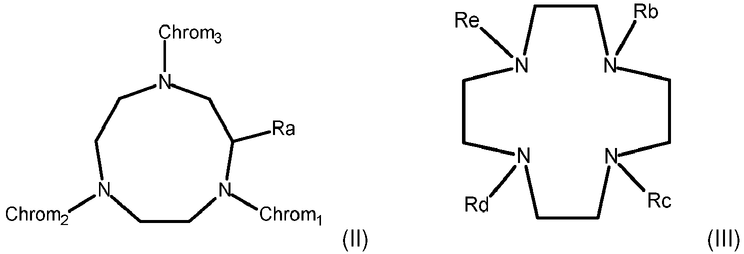 wherein:
Rₐ is absent or is -CH₂NH₂;
one (and only one) of R_{b}, R_{c}, R_{d} and Rₑ is a group of formula (IV) and the others are each independently selected from -CH₂COOR_{f} and -CH₂PO(OH)R_{g};
R_{f} is H, (C₁-C₄)alkyl, -NHCH(Rₕ)-(C₁-C₄)alkyl or -NHCH(Rₕ)-C(O)ORⱼ;
R_{g} is (C₁-C₄)alkyl;
Rₕ is (C₁-C₄)alkyl or phenyl;
Rⱼ is H or (C₁-C₄)alkyl;

Chrom₁, Chrom₂ and Chrom₃ are each independently selected from a group of formula (IV) and a group of formula (V): 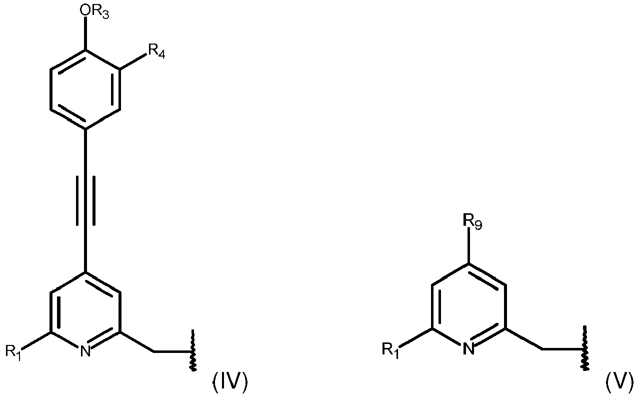 wherein:
each R₁ is -CO₂H or -PO(OH)R₅;
R₃ is a (C₁-C₆)alkyl optionally substituted with a group -L₁-E or with a group G;
R₄ is -(CH₂)ₘ-NR₆R₇;
R₅ is a (C₁-C₄)alkyl, preferably a methyl; a phenyl optionally substituted with a group -SO₃⁻, the latter preferably being in the meta or para position; or a benzyl;
R₆ is H or a (C₁-C₄)alkyl;
R₇ is a (C₁-C₆)alkyl optionally substituted with a group -L₁-E;
or R₆ and R₇, together with the nitrogen atom to which they are bonded, form a piperidine, a morpholine, or a piperazine optionally N-protected by a *tert*-butoxycarbonyl group or optionally N-substituted with a group R₈;
R₈ is a (C₁-C₆)alkyl optionally substituted with a group -L₁-E;
R₉ is a (C₁-C₆)alkyl optionally substituted with a group -L₁-E or with a group G;
L₁ is a direct bond, -CONH-(CH₂)ₙ- or -NHCO-(CH₂)ₙ;
E is -SO₃H, -SO₂(OCH₂CF₃), -N⁺Alk₁Alk₂Alk₃, a carbohydrate residue, or a sulfo-betaine;
G is a carboxyl group optionally protected in the form of an ester (for example a group -CO₂Me or -CO₂^{t}Bu), an amino group optionally protected by a *tert*-butoxycarbonyl group, a succinimidyl ester, a haloacetamido group, a hydrazine group, an isothiocyanato group, or a maleimido group;
Alk₁, Alk₂, and Alk₃ each independently represent a (C₁-C₆)alkyl, preferably a (C₁-C₄)alkyl; m is 0, 1, 2 or 3;
n is 0, 1, 2 or 3;
provided that the compound of formula (II) comprises at least one group of formula (IV).

A preferred family of complexing agents include the compounds of formula (II). Another preferred family of compounds include the compounds of formula (III).

In one embodiment, the complexing agent of formula (II) comprises only one group of formula (IV), i.e. one of Chrom₁, Chrom₂ and Chrom₃ is a group of formula (IV) and the other two are a group of formula (V) and may be identical or different, preferably identical.

In one embodiment, the complexing agent of formula (II) comprises two groups of formula (IV), i.e. two of Chrom₁, Chrom₂ and Chrom₃ are a group of formula (IV) and may be identical or different, preferably identical, and the other one is a group of formula (V).

In one embodiment, the complexing agent of formula (II) comprises three groups of formula (IV), i.e. Chrom₁, Chrom₂ and Chrom₃ are each a group of formula (IV) and may be identical or different, preferably identical.

In one embodiment, each R₁ is -PO(OH)R₅ where R₅ is a (C₁-C₄)alkyl, preferably a methyl. In one embodiment, R₃ is a (C₁-C₄)alkyl optionally substituted with a group -L₁-E or with a group G.

In one embodiment, R₄ is -NR₆R₇ where R₆ and R₇ are each independently a (C₁-C₄)alkyl, or R₆ and R₇, together with the nitrogen atom to which they are bonded, form a piperazine optionally N-protected by a *tert*-butoxycarbonyl group or optionally N-substituted with a group R₈.

In one embodiment, R₈ is a (C₁-C₄)alkyl optionally substituted with a group -L₁-E.

In one embodiment, L₁ is a direct bond or -CONH-(CH₂)ₙ-.

In one embodiment, E is -SO₃H or -SO₂(OCH₂CF₃).

In one embodiement, G is a carboxyl group optionally protected in the form of an ester (for example a group -CO₂Me or -CO₂^{t}Bu) or an amino group optionally protected by a *tert-*butoxycarbonyl group.

In one embodiment, m is 0. In another embodiment m is 1; in yet another embodiment m is 2. In yet another embodiment m is 3.

In one embodiment, n is 0. In another embodiment n is 1; in yet another embodiment n is 2. In yet another embodiment n is 3.

In another aspect, the present invention relates to a europium complex comprising a europium(III) ion (Eu³⁺) and a compound of formula (I) where R₁ and R₂ are each -CH₂N(CH₂COOH)₂.

In another aspect, the present invention relates to a europium complex comprising a europium(III) ion and a compound of formula (II) or (III) as disclosed herein.

The europium complexes of the invention can be prepared by bringing into contact the compounds of formula (I), (II) or (III) as disclosed herein to the invention with a europium salt. Typically, reacting one equivalent of compound with 1 to 5 equivalents of europium salt (e.g. chloride, acetate or triflate) in a solvent (acetonitrile, methanol or other solvent compatible with these salts) or a buffer in aqueous solution, at room temperature for a few minutes, leads to the corresponding complex.

A representative europium complex of the invention has the following structure:

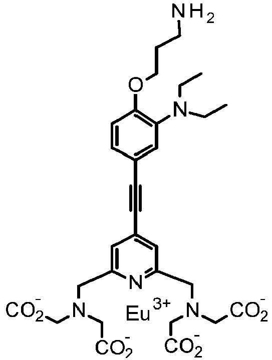

Another representative europium complex of the invention has the following structure: 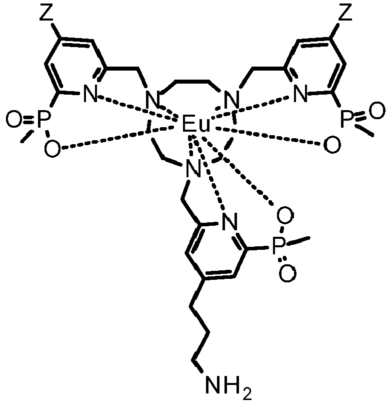 where Z is:

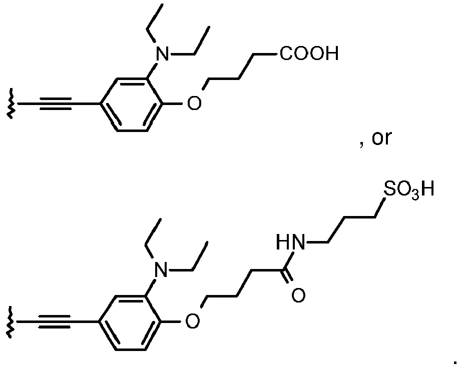

The compounds of formula (I), (II) and (III) disclosed herein (and their corresponding europium complexes) which comprise a group G are particularly suitable for labelling organic or biological molecules comprising a functional group capable of reacting with the reactive group to form a covalent bond.

Thus, in one aspect, the invention relates to conjugates obtained by reaction between (i) a europium complex as disclosed herein, said complex bearing a group G, and (ii) a molecule of interest. Any organic or biological molecule of interest can be conjugated with a europium complex as disclosed herein as long as it bears a functional group capable of reacting with the group G borne by the complex.

In one embodiment, the molecule of interest is selected from: an amino acid, a peptide, a protein, an antibody, a sugar, a carbohydrate chain, a nucleoside, a nucleotide (DNA, RNA), an oligonucleotide, and an enzyme substrate, notably a suicide enzyme substrate such as a benzylguanine or a benzylcytosine (enzyme substrates marketed under the names Snaptag and Cliptag), a chloroalkane (enzyme substrate marketed under the name Halotag), or coenzyme A (enzyme substrate marketed under the name ACPtag or MCPtag).

The europium complexes of the invention display a p*K*ₐ value, when undergoing protonation, in the range 4.5 to 6.5. Usually, when a molecule possesses several protonation sites, each protonation site displays a p*K*ₐ value. It has unexpectedly been found that the complexes of the invention display only one observable p*K*ₐ value regardless the number of chromophores (i.e., compounds of formula (I), (IV) or (V)) fixed on the complex).

The europium complexes of the invention can also be bio-conjugated to a molecule of interest on a site which does not perturb the fluorescence intensity upon pH variation of the medium. The europium complexes of the invention have an excellent emission brightness which is very desirable to detect biological events at low concentrations.

As mentioned above, the europium complexes of the invention can comprise 1 to 3 chromophores and can be functionalized to allow the introduction of water-soluble moieties and functional groups for bioconjugation. According to the desired complex, one can choose the corresponding synthetic pathway (schemes 1-3). For a complex bearing 1 chromophore, 2 chromophores or 3 non identical chromophores, two options are possible which are depicted in schemes 1-2 and exemplified in further detail in the experimental section. For a complex bearing 3 identical chromophores, the synthesis is depicted in scheme 3.

For example, triazacyclononane is alkylated either with the chromophores or with the pyridine moieties, according to the desired number of chromophores required on the complex. After deprotection of the Boc group, the last chromophore or pyridine moiety is introduced on the di-chromophore or di-pyridinyl alkylated macrocycle. The phosphinate ethyl ester functionalities are hydrolysed and the europium complex is formed through addition of europium(III) chloride. Optionally and if appropriate, complexes can be functionalized with sulfonate moieties allowing a better water-solubility and with a functional group for conjugation with proteins, antibodies, peptides and enzymatic substrates.

### General synthetic scheme for complexes with 1, 2 and 3 chromophores (option 1)

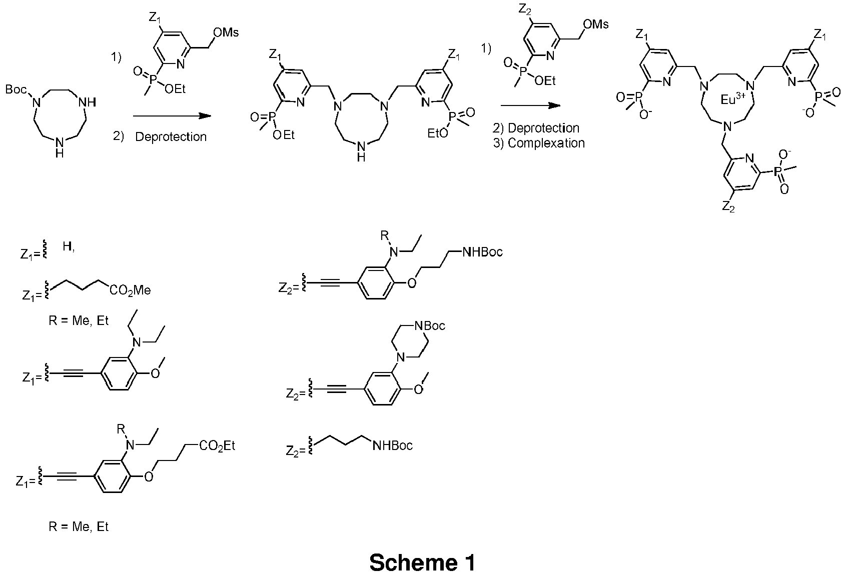

### General synthetic scheme for complexes with 1 & 2 chromophores (option 2)

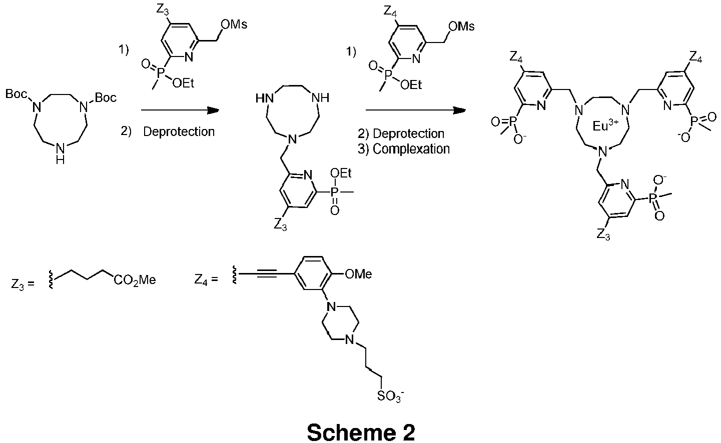

### General synthetic scheme for complexes with 3 identical chromophores

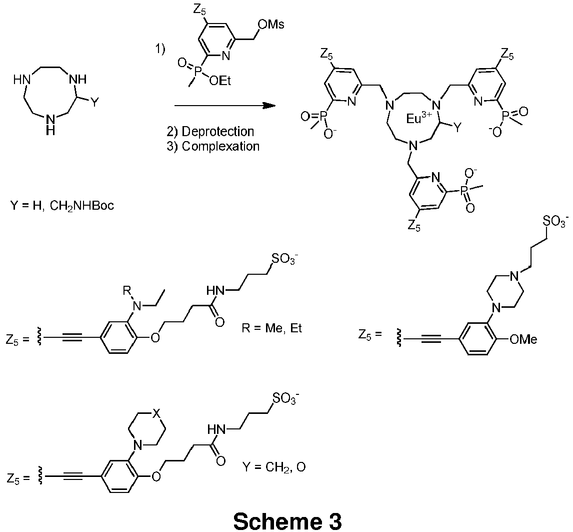

### General synthesis of acyclic complex

The skeleton synthesis has been described previously (Helvetica Chimica Acta, 1993, 76, 877). The synthesis is supplemented with the introduction of an N,N-dialkyl moiety in the meta position of the triple bond and with an O-alkyl group in the para position. An example is given in scheme 4.

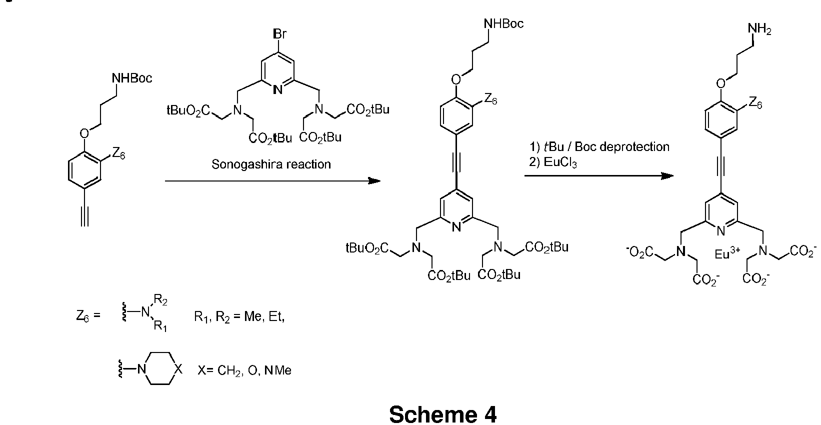

### General synthesis of complex based on DOTA macrocycle

To emphasize the general features of the invention, the chromophore has been introduced on the DOTA system using the classical synthetic strategy described elsewhere (WO 2006/120444, WO 2009/010580, WO 2010/084090, Acc. Chem. Res 2009, 42, 925). Scheme 5 depicts the strategy used leading to the corresponding europium complex decorated with one chromophore.

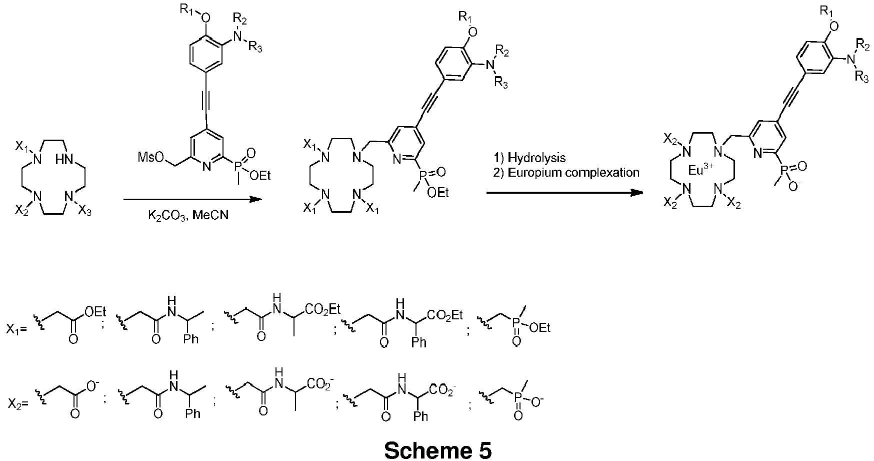

Other routes for preparing the complexing agents disclosed herein and the corresponding europium complexes are described schematically below and are further illustrated in the examples.

### Non-functionalized chromophore synthesis

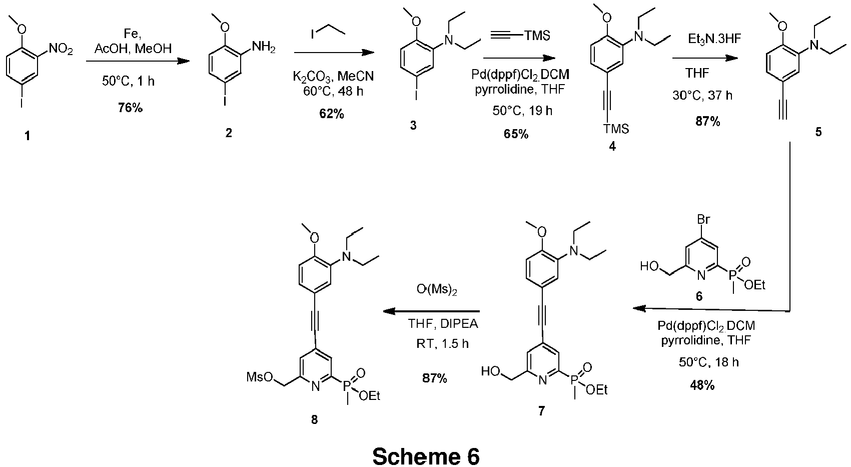

Compound 1, which is commercially available, was reduced into the corresponding aniline which was alkylated in the presence of iodoethane for 48 h. The chromophore skeleton was obtained by using two Sonogashira reactions leading to compound **7.** The resulting alcohol was converted into the corresponding mesylate **8** and used for the subsequent macrocycle alkylation.

### Non-functionalized complex synthesis with 1 chromophore

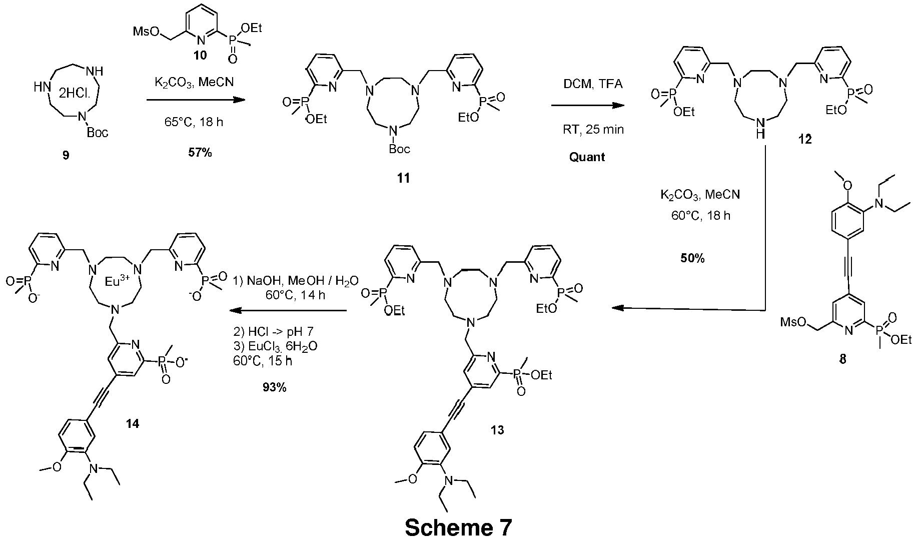

The synthesis of the europium complex with one chromophore is depicted in scheme 7. Triazacyclonane **9** was alkylated with the mesylated pyridine **10.** The third nitrogen atom was deprotected with TFA and alkylated with the chromophore **8.** The phosphinate ethyl esters were hydrolysed and the macrocycle was complexed with europium to give the europium(III) complex **14.**

### Non-functionalized complex synthesis with 2 chromophores

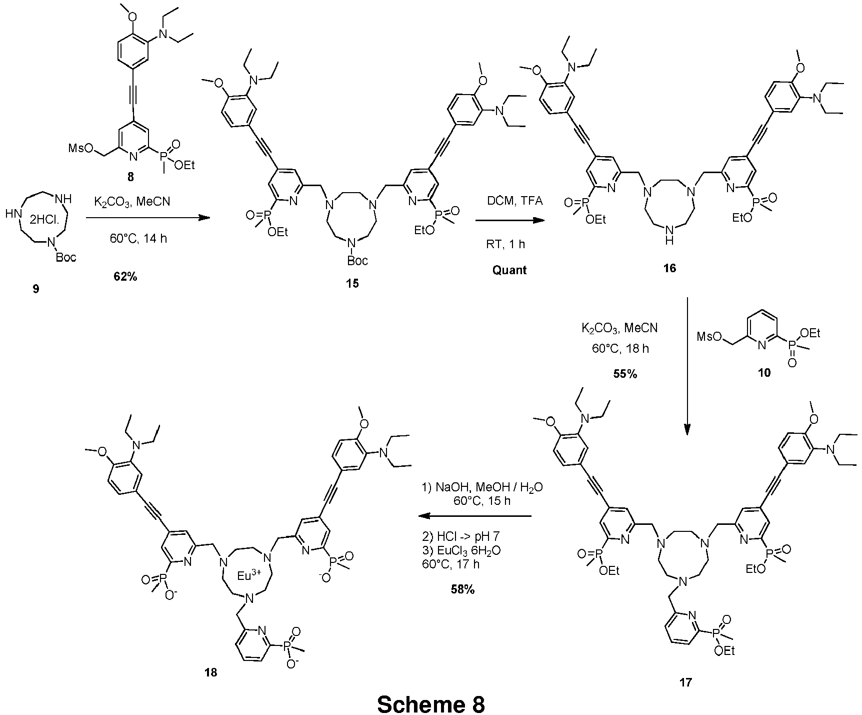

To obtain a complex with two chromophores, such as europium complex **18**, the strategy applied was identical to the previous one, but reversing the order of introducing the pyridine derivatives (compound **8** and **10**), as displayed in scheme **8**.

### Non-functionalized complex synthesis with 3 chromophores

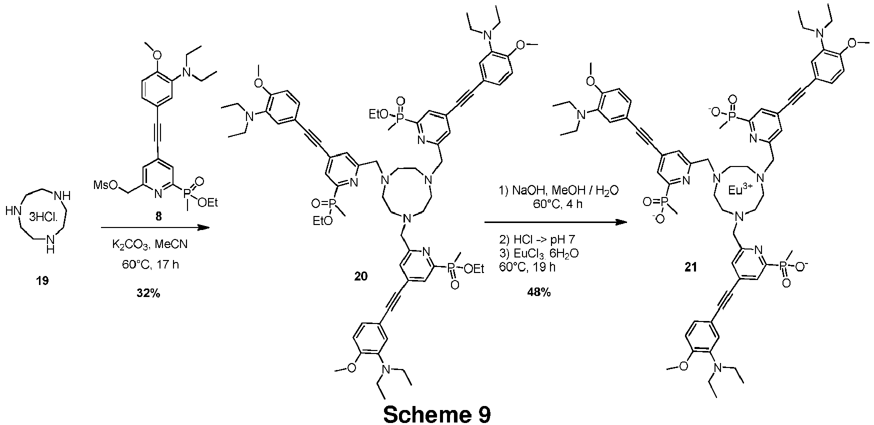

The complex bearing 3 chromophores was synthesized using the unprotected triazacyclononane **19** and chromophore **8** leading to the corresponding europium complex **21**.

### Ethyl ester functionalized chromophore synthesis

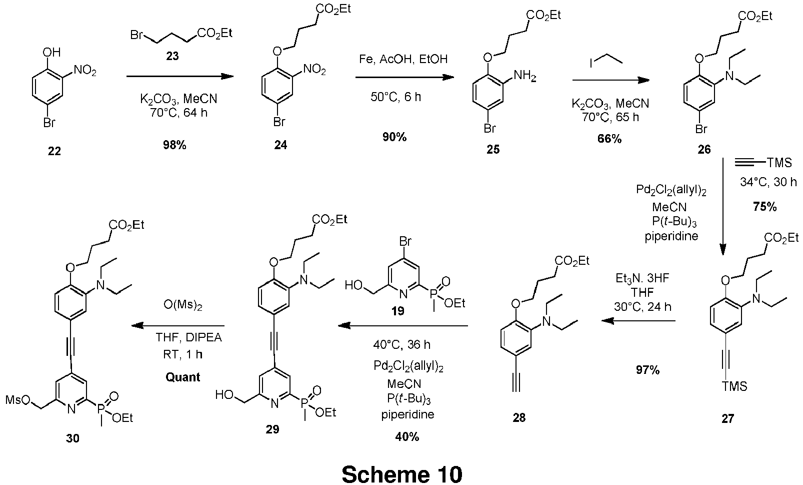

To functionalize the chromophore **8,** the methyl group of the methoxy group was replaced with an aliphatic chain bearing an ester moiety in order to later introduce a sulfonate or other hydrophilic functions. Thus, bromonitrophenol **22** was alkylated and the nitro group subsequently reduced before being alkylated with iodoethane. Two Sonogashira reactions allowed the chromophore to be obtained which was activated as the mesylate derivative **30.**

### NHBoc functionalized chromophore synthesis

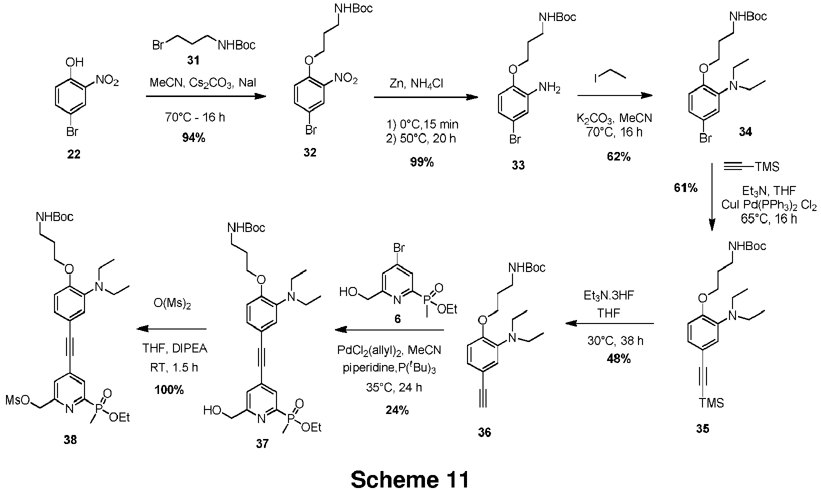

Instead of being functionalized with a carboxylic acid protected in the form of an ethyl ester, the chromophore was functionalized with a protected amine, in the form of a classical carbamate (Boc group). The synthetic pathway was the same as the previous one using N-Boc protected bromopropyl amine (Scheme 11).

### Ethyl ester functionalized chromophore synthesis

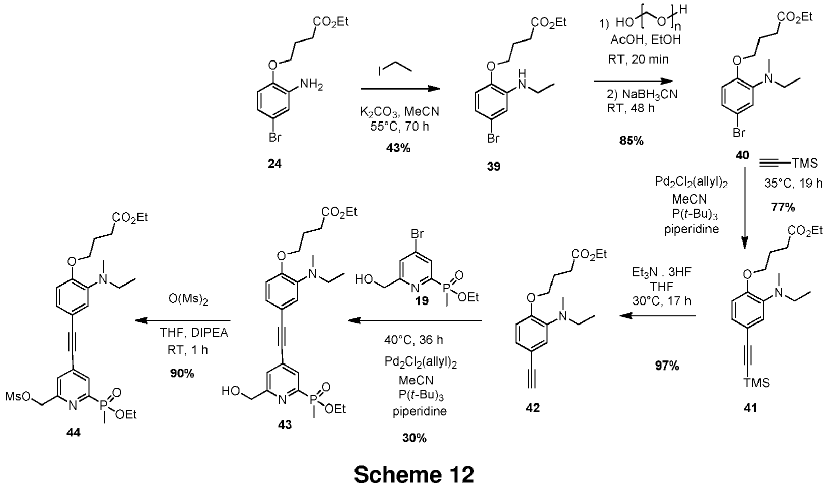

Alkylation of the aniline was controlled by selecting the molar ratio of alkylating reagent and aniline derivative. Thus, compound **36** was mono-alkylated with iodoethane. The resulting secondary aniline was methylated using reductive amination. The final steps of the synthesis were identical to those described above.

### Synthesis of a cyclic amine system on the chromophore

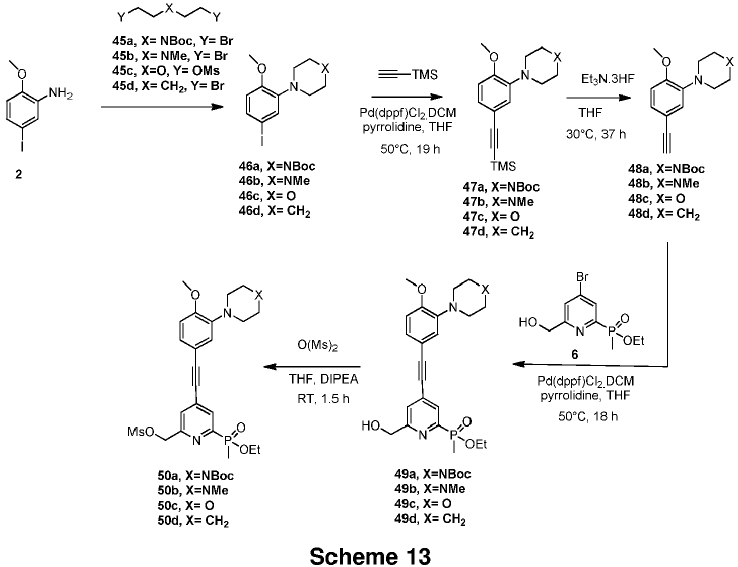

The aniline derivative was converted to a cyclic amine (piperidine, piperazine, morpholine). In the case of compounds **46a-d,** the secondary amine is useful to either attach a soluble moiety or can serve as a bioconjugation point.

### Synthesis of N,N-alkylpropylsulfonate precursor chromophore

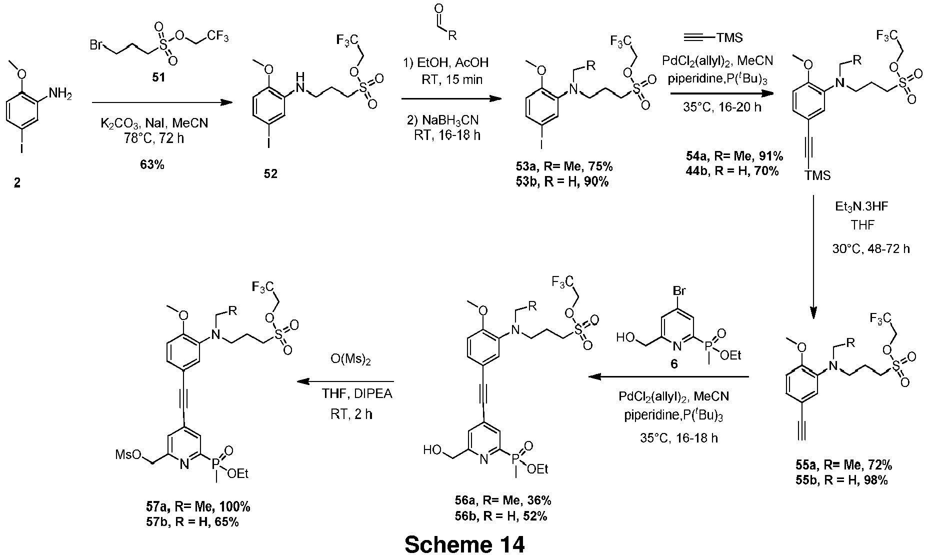

The aniline was substituted on one side with an alkyl chain bearing a terminal protected sulfonate and on the other side with an alkyl chain. Thus, the corresponding chromophore was made using the same methodology used in the previous schemes but employing the corresponding bromopropyl sulfonate substituted by a trifluoroethyl moiety as a protective group. The synthetic scheme is depicted in scheme 14.

### NHBoc functionalised pyridine synthesis

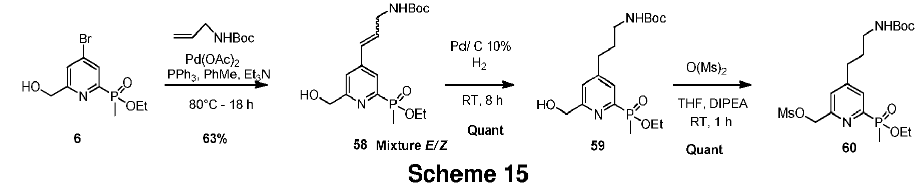

### Methyl ester functionalized pyridine synthesis

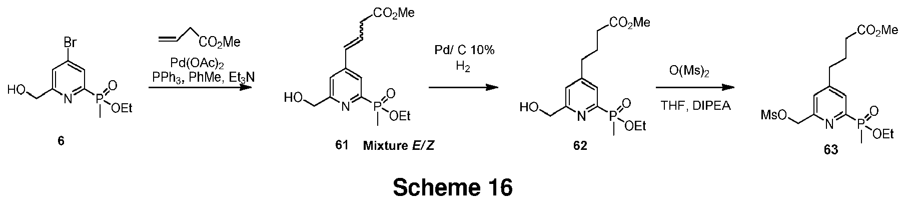

Compounds 60 and 63 were synthesized from pyridine 6. The first C-C bond forming reaction was carried out using a Heck reaction. The resulting double bound was hydrogenated in the classical manner. The alcohol function was activated to lead to the corresponding mesylate derivatives, **60** and **63** (schemes 15, 16).

### Synthesis of functionalized complex with one chromophore

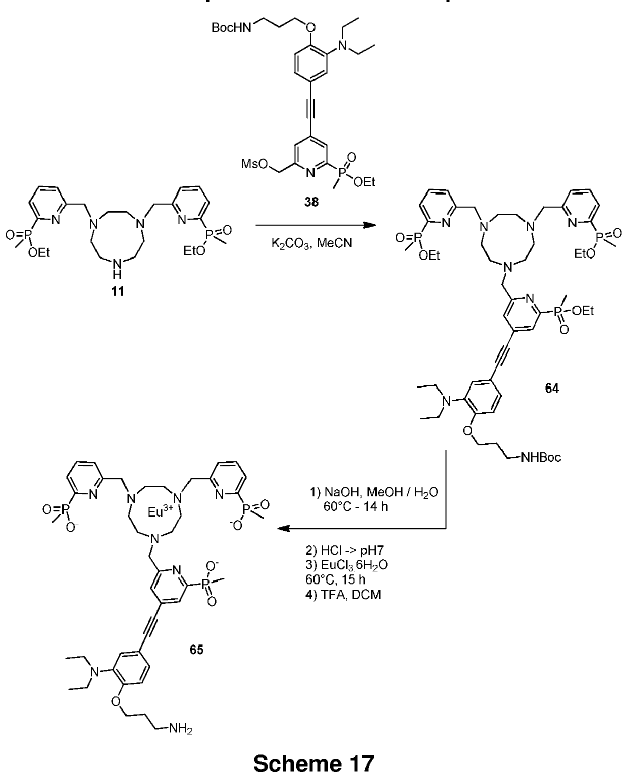

Scheme 17 describes the synthesis of complex **65** incorporating 1 chromophore.

### Synthesis of functionalized complex with one chromophore

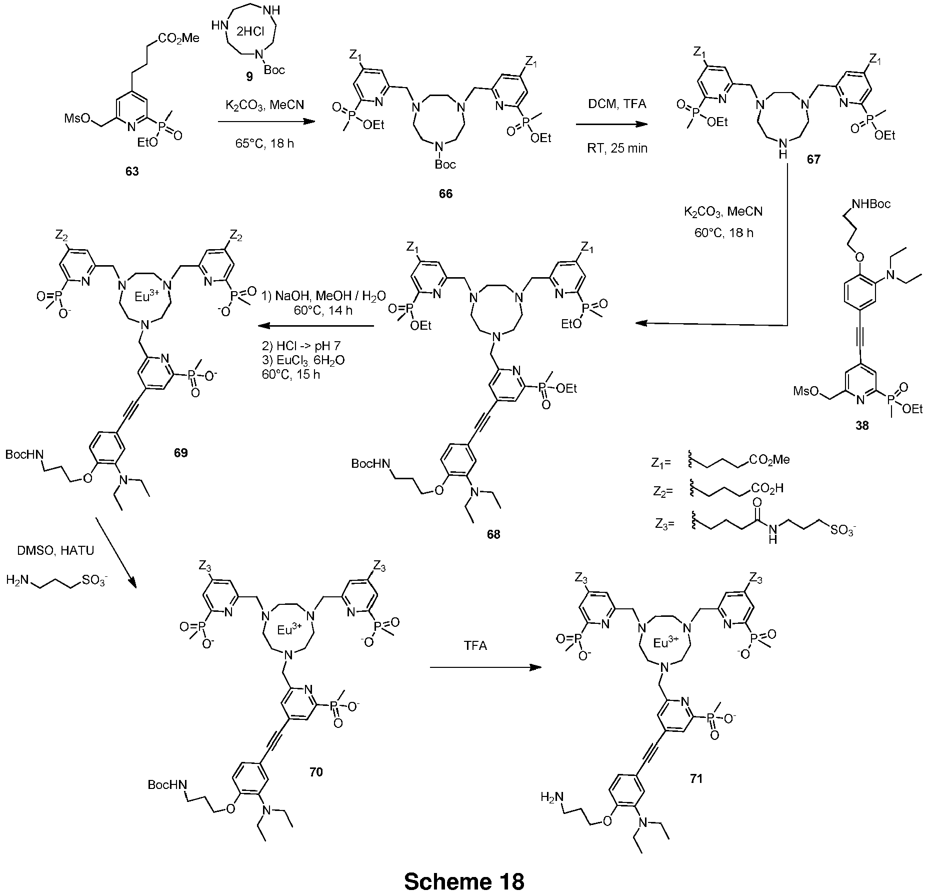

The synthesis of complex **71** is depicted in scheme 18. The sulfonate moieties were introduced on the pyridine heterocycles at the end of the synthesis.

### Synthesis of functionalized complex with one chromophore

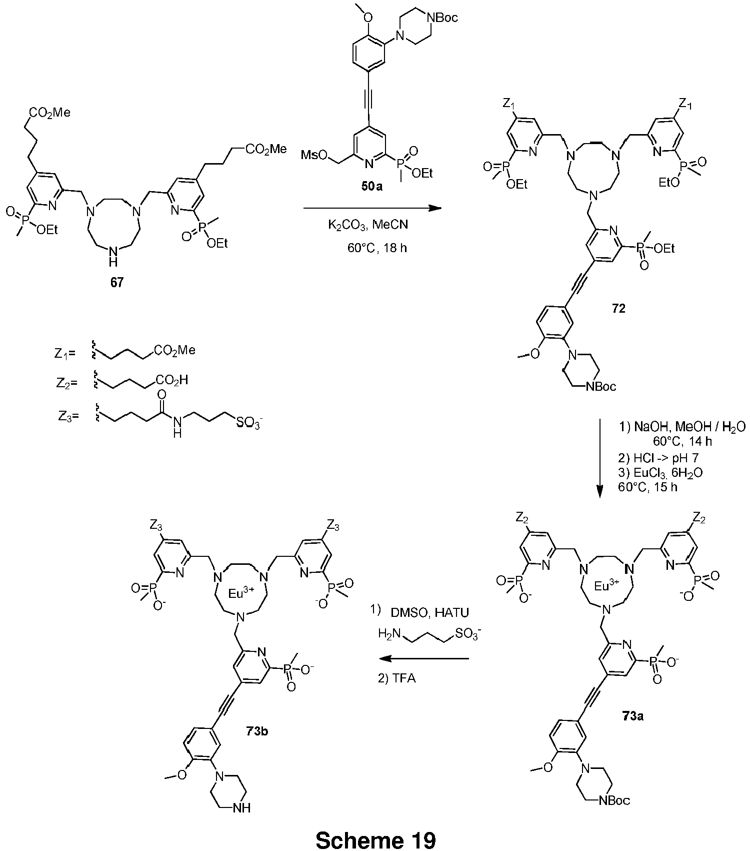

Scheme 19 illustrates the synthesis of a complex which can be functionalized through the piperazinyl moiety on the secondary amine (compound **73b**).

### Synthesis of functionalized complex with two chromophores

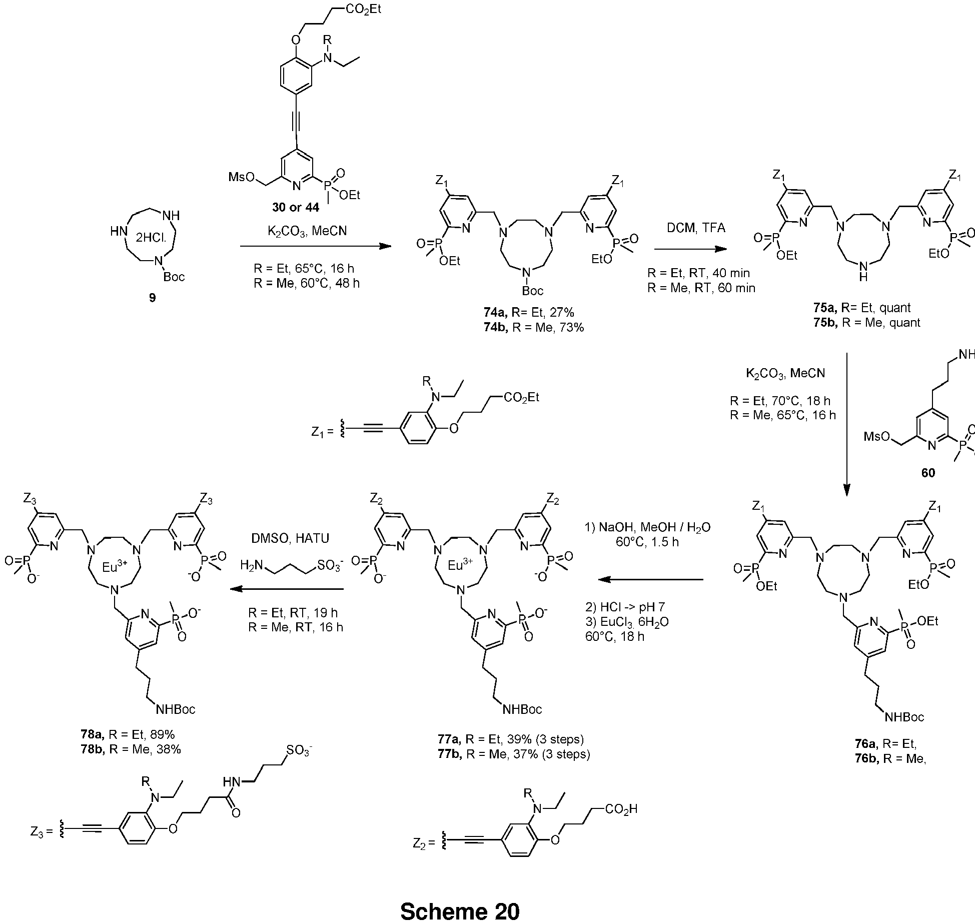

Scheme 20 describes the synthesis of two complexes bearing 2 chromophores. The p*K*ₐ of each complex was tuned by the nature of the alkyl group substituting the nitrogen atom. The synthesis started with the alkylation of the triazacyclononane with the mesylate chromophore. Deprotection of the Boc group followed by the third alkylation led to compounds **76a-b.** Hydrolysis of the phosphinate and europium complexation gave the complexes **77a-b** which were treated with homotaurine to lead to the desired water-soluble europium (III) complex.

### Synthesis of functionalized complex with two chromophores

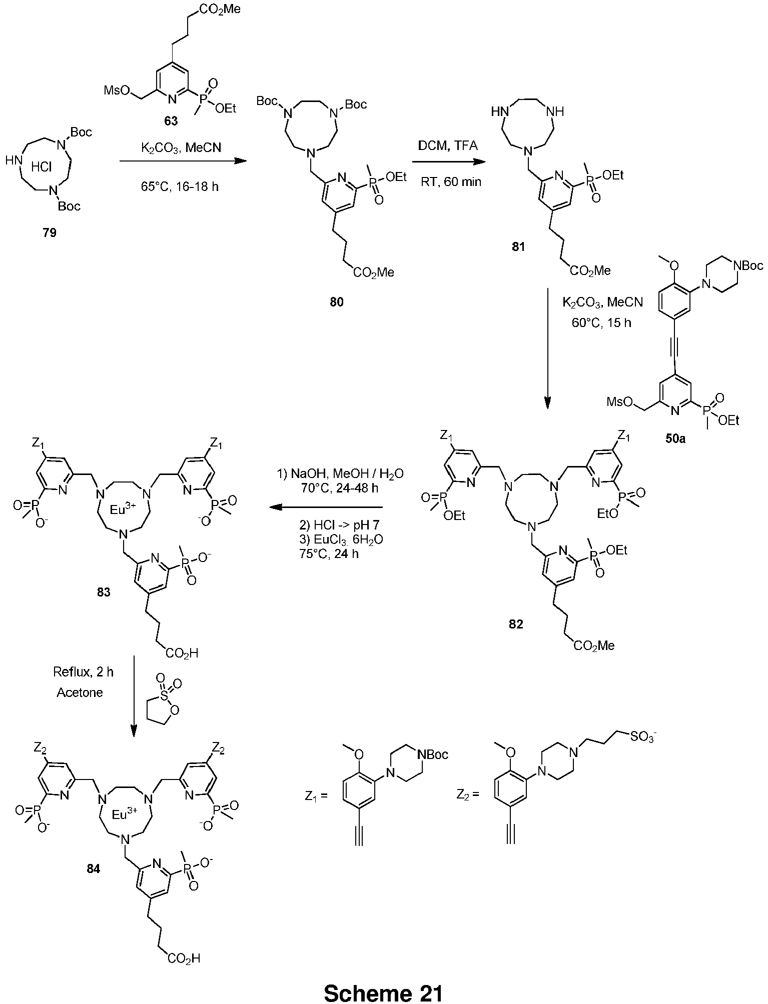

The synthesis of complex **84** which possesses piperazinyl moieties is described in scheme 21. It followed the same strategy to those described before. The commercially available diprotected triazacyclononane, **79,** was mono-alkylated. Deprotection of both Boc groups was carried out in the presence of trifluoroacetic acid. The chromophore was introduced at this stage then the phosphinate ester functionalities were hydrolyzed and the europium complex was formed after addition of europium chloride. The secondary amine reacted with propanesultone to give the desired complex **84.**

### Synthesis of functionalized complex with two chromophores

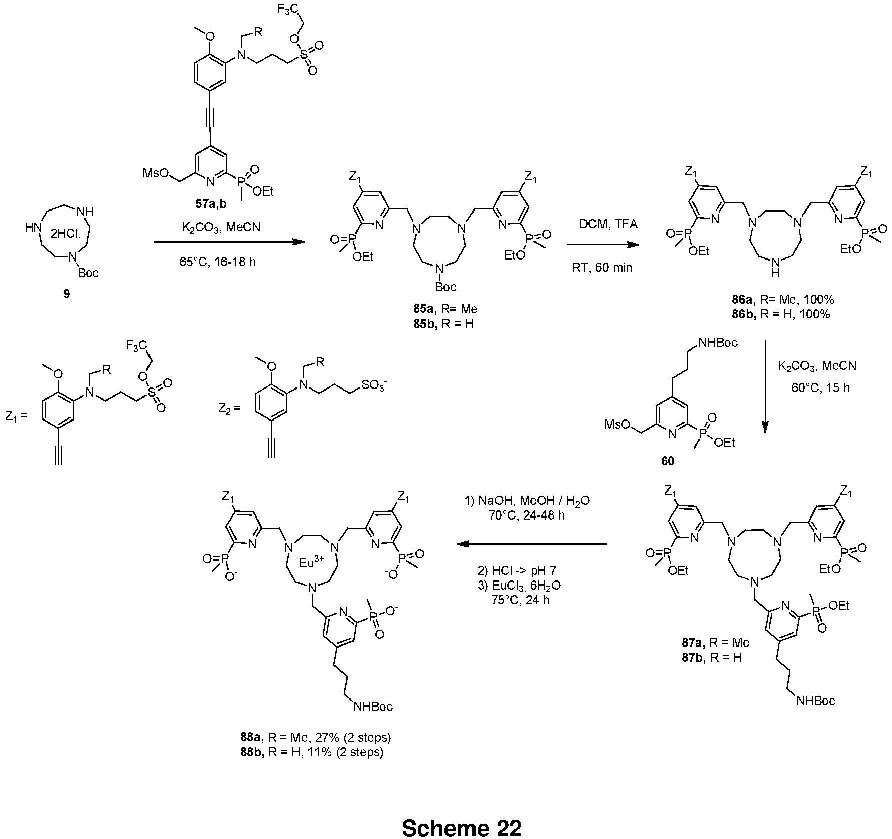

As previously described, the sulfonate group was introduced on the terminal part of one of the alkyl groups of the aniline functionality. The synthesis followed the same strategy using the chromophores **57a-b:** alkylation of the macrocycle, deprotection of the Boc group, introduction of the last pyridine derivative, hydrolysis of the phosphinate ester and complexation with europium. Also, the final basic phosphinate ester hydrolysis allowed the deprotection of the trifluoroethyl protective group, leading to the free sulfonate function moiety (scheme 22).

### Synthesis of functionalized complex with three chromophores

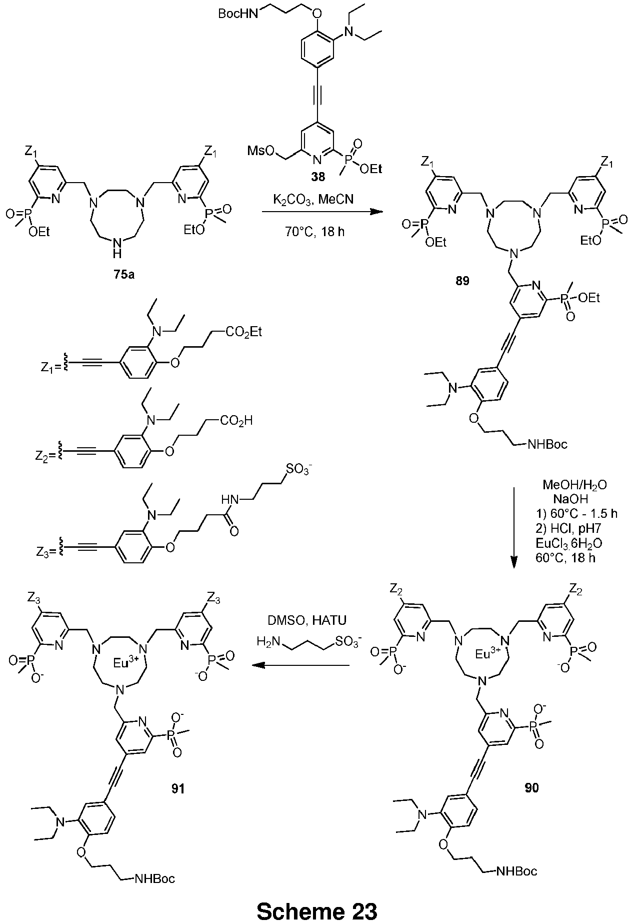

Complexes with 3 chromophores were prepared as described in scheme 23. The synthetic pathway was based on the same strategy as described before, leading to complex **91.**

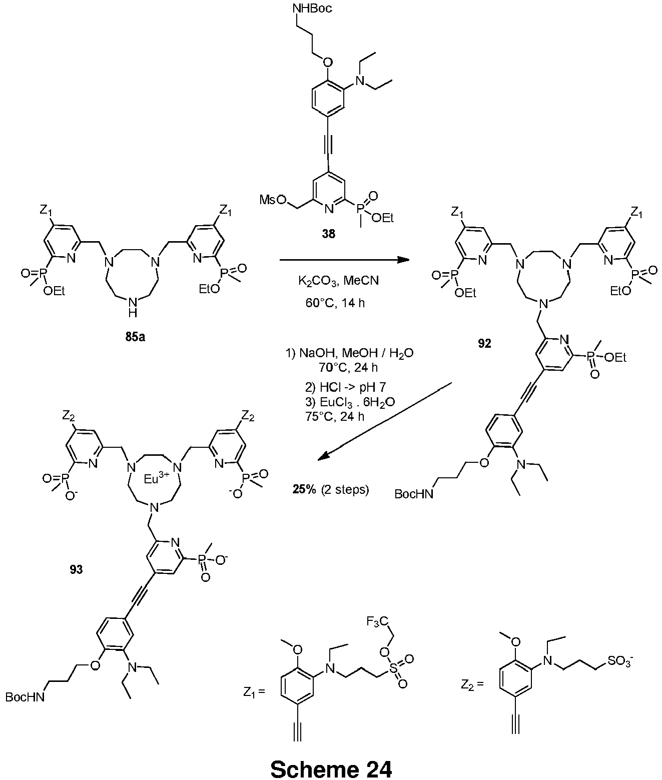

Scheme 24 shows the preparation, using the same strategy as described before, of dissymmetrical complex, **93,** with 2 identical chromophores (N,N-ethylpropylsulfonate), the third one being the moiety bearing an N,N-diethylaniline group. This complex can be used for subsequent bioconjugation.

### Synthesis of functionalized complex with three chromophores

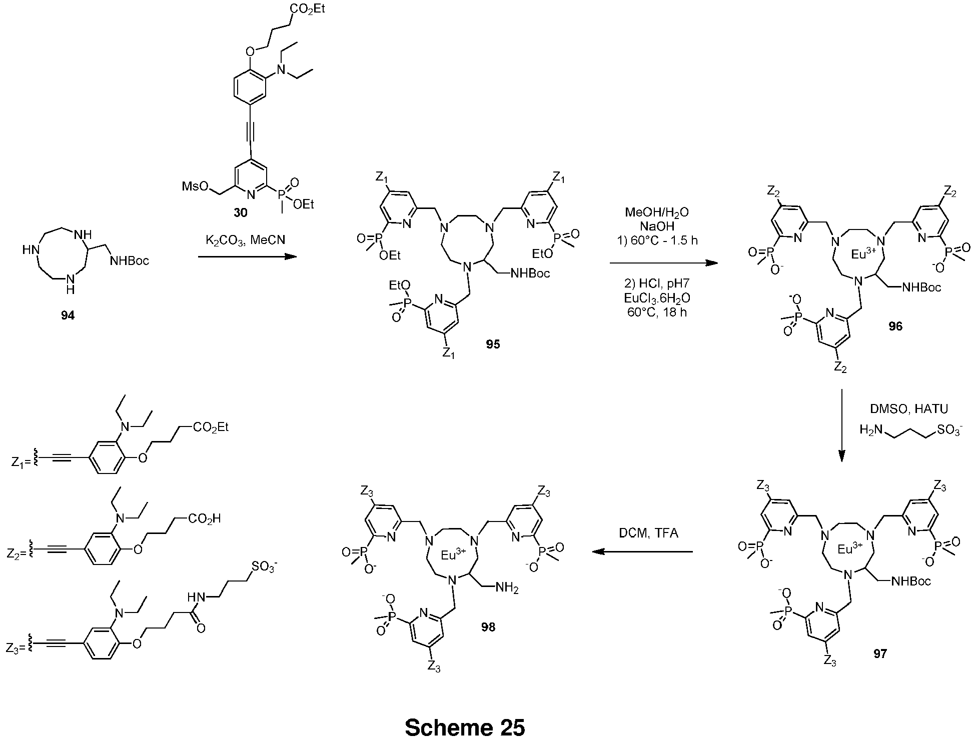

Another way to obtain a complex with 3 chromophores was to use the triazacyclononane with a pendant arm methylene NHBoc, previously disclosed in US9,981,967. This synthesis was straightforward and allowed the desired complex **98** with 3 identical chromophores to be obtained.

### Synthesis of bioconjugated complexes with 2 chromophores

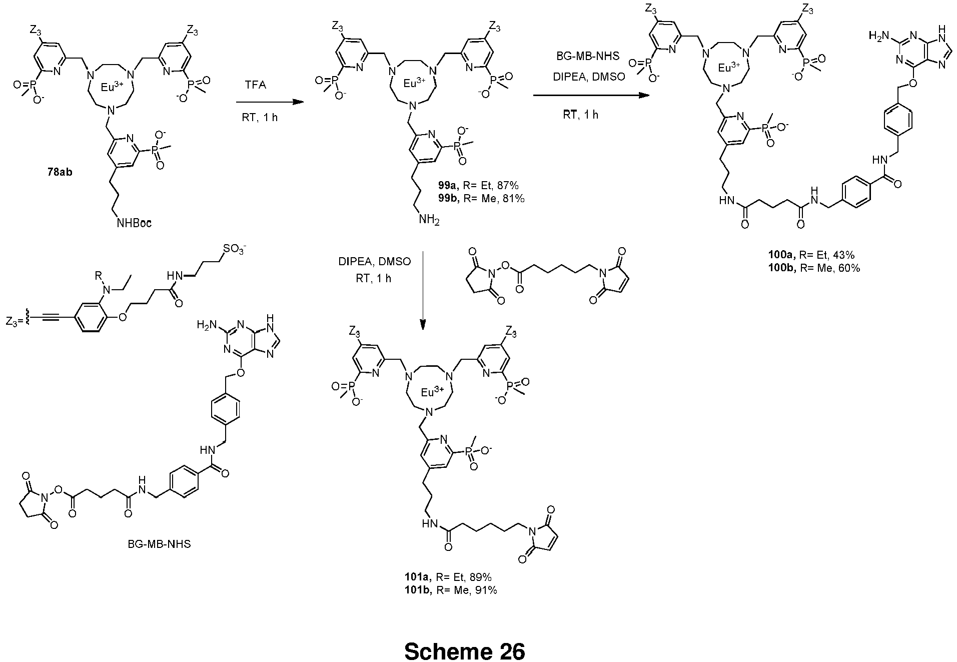

Scheme 26 illustrates the conjugation of europium complex **78a-b** with benzylguanine methyl benzamide NHS ester (BG-MB-NHS), a well-known enzymatic substrate (WO 2010/034931), to give the benzylguanine complexes, **100a-b,** or with maleimide to give complexes **101a-b** which can be used for antibody or protein labelling.

### Synthesis of acyclic complex

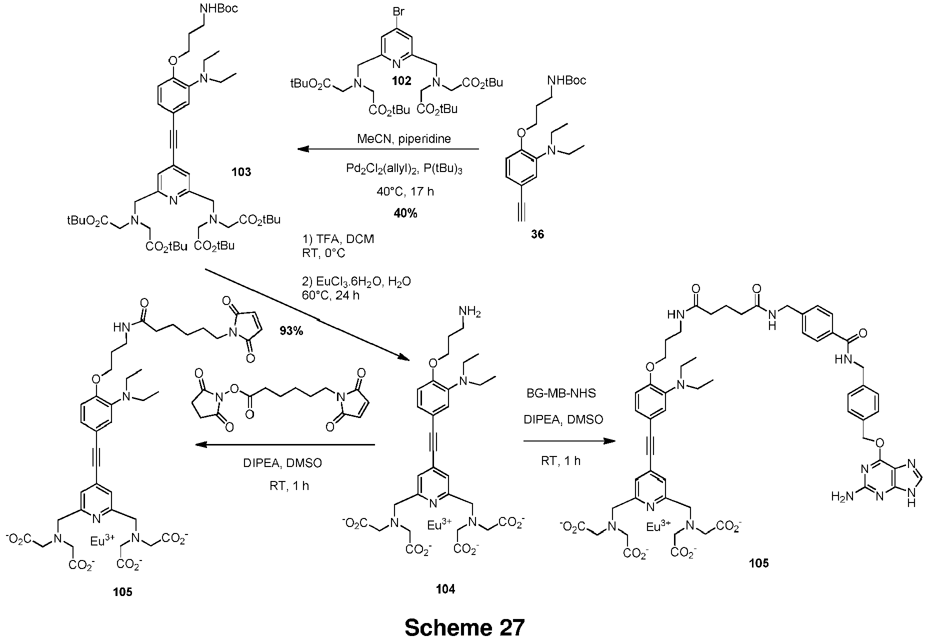

This scheme shows the synthesis of an acyclic compound. The synthesis of the pyridine ethynyl aryl derivative has been described previously by Takalo (op. cit.). An appropriate building block was used in order to obtain a europium chelate which was functionalized to allow bioconjugation. The corresponding maleimide and benzyl guanine derivatives **105** and **106** were prepared in a similar manner.

### Experimental part

The following abbreviations are used in the experimental part.
- 9-N₃: 1,4,7-triazacyclononane
- app.: apparent
- BG: benzylguanine
- Boc: *tert*-butoxycarbonyl
- br: broad (NMR)
- BSA: bovine serum albumin
- CPL: circularly polarised luminescence
- d: day(s) (reaction time)
- d: doublet (NMR)
- DCM: dichloromethane
- dd: doublet of doublets (NMR)
- ddq: doublet of doublet of quartets (NMR)
- DIPEA: diisopropylethylamine
- dm: doublet of multiplets (NMR)
- DMF: dimethylformamide
- DMSO: dimethylsulfoxide
- dppf: 1,1'-bis(diphenylphosphino)ferrocene
- EDTA: ethylenediaminetetraacetic acid
- ESI: electrospray ionization
- EtOAc: ethyl acetate
- EtOH: ethanol
- equiv.: equivalent(s)
- FA: formic acid
- GPCR: G-protein coupled receptor
- h: hour(s) (reaction time)
- HATU: (1-[Bis(dimethylamino)methylene]-1H-1,2,3-triazolo[4,5-b]pyridinium 3-oxide hexafluorophosphate
- HEPES: 4-(2-hydroxyethyl)-1-piperazineethanesulfonic acid
- HRMS: high resolution mass spectrometry
- ICP-MS: inductively coupled plasma mass spectrometry
- ICT: intramolecular charge transfer
- LRMS: low resolution mass spectrometry
- Ln: lanthanide
- LTG: LysoTracker Green
- m: multiplet (NMR)
- MeOH: methanol
- MES: 2-(*N*-morpholino)ethanesulfonic acid
- M.p.: melting point
- MS: mass spectrometry
- NMR: nuclear magnetic resonance
- PBS: phosphate buffered saline
- PDA: photo-diode array
- Pd/C: Palladium on carbon
- Pd(dppf)Cl₂: [1,1'-Bis(diphenylphosphino)ferrocene]palladium(II) dichloride
- q: quartet (NMR)
- quant.: quantitative
- RT: room temperature
- s: singlet (NMR)
- Sₙ: the nth singlet state (energy level)
- t: triplet (NMR)
- TEEAc: triéthylamine
- TFA: trifluoroacetic acid
- THF: tetrahydrofuran
- TLC: thin layer chromatography
- TMS: trimethylsilyl
- UV: ultraviolet
- Vis: visible

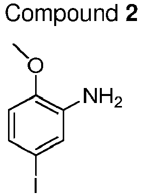

4-lodo-1-methoxy-2-nitrobenzene **1** (1.00 g, 3.58 mmol), glacial acetic acid (5 mL, 87 mmol) and MeOH (5 mL) were combined under argon. Iron powder (1.02 g, 18.3 mmol) was added and the mixture was heated to 50°C for 1 h. The mixture was filtered and the solvent was removed under reduced pressure. The resulting residue was dissolved in CH₂Cl₂ (100 mL) and an aqueous NaOH solution (25 mL, 2 M) was added; the subsequent precipitate was removed by filtration. The aqueous layer was separated and extracted with CH₂Cl₂ (5 x 15 mL). The organic layers were combined, dried over MgSO₄ and the solvent was removed under reduced pressure to yield **2** as pale brown solid (674 mg, 76%); ¹H-NMR (700 MHz, CDCl₃) *δ* 7.00 (dd, ³*J*_{H-H} = 8.3, ⁴*J*_{H-H} = 2.2, 1H), 6.98 (d, ⁴*J*_{H-H} = 2.2, 1H), 6.51 (d, ³*J*_{H-H} = 8.3, 1H), 3.81 (s, 3H); ¹³C-NMR (176 MHz, CDCl₃) *δ* 147.2, 138.0, 126.9, 123.0, 112.3, 83.4, 55.6; ESI-LRMS (+) *m*/*z* 250 [M+H]⁺; ESI-HRMS (+) calcd for [C₇H₉NO¹²⁷I]⁺ 249.9729, found 249.9750; m.p. 90°C.

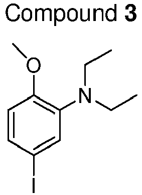

Compound **2** (2.83 g, 11.4 mmol), iodoethane (7 mL, 87 mmol) and K₂CO₃ (6.3 g, 45.6 mmol) were combined in anhydrous CH₃CN (15 mL) under argon. The reaction mixture was heated to 60°C for 48 h. After this time, the solvent was removed under reduced pressure and the residue was dissolved in CH₂Cl₂ (30 mL), washed with water (5 x 20 mL) and dried over K₂CO₃. Removal of the solvent under reduced pressure yielded a pale orange oil (2.16 g, 62%); ¹H-NMR (600 MHz, CDCl₃) *δ* 7.24 (dd, ³*J*_{H-H} = 8.5, ⁴*J*_{H-H} = 1.9, 1H), 7.15 (d, ⁴*J*_{H-H} = 1.9, 1H), 6.58 (d, ³*J*_{H-H} = 8.5, 1H), 3.81 (s, 3H), 3.12 (q, ³*J*_{H-H} = 7.1, 4H), 1.02 (t, ³*J*_{H-H} = 7.1, 6H); ¹³C-NMR (151 MHz, CDCl₃) *δ* 153.5, 141.0, 131.0, 130.0, 113.4, 83.0, 55.4, 45.9, 11.9; ESI-LRMS (+) *m*/*z* 306 [M+H]⁺; ESI-HRMS (+) calcd for [C_{H}H₁₇NO¹²⁷I]⁺ 306.0355, found 306.0363.

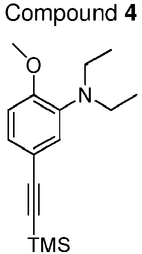

Compound **3** (548 mg, 1.80 mmol), trimethylsilylacetylene (0.5 mL, 3.6 mmol), Pd(dppf)Cl₂, DCM (150 mg, 0.184 mmol) and pyrrolidine (0.45 mL, 5.4 mmol) were combined under argon in anhydrous THF (3 mL). The reaction mixture was heated to 50°C for 19 h before removal of the solvent under reduced pressure. The subsequent residue was dissolved in CH₂Cl₂ (30 mL), washed with H₂O (4 x 30 mL) and dried over K₂CO₃. Removal of solvent under reduced pressure gave the crude product which was purified by column chromatography (SiO₂, 100% hexane to 6% EtOAc in hexane) to yield a pale orange oil (323 mg, 65%); ¹H-NMR (700 MHz, CDCl₃) *δ* 7.12 (d, ³*J*_{H-H} = 8.5, 1H), 7.03 (s, 1H), 6.75 (d, ³*J*_{H-H} = 8.5, 1H), 3.85 (s, 3H), 3.14 (q, ³*J*_{H-H} = 7.1, 4H), 1.01 (t, ³*J*_{H-H} = 7.1, 6H), 0.24 (s, 9H); ¹³C-NMR (176 MHz, CDCl₃) *δ* 154.5, 139.1, 127.2, 125.3, 115.1, 111.2, 105.9, 91.9, 55.6, 46.1, 12.1, 0.3; ²⁹Si-NMR (139 MHz, CDCl₃) *δ* -18.3; ESI-LRMS (+) *m*/*z* 276 [M+H]⁺; ESI-HRMS (+) calcd for [C₁₆H₂₆NOSi]⁺ 276.1784, found 276.1790; R_{f} = 0.40 (SiO₂, 10% EtOAc in hexane).

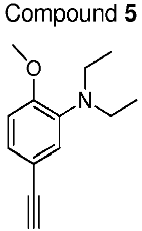

Triethylamine trihydrofluoride (0.75 mL, 3.28 mmol) was added to a solution of compound **4** (115 mg, 0.306 mmol) in anhydrous THF (3 mL) under argon. The solution was heated to 30°C for 37 h before removal of solvent under reduced pressure. The resulting oil was dissolved in CH₂Cl₂ (30 mL) and washed with water (3 x 40 mL). The combined aqueous layers were extracted with CH₂Cl₂ (5 x 40 mL) and the organic layers were combined and dried over K₂CO₃. Removal of the solvent under reduced pressure yielded a pale orange oil (81 mg, 87%); ¹H-NMR (700 MHz, CDCl₃) *δ* 7.14 (dd, ³*J*_{H-H} = 8.4, ⁴*J*_{H-H} = 2.0, 1H), 7.06 (d, ⁴*J*_{H-H} = 2.0, 1H), 6.78 (d, ³*J*_{H-H} = 8.4, 1H), 3.86 (s, 3H), 3.15 (q, ³*J*_{H-H} = 7.1, 4H), 2.98 (s, 1H), 1.02 (t, ³*J*_{H-H} = 7.1, 6H); ¹³C-NMR (176 MHz, CDCl₃) *δ* 154.6, 139.2, 127.1, 125.3, 113.9, 111.3, 84.4, 75.3, 55.7, 46.1, 12.1; ESI-LRMS (+) *m*/*z* 204 [M+H]⁺; ESI-HRMS (+) calcd for [C₁₃H₁₈NO]⁺ 204.1388, found 204.1401.

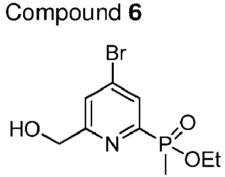

Compound **6** was synthesized in nine steps from 2-bromo-6-methylpyridine following the procedure described in US 2015/361116.

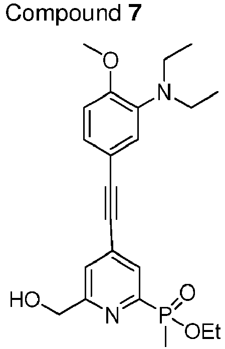

To a solution of compound **5** (120 mg, 0.59 mmol) and compound **6** (175 mg, 0.60 mmol) in anhydrous THF (2.5 mL) under argon was added pyrrolidine (0.1 mL, 1.22 mmol) and Pd(dppf)Cl₂.DCM (60 mg, 0.073 mmol). The reaction mixture was heated to 50°C for 18 h, the solvent was then removed under reduced pressure and the resulting residue was dissolved in CH₂Cl₂ (40 mL) and washed with water (3 × 40 mL). The organic layer was dried over K₂CO₃ and the solvent was removed under reduced pressure to yield a brown residue. This crude residue was purified by reverse phase HPLC (10 to 100% CH₃CN in H₂O over 10 min, *t*ᵣ = 10.7 min) to afford a pale yellow oil (118 mg, 48%); ¹H-NMR (600 MHz, CDCl₃) *δ* 8.03 (d, ³*J*_{H-P} = 6.0, 1H), 7.49 (s, 1H), 7.18 (d, ³*J*_{H-H} = 8.5, 1H), 7.09 (s, 1H), 6.82 (d, ³*J*_{H-H} = 8.5, 1H), 4.80 (s, 2H), 4.14 - 3.82 (m, 2H), 3.87 (s, 3H), 3.16 (q, ³*J*_{H-H} = 6.9, 4H), 1.77 (d, ²*J*_{H-P} = 15, 3H), 1.26 (t, ³*J*_{H-H} = 7.0, 3H), 1.03 (t, ³*J*_{H-H} = 6.9, 6H); ¹³C-NMR (151 MHz, CDCl₃) *δ* 160.8 (d, ³*J*_{C-P} = 19), 155.2, 153.2 (d, ¹*J*_{C-P} = 155), 139.5, 133.3 (d, ³*J*_{C- P} = 11), 128.3 (d, ²*J*_{C-P} = 22), 127.2, 125.0, 124.1 (d, ⁴*J*_{C-P} = 3), 113.5, 111.5, 97.0, 84.9, 64.2, 61.3 (d, ²*J*_{C-P} = 6), 55.7, 46.0, 16.5 (d, ³*J*_{C-P} = 6), 13.6 (d, ¹*J*_{C-P} = 105), 12.0; ³¹P{¹H}-NMR (243 MHz, CDCl₃) *δ* +39.4; ESI-LRMS (+) *m*/*z* 417 [M+H]⁺; ESI-HRMS (+) calcd for [C₂₂H₃₆N₂O₄P]⁺ 417.1943, found 417.1954.

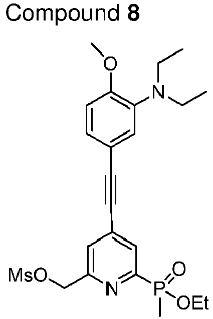

Compound **7** (67 mg, 0.161 mmol), methanesulfonic anhydride (56 mg, 0.322 mmol) and DIPEA (0.07 mL, 0.402 mmol) were combined in anhydrous THF (1.5 mL) under argon and stirred at RT for 90 min. After this time, the solvent was removed under reduced pressure. To the resulting residue was added CH₂Cl₂ (30 mL) and H₂O (30 mL). The organic layer was separated and washed with H₂O (2 × 30 mL), and the combined aqueous layers were extracted with CH₂Cl₂ (1 × 30 mL). The combined organic layers were dried over K₂CO₃ and the solvent was removed under reduced pressure to afford a pale orange oil which was used directly in the next step without further purification (69 mg, 87%); ¹H-NMR (400 MHz, CDCl₃) *δ* 8.09 (dd, ³*J*_{H-P} = 6.0, ⁴*J*_{H-H} = 1.5, 1H), 7.64 - 7.62 (m, 1H), 7.19 (dd, ³*J*_{H-H} = 8.5, ⁴*J*_{H-H} = 2.1, 1H), 7.10 (d, ⁴*J*_{H-H} = 2.1, 1H), 6.84 (d, ³*J*_{H-H} = 8.5, 1H), 5.36 (s, 2H), 4.19 - 3.81 (m, 5H), 3.17 (q, ³*J*_{H-H} = 7.1, 4H), 3.13 (s, 3H), 1.77 (d, ²*J*_{H-P} = 15, 3H), 1.27 (t, ³*J*_{H-H} = 7.0, 3H), 1.04 (t, ³*J*_{H-H} = 7.1, 6H); ESI-LRMS (+) *m*/*z* 495 [M+H]⁺. Compound **9** was synthesized in 3 steps following the procedure described in WO 2014/111661.

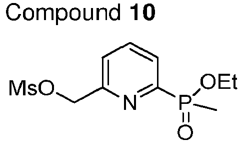

Compound **10** was synthesized in five steps from 2-bromo-6-methylpyridine following a procedure described in Inorg. Chem., 2012, 51, 8042.

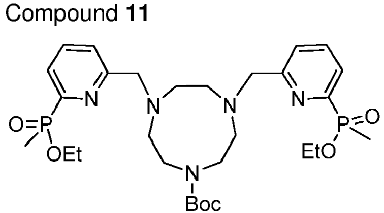

The dihydrochloride salt of 1-(*tert*-butoxycarbonyl)-1,4,7-triazacyclononane **9** (28 mg, 0.0926 mmol), compound **10** (64 mg, 0.218 mmol) and K₂CO₃ (40 mg, 0.289 mmol) were combined in anhydrous CH₃CN (2 mL) under argon and heated to 65°C for 18 h. After this time, the solution was separated from the inorganic salts and purified by reverse phase HPLC (10 to 100% CH₃CN in H₂O over 10 min, *t*ᵣ = 11.8 min) to yield a pale yellow oil (33 mg, 57%); ¹H-NMR (700 MHz, CDCl₃) *δ* 7.92 - 7.88 (m, 2H), 7.78 - 7.72 (m, 2H), 7.62 (d, ³*J*_{H-H} = 7.9, 1H), 7.55 (d, ³*J*_{H-H} = 7.9, 1H), 4.11 - 4.03 & 3.87 - 3.79 (m, 4H), 3.95 - 3.90 m, 4H), 3.40 - 3.29 (m, 4H), 3.11 - 2.96 (m, 4H), 2.72 - 2.59 (m, 4H), 1.75 (d, ²*J*_{H-P} = 15, 3H), 1.74 (d, ²*J*_{H-P} = 15, 3H), 1.44 (s, 9H), 1.25 - 1.21 (2 × t, ³*J*_{H-H} = 7.1, 6H); ¹³C-NMR (176 MHz, CDCl₃) *δ* 161.3 (br s), 155.7, 153.9 (d, ¹*J*_{C-P} = 160), 153.7 (d, ¹*J*_{C-P} = 160), 136.5 (d, ³*J*_{C-P} = 10), 136.4 (d, ³*J*_{C-P} = 10), 125.9 (2 × d, ²*J*_{C-P} = 20), 125.5, 125.3, 79.5, 63.4, 63.1, 61.0 (2 × d, ²*J*_{C-P} = 6), 56.4), 55.3, 54.8, 54.2, 50.1, 49.7, 28.7, 16.6, 16.5, 13.5 (d, ¹*J*_{C-P} = 104), 13.4 (d, ¹*J*_{C-P} = 104); ³¹P{¹H}-NMR (162 MHz, CDCl₃) *δ* +40.3, +40.2; ESI-LRMS (+) *m*/*z* 624 [M+H]⁺; ESI-HRMS (+) calcd for [C₂₉H₄₈N₅O₆P₂]⁺ 624.3080, found 624.3105.

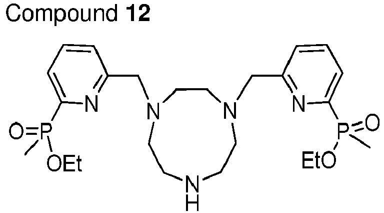

A solution of compound **11** (33 mg, 0.053 mmol) in trifluoroacetic acid and CH₂Cl₂ (20% v/v, 4 mL total) was prepared. The solution was stirred at RT for 25 min before removal of the solvent under reduced pressure to give an orange residue. To this residue was added CH₂Cl₂ (30 mL) before removal of the solvent under reduced pressure once again. This procedure was repeated five times to afford a pale orange oil (28 mg, quant.); ¹H-NMR (700 MHz, CDCl₃) *δ* 7.84 - 7.79 (m, 4H), 7.48 - 7.43 (m, 2H), 4.30 (s, 4H), 4.14 - 3.87 (m, 4H), 3.57 - 3.38 (m, 8H), 3.30 - 3.17 (m, 4H), 1.73 (d, ²*J*_{H-P} = 15, 6H), 1.28 (t, ³*J*_{H-H} = 7.0, 6H); ¹³C-NMR (176 MHz, CDCl₃) *δ* 156.8 (d, ³*J*_{C-P} = 20), 153.2 (d, ¹*J*_{C-P} = 160), 137.6 (d, ³*J*_{C-P} = 10), 126.2 (d, ²*J*_{C-P} = 21), 62.0 (d, ²*J*_{C-P} = 6), 59.9, 51.6, 49.4, 44.5 (2 × s), 16.3 (d, ³*J*_{C-P} = 6), 13.6 (d, ¹*J*_{C-P} = 102); ³¹P{¹H}-NMR (162 MHz, CDCl₃) *δ* +40.8, +40.7; ESI-LRMS (+) *m*/*z* 524 [M+H]⁺; ESI-HRMS (+) calcd for [C₂₄H₄₀N₅O₄P₂]⁺ 524.2556, found 524.2563.

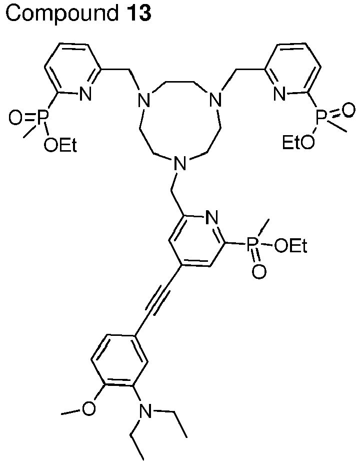

Compound **12** (8.5 mg, 16.2 µmol), compound **8** (16 mg, 32.4 µmol) and K₂CO₃ (5 mg, 35.4 µmol) were combined in anhydrous CH₃CN (1 mL) under argon and heated to 60°C for 18 h. After this time, the crude mixture was separated from the inorganic salts by filtration. The resulting solution was directly subjected to reverse phase HPLC (10 to 100% CH₃CN in H₂O over 10 min, *t*ᵣ = 11.0 min) to yield compound **13** as a pale yellow oil (7.4 mg, 50%); ¹H-NMR (400 MHz, CDCl₃) *δ* 8.01 (d, ³*J*_{H-P} = 6.0, 1H), 7.94 - 7.87 (m, 2H), 7.82 - 7.74 (m, 2H), 7.67 - 7.61 (m, 3H), 7.18 (d, ³*J*_{H-H} = 8.4, 1H), 7.09 (s, 1H), 6.84 (d, ³*J*_{H-H} = 8.4, 1H), 4.18 - 3.76 (m, 15H), 3.17 (q, ³*J*_{H-H} = 6.9, 4H), 2.97 - 2.80 (m, 12H), 1.76 (d, ²*J*_{H-P} = 15, 3H), 1.75 (d, ²*J*_{H-P} = 15, 3H), 1.29 - 1.20 (m, 9H), 1.04 (t, ³*J*_{H-H} = 6.9, 6H); ¹³C-NMR (101 MHz, CDCl₃) *δ* 161.5 (d+d, ³*J*_{C-P} = 12), 155.0, 153.6 (d+d, ¹*J*_{C-P} = 159), 139.4, 136.4 (d, ³*J*_{C-P} = 9), 133.3 (d, ³*J*_{C-P} = 16), 127.8 (d, ²*J*_{C-P} = 22), 127.0, 126.4 (d, ⁴*J*_{C-P} = 4), 125.9, 125.6 (d, ²*J*_{C-P} = 20), 124.8, 113.4, 111.3, 96.4, 85.1, 64.4 (2 × s), 60.9 (d+d, ²*J*_{C-P} = 6), 55.6, 45.9, 16.5 (d+d, ³*J*_{C-P} = 6.0), 13.4 (d+d, ¹*J*_{C-P} = 104), 11.9; ³¹P{¹H}-NMR (162 MHz, CDCl₃) *δ* + 40.2(2P), +40.0 (1P); ESI-LRMS (+) *m*/*z* 922 [M+H]⁺; ESI-HRMS (+) calcd for [C₄₆H₆₇N₇O₇P₃]⁺ 922.4315, found 922.4355.

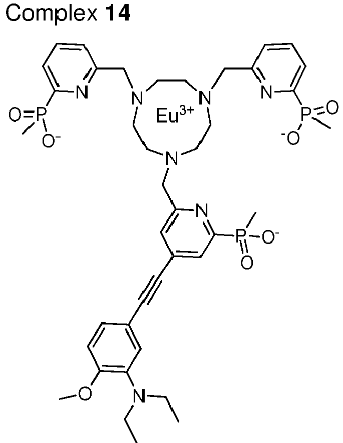

The ligand **13** (2 mg, 2 µmol) was dissolved in a mixture of CH₃OH/H₂O (1:1, 2 mL total) and the pH was adjusted to 12 using an aqueous NaOH solution. The solution was heated to 60°C for 14 h. After cooling and adjusting the pH to 7 using dilute hydrochloric acid (0.1 M), EuCl₃.6H₂O (3 mg, 8 µmol) was added and the reaction mixture was heated to 60°C for 15 h. The reaction mixture was purified by reverse phase HPLC (10 to 100% CH₃CN in H₂O over 10 min, *t*ᵣ = 8.5 min) to yield a yellow solid (2 mg, 93%); ESI-LRMS (+) *m*/*z* 986 [M+H]⁺, 495 [M+2H]²⁺; ESI-HRMS (+) calcd for [C₄₀H₅₂N₇O₇P₃¹⁵¹Eu]⁺ 986.2340, found 986.2327; *τ*_{H2O} (ms) = 0.50 (pH 9), 0.53 (pH 8), 0.74 (pH 7), 1.06 (pH 6), 1.15 (pH 5), 1.16 (pH 4); *ε*_{331 nm} = 11450 M⁻¹ cm⁻¹; *Φ*_{pH 8} = 0.5%, *Φ*_{pH 4} = 16.8% (*λ*_{exc} = 331 nm).

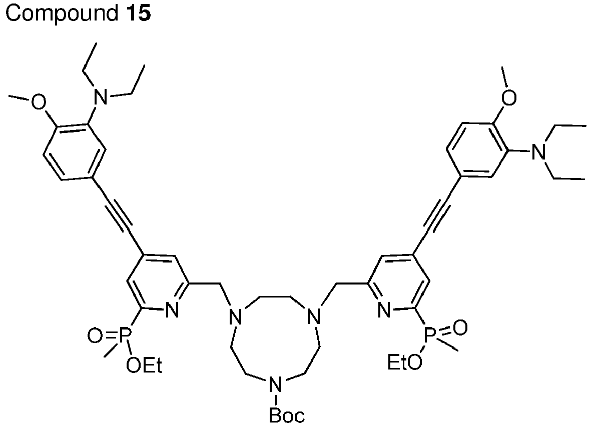

The dihydrochloride salt of 1-(*t*-butoxycarbonyl)-1,4,7-triazacyclononane **9** (31 mg, 0.10 mmol), mesylate **8** (122 mg, 0.26 mmol) and K₂CO₃ (60 mg, 0.43 mmol) were combined in anhydrous CH₃CN (2 mL) under argon. The resulting mixture was heated to 60°C for 14 h before separating the crude solution from the inorganic salts and removing the solvent under reduced pressure to yield an orange oil that was purified by reverse phase HPLC (10 to 100% CH₃CN in H₂O over 10 min, *t*ᵣ = 15.6 min) to afford a pale yellow oil (66 mg, 62%); ¹H-NMR (400 MHz, CDCl₃) *δ* 8.03 - 7.96 (m, 2H), 7.66 (s, 1H), 7.58 (s, 1H), 7.17 (d, 2H), 7.08 (s, 2H), 6.82 (d, 2H), 4.15 - 4.02 & 3.91 - 3.78 (m, 10H), 3.94 (2 × s, 4H), 3.44 - 3.29 (m, 4H), 3.20 - 3.04 (m, 12H), 2.76 - 2.61 (m, 4H), 1.76 (2 × d, 6H), 1.48 (s, 9H), 1.24 (2 × t, 6H), 1.02 (t, 12H); ¹³C-NMR (101 MHz, CDCl₃) *δ* 161.7 (d, ³*J*_{C-P} = 21), 161.5 (d, ³*J*_{C-P} = 21), 155.7, 155.1, 155.0, 153.8 (d, ¹*J*_{C-P} = 157), 153.6 (d, ¹*J*_{C-P} = 157), 139.9 (2 × s), 132.8 (d, ³*J*_{C-P} = 12), 132.7 (d, ³*J*_{C-P} = 12), 128.0 (d, ²*J*_{C-P} = 22), 127.9 (d, ²*J*_{C-P} = 22), 127.1, 126.4 (d, ⁴*J*_{C-P} = 3), 126.1 (d, ⁴*J*_{C-P} = 3), 125.0 (2 × s), 113.7, 113.6, 111.4 (2 × s), 96.5, 96.2, 85.3 (d, ⁴*J*_{C-P} = 2), 85.1 (d, ⁴*J*_{C-P} = 2), 79.5, 62.9, 62.6, 61.1 (2 × d, ²*J*_{C-P} = 6), 56.1, 55.7, 55.0, 54.6, 54.0, 50.0, 49.7, 46.0, 28.8, 16.6 (d), 13.5 (d, ¹*J*_{C-P} = 1.5), 13.4 (d, ¹*J*_{C-P} = 1.5), 12.0; ³¹P{¹H}-NMR (162 MHz, CDCl₃) *δ* + 40.2 (1P), +40.1 (1P); ESI-LRMS (+) *m*/*z* 1027 [M+H]⁺; ESI-HRMS (+) calcd for [C₅₅H₇₈N₇O₈P₂]⁺ 1026.539, found 1026.543.

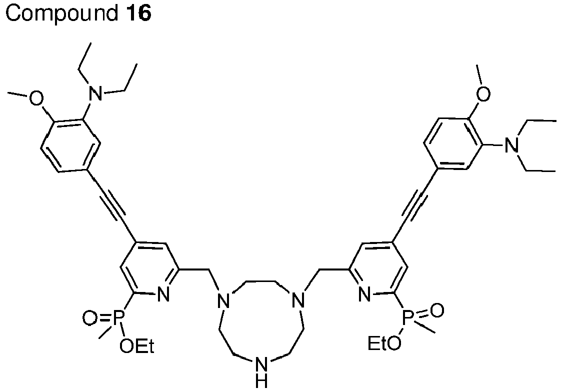

A solution of compound 15 (66 mg, 0.0643 mmol) in trifluoroacetic acid and CH₂Cl₂ (10% v/v, 3 mL total) was prepared. The solution was stirred at RT for 1 h before removal of the solvent under reduced pressure to give an orange residue. To this residue was added CH₂Cl₂ (30 mL) before removal of the solvent under reduced pressure once again. This procedure was repeated five times to afford a pale orange oil (67 mg, quant.); ¹H-NMR (600 MHz, CD₃OD) *δ* 7.97 (s, 2H), 7.95 (dd, ³*J*_{H-P} = 6, ⁴*J*_{H-H} = 1.0, 2H), 7.79 (dd, ³*J*_{H-H} = 8.7, ⁴*J*_{H-H} = 1.9, 2H), 7.72 - 7.70 (m, 2H), 7.41 (d, ³*J*_{H-H} = 8.7, 2H), 4.24 (s, 4H), 4.17 - 4.10 & 4.01 - 3.95 (m, 4H), 4.07 (s, 6H), 3.70 (q, ³*J*_{H-H} = 7.2, 8H), 3.42 - 3.35 (m, 4H), 3.29 - 3.23 (m, 4H), 3.06 - 2.99 (m, 4H), 1.82 (d, ²*J*_{H-P} = 15, 6H), 1.30 (t, ³*J*_{H-H} = 7.0, 6H), 1.12 (t, ³*J*_{H-H} = 7.2, 12H); ¹³C-NMR (151 MHz, CD₃OD) *δ* 160.8 (d, ³*J*_{C-P} = 20), 155.6, 155.2 (d, ¹*J*_{C-P} = 159), 136.8, 133.8 (d, ³*J*_{C-P} = 12), 128.6, 128.5 (d, ²*J*_{C-P} = 24), 127.5, 126.2, 116.9, 114.9, 94.6, 87.3 (d, ⁴*J*_{C-P} = 2), 63.1 (d, ²*J*_{C-P} = 7), 60.6, 57.7, 55.0, 52.3, 49.9, 45.5, 16.8 (d, ³*J*_{C-P} = 6.2), 13.6 (d, ¹*J*_{C-P} = 103), 10.4, ³¹P{¹H}-NMR (162 MHz, CD₃OD) *δ* +40.8; ESI-LRMS (+) *m*/*z* 926 [M+H]⁺, 464 [M+2H]²⁺, 309 [M+3H]³⁺; ESI-HRMS (+) calcd for [C₅₀H₇₀N₇O₆P₂]⁺ 926.4863, found 926.4865.

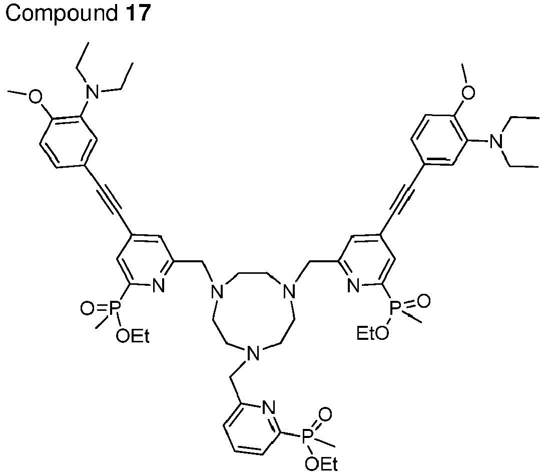

Compound **16** (39 mg, 0.0375 mmol), mesylate **10** (36 mg, 0.123 mmol) and K₂CO₃ (30 mg, 0.217 mmol) were combined in anhydrous CH₃CN (2 mL) under argon and heated to 60°C for 18 h. After this time, the crude mixture was separated from the inorganic salts by centrifugation and the resulting solution was directly subjected to reverse phase HPLC (10 to 100% CH₃CN in 25 mM ammonium bicarbonate buffer over 10 min, *t*ᵣ = 11.8 min) to yield a pale yellow oil (23 mg, 55%); ¹H-NMR (600 MHz, CDCl₃) *δ* 8.00 (dd, ³*J*_{H-P} = 6.0, ⁴*J*_{H-H} = 1.3, 2H), 7.93 - 7.88 (m, 1H), 7.83 - 7.76 (m, 1H), 7.73 - 7.67 (m, 1H), 7.65 (s, 2H), 7.18 (dd, ³*J*_{H-H} = 8.5, ⁴*J*_{H-H} = 1.8, 2H), 7.09 (d, ⁴*J*_{H-H} = 1.8, 2H), 6.83 (d, ³*J*_{H-H} = 8.5, 2H), 4.14 - 4.04 & 3.90 - 3.82 (m, 12H), 4.01 - 3.91 (m, 4H), 3.16 (q, ³*J*_{H-H} = 7.1, 8H), 3.07 - 2.83 (m, 12H), 1.76 (d, ²*J*_{H-P} = 15, 6H), 1.75 (d, ²*J*_{H-P} = 15, 3H), 1.25 (t, ³*J*_{H-H} = 7.0, 6H), 1.23 (t, ³*J*_{H-H} = 7.0, 3H), 1.03 (t, ³*J*_{H-H} = 7.1, 12H); ESI-LRMS (+) *m*/*z* 1124 [M+H]⁺, 563 [M+2H]²⁺, 375 [M+3H]³⁺; ESI-HRMS (+) calcd for [C₅₉H₈₂N₈O₈P₃]⁺ 1123.547, found 1123.548.

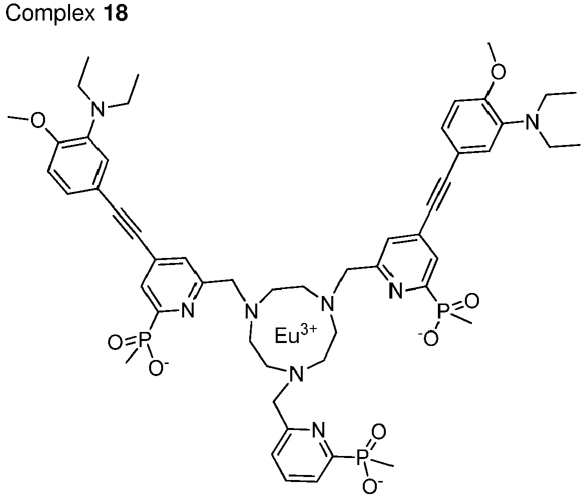

The ligand **17** (11.5 mg, 0.01024 mmol) was dissolved in a mixture of CH₃OH/H₂O (1:1, 4 mL total) and the pH was adjusted to 12 using an aqueous 1M NaOH solution. The solution was heated to 60°C for 15 h. After cooling and adjusting the pH to 6 using dilute hydrochloric acid (0.1 M), EuCl₃.6H₂O (6 mg, 0.0164 mmol) was added and the reaction mixture was heated to 60°C for 17 h. After this time, the solution was separated from the inorganic salts by centrifugation and purified by reverse phase HPLC (10 to 100% CH₃OH in H₂O over 10 min, *t*ᵣ = 13.6 min) to yield a yellow solid (7 mg, 58%); ESI-LRMS (+) *m*/*z* 1189 [M+H]⁺, 595 [M+2H]²⁺, 397 [M+3H]³⁺; ESI-HRMS (+) calcd for [C₅₃H₆₈N₈O₈P₃¹⁵¹Eu]²⁺ 595.1796, found 595.1741; *τ*_{H2O} (ms) = 0.34 (pH 9), 0.34 (pH 8), 0.47 (pH 7), 0.78 (pH 6), 0.96 (pH 5), 1.00 (pH 4);

Compound **19** is commercially available.

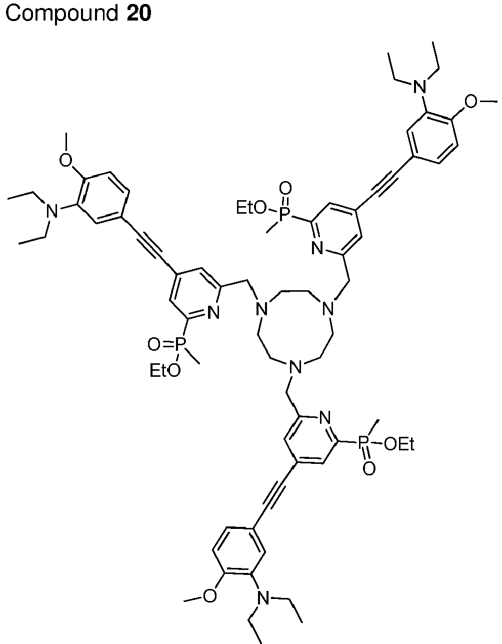

The trihydrochloride salt of 1,4,7-triazacyclononane **19** (4.5 mg, 0.0189 mmol), compound **8** (33 mg, 0.067 mmol) and K₂CO₃ (20 mg, 0.145 mmol) were combined in anhydrous CH₃CN (1.5 mL) under argon and heated to 60°C for 17 h. After this time, the crude mixture was separated from the inorganic salts by filtration. The resulting solution was subjected directly to reverse phase HPLC (10 to 100% CH₃CN in H₂O over 10 min, *t*ᵣ = 16.5 min) to yield a pale yellow oil (8 mg, 32%); ¹H-NMR (600 MHz, CDCl₃) *δ* 8.01 (d, ³*J*_{H-P} = 5.0, 3H), 7.68 (s, 3H), 7.18 (dd, ³*J*_{H-H} = 8.5, ⁴*J*_{H-H} = 1.9, 3H), 7.09 (d, ⁴*J*_{H-H} = 1.9, 3H), 6.82 (d, ³*J*_{H-H} = 8.5, 3H), 4.13 - 4.05 & 3.87 - 3.81 (m, 6H), 3.92 (s, 6H), 3.88 (s, 9H), 3.16 (q, ³*J*_{H-H} = 7.2, 12H), 2.94 (br s, 12H), 1.76 (d, ²*J*_{H-P} = 15, 9H), 1.24 (t, ³*J*_{H-H} = 7.0, 9H), 1.03 (t, ³*J*_{H-H} = 7.2, 18H); ¹³C-NMR (151 MHz, CDCl₃) *δ* 161.9 (d, ³*J*_{C-P} = 20), 155.4, 154.0 (d, ¹*J*_{C-P} = 157), 139.8, 133.0 (d, ³*J*_{C-P} = 12), 128.0 (d, ²*J*_{C-P} = 23), 127.3, 126.8, 125.2, 113.9, 111.7, 96.6, 85.5, 64.2, 61.3 (d, ²*J*_{C-P} = 7), 56.1, 55.9, 46.3, 16.8 (d, ³*J*_{C-P} = 6), 13.7 (d, ¹*J*_{C-P} = 104), 12.3; ³¹P{¹H}-NMR (162 MHz, CDCl₃) *δ* +40.1; ESI-LRMS (+) *m*/*z* 1325 [M+H]⁺, 663 [M+2H]²⁺, 443 {M+3H]³⁺; ESI-HRMS (+) calcd for [C₇₂H₉₈N₉O₉P₃]²⁺ 662.8350, found 662.8334.

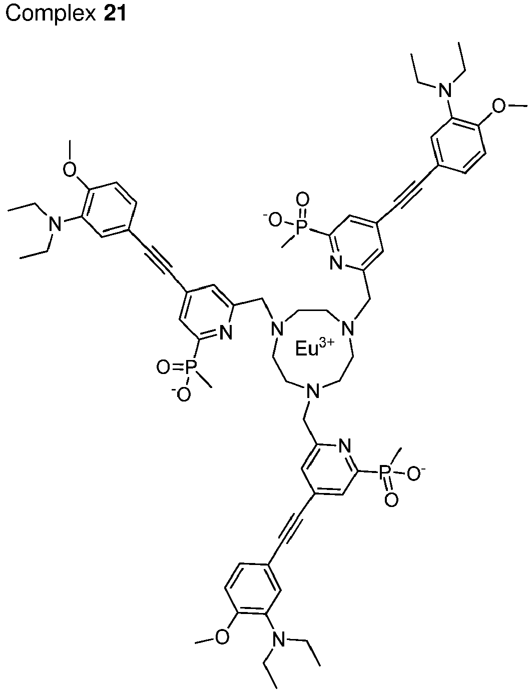

The ligand **20** (8 mg, 6 µmol) was dissolved in a mixture of CH₃OH/H₂O (1:1, 2 mL total) and the pH was adjusted to 12 using an aqueous NaOH solution (1.0 M). The solution was heated to 60°C for 4 h. After cooling and adjusting the pH to 7 using hydrochloric acid (1.0 M), EuCl₃.6H₂O (3 mg, 8 µmol) was added and the reaction mixture was heated to 60°C for 19 h. The reaction mixture was purified by reverse phase HPLC (10 to 100% CH₃CN in H₂O over 10 min, *t*ᵣ = 12.4 min) to yield a yellow solid (4 mg, 48%); ESI-LRMS (+) *m*/*z* 1390 [M+H]⁺, 696 [M+2H]²⁺, 464 [M+3H]³⁺; ESI-HRMS (+) calcd for [C₆₆H₈₂N₉O₉P₃¹⁵¹Eu]⁺ 1390.467, found 1390.470; *τ*_{H2O} (ms) = 0.24 (pH 9), 0.25 (pH 8), 0.32 (pH 7), 0.59 (pH 6), 0.83 (pH 5), 0.84 (pH 4); *ε*_{331 nm} = 45000 L M⁻¹ cm⁻¹.

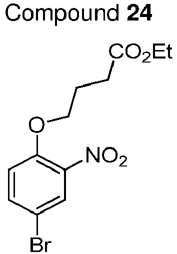

To a mixture of 4-bromo-2-nitrophenol **22** (2.49 g, 11.4 mmol) and K₂CO₃ (2.31 g, 16.7 mmol) in anhydrous CH₃CN (40 mL) under argon was added ethyl 4-bromobutyrate **23** (2.3 mL, 17.2 mmol). The mixture was heated to 70°C for 64 h before removing solvent under reduced pressure. CH₂Cl₂ (50 mL) was added to the residue and the resulting suspension was washed with H₂O (5 x 50 mL). The organic layer was dried over K₂CO₃ before removing the solvent under reduced pressure to yield a crude residue which was purified by column chromatography (SiO₂, 1:1 hexane/CH₂Cl₂ to 100% CH₂Cl₂) to afford a pale yellow oil (3.38 g, 98%); ¹H-NMR (700 MHz, CDCl₃) *δ* 7.95 (d, ⁴*J*_{H-H} = 2.5, 1H), 7.60 (dd, ³*J*_{H-H} = 8.9, ⁴*J*_{H-H} = 2.5, 1H), 6.97 (d, ³*J*_{H-H} = 8.9, 1H), 4.17 - 4.12 (m, 4H), 2.55 (t, ³*J*_{H-H} = 7.0, 2H), 2.18-2.12 (m, 2H), 1.25 (t, ³*J*_{H-H} = 7.1); ¹³C-NMR (176 MHz, CDCl₃) *δ* 173.1, 151.6, 140.4, 137.0, 128.5, 116.3, 112.1, 68.9, 60.8, 30.3, 24.3, 14.4; ESI-LRMS (+) m/z 332 [M+H]⁺; ESI-HRMS (+) calcd for [C₁₂H₁₅NO₅Br]⁺ 332.0134, found 332.0146; R_{f} = 0.47 (SiO₂, 100% CH₂Cl₂).

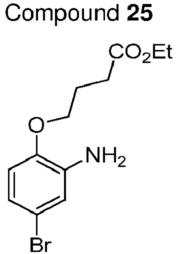

Compound **24** (4.30 g, 12.95 mmol), iron powder (3.60 g, 64.5 mmol) and glacial acetic acid (3.7 mL, 64.6 mmol) were combined in EtOH (20 mL) under argon and heated to 50°C for 6 h. The mixture was allowed to cool before filtering and removing the solvent under reduced pressure. DCM (50 mL) was added to the resulting mixture and the resulting solution was washed with a saturated aqueous Na₄EDTA solution (3 x 40 mL) and H₂O (2 x 40 mL). The combined aqueous layers were extracted with CH₂Cl₂ (6 x 40 mL). The combined organic layers were dried over K₂CO₃ and the solvent was removed under reduced pressure to afford a pale golden oil (3.5 g, 90%); ¹H-NMR (700 MHz, CDCl₃) *δ* 6.75 (d, ⁴*J*_{H-H} = 2.4, 1H), 6.70 (dd, ³*J*_{H-H} = 8.4, ⁴*J*_{H-H} = 2.4, 1H), 6.55 (d, ³*J*_{H-H} = 8.4, 1H), 4.11 (q, ³*J*_{H-H} = 7.2, 2H), 3.93 (t, ³*J*_{H-H} = 6.1, 2H), 3.89 (br s, 2H), 2.46 (t, ³*J*_{H-H} = 7.1, 1H), 2.11 - 2.06 (m, 2H), 1.22 (t, ³*J*_{H-H} = 7.2, 3H); ¹³C-NMR (176 MHz, CDCl₃) *δ* 173.1, 145.3, 138.0, 120.3, 117.2, 113.3, 112.6, 67.4, 60.5, 31.0, 24.6, 14.2; ESI-LRMS (+) *m*/*z* 302 [M+H]⁺; ESI-HRMS (+) calcd for [C₁₂H₁₇NO₃Br]⁺ 302.0392, found 302.0402.

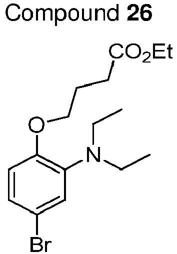

To compound **25** (3.19 g, 10.6 mmol) and K₂CO₃ (3.73 g, 27.0 mmol) in anhydrous CH₃CN (10 mL) under argon was added iodoethane (4 mL, 50 mmol). The mixture was heated to 70°C for 65 h before removing the solvent under reduced pressure. The resulting residue was dissolved in CH₂Cl₂ (50 mL) and washed with H₂O (4 x 50 mL), before drying over K₂CO₃. Removal of the solvent under reduced pressure yielded a crude residue that was purified by column chromatography (SiO₂, neat CH₂Cl₂ to 1% CH₃OH in CH₂Cl₂) to afford a light red oil (2.51 g, 66%); ¹H-NMR (700 MHz, CDCl₃) *δ* 6.99 - 6.96 (m, 2H), 6.69 (d, ³*J*_{H-H} = 8.4, 1H), 4.13 (q, ³*J*_{H-H} = 7.2, 2H), 3.99 (t, ³*J*_{H-H} = 6.3, 2H), 3.13 (q, ³*J*_{H-H} = 7.0, 4H), 2.51 (t, ³*J*_{H-H} = 7.4, 2H), 2.15 - 2.10 (m, 2H), 1.25 (t, ³*J*_{H-H} = 7.0, 3H), 1.04 (t, ³*J*_{H-H} = 7.0, 6H); ¹³C-NMR (176 MHz, CDCl₃) *δ* 173.2, 151.8, 141.8, 124.4, 124.1, 114.8, 113.4, 67.8, 60.6, 45.7, 31.0, 24.9, 14.4, 12.5; ESI-LRMS (+) *m*/*z* 358 [M+H]⁺; ESI-HRMS (+) calcd for [C₁₆H₂₅NO₃Br]⁺ 358.1018, found 358.1020; R_{f} = 0.04 (SiO₂, neat CH₂Cl₂), 0.3 (1% CH₃OH in CH₂Cl₂).

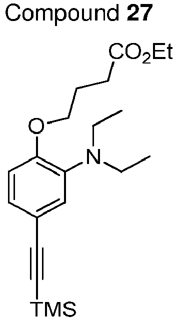

Compound 26 (3.03 g, 8.46 mmol) and Pd₂Cl₂(allyl)₂ (310 mg, 0.85 mmol) were combined in a vessel which was degassed then back-filled with argon in three cycles followed by the addition of anhydrous CH₃CN (15 mL). To this mixture was added P(*t*-Bu)₃ (0.31 mL, 1.28 mmol), trimethylsilylacetylene (2.4 mL, 17.3 mmol) and piperidine (2.1 mL, 21.3 mmol) in that order. The reaction mixture was stirred at 34°C for 30 h. The solvent was subsequently removed under reduced pressure and the residue was dissolved in CH₂Cl₂ (50 mL). The solution was washed with H₂O (4 x 50 mL) before drying over K₂CO₃. Removal of the solvent under reduced pressure yielded a brown oil which was purified by column chromatography (SiO₂, 100% hexane to 6% EtOAc in hexane) to afford a yellow oil (2.29 mg, 75%); ¹H-NMR (600 MHz, CDCl₃) *δ* 7.06 (dd, ³*J*_{H-H} = 8.4, ⁴*J*_{H-H} = 2.0, 1H), 7.01 (d, ⁴*J*_{H-H} = 2.0, 1H), 6.74 (d, ³*J*_{H-H} = 8.4, 1H), 4.14 (q, ³*J*_{H-H} = 7.1, 2H), 4.03 (t, ³*J*_{H-H} = 6.1, 2H), 3.13 (q, ³*J*_{H-H} = 7.2, 4H), 2.52 (t, ³*J*_{H-H} = 7.3, 2H), 2.17 - 2.11 (m, 2H), 1.27 (t, ³*J*_{H-H} = 7.1, 3H), 1.03 (t, ³*J*_{H-H} = 7.2, 6H), 0.24 (s, 9H); ¹³C-NMR (151 MHz, CDCl₃) *δ* 173.1, 153.3, 139.7, 126.6, 125.0, 115.2, 112.6, 105.8, 91.7, 67.3, 60.4, 45.6, 30.9, 24.6, 14.2, 12.3, 0.10; ²⁹Si NMR (139 MHz, CDCl₃) *δ* -16.0; ESI-LRMS (+) *m*/*z* 376 [M+H]⁺; ESI-HRMS (+) calcd for [C₂₁H₃₄NO₃Si]⁺ 376.2308, found 376.2299; R_{f} = 0.3 (SiO₂, 10% EtOAc in hexane).

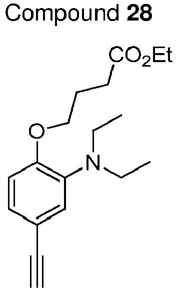

Triethylamine trihydrofluoride (6.25 mL, 38 mmol) was added to compound **27** (960 mg, 2.56 mmol) in anhydrous THF (8 mL) under argon. The solution was stirred at 30°C for 24 h before removing the solvent under reduced pressure. The residue was subsequently dissolved in CH₂Cl₂ (30 mL) and washed with water (6 x 30 mL). The combined aqueous layers were extracted with CH₂Cl₂ (2 x 30 mL) then dried over K₂CO₃ to yield a yellow oil (749 mg, 97%). The product was used in the next step without further purification; ¹H-NMR (700 MHz, CDCl₃) *δ* 7.07 (dd, ³*J*_{H-H} = 8.3, ⁴*J*_{H-H} = 2.0, 1H), 7.03 (d, ⁴*J*_{H-H} = 2.0, 1H), 6.75 (d, ³*J*_{H-H} = 8.3, 1H), 4.13 (q, ³*J*_{H-H} = 7.2, 2H), 4.03 (t, ³*J*_{H-H} = 6.3, 2H), 3.13 (q, ³*J*_{H-H} = 7.2, 4H), 2.97 (s, 1H), 2.52 (t, ³*J*_{H-H} = 7.4, 2H), 2.17 - 2.12 (m, 2H), 1.25 (t, ³*J*_{H-H} = 7.2, 3H), 1.03 (t, ³*J*_{H-H} = 7.1, 6H); ¹³C-NMR (176 MHz, CDCl₃) *δ* 173.1, 153.4, 139.8, 126.4, 125.0, 114.0, 112.7, 84.3, 75.2, 67.3, 60.4, 45.6, 30.9, 24.6, 14.2, 12.3; ESI-LRMS (+) m/z 304 [M+H]⁺; ESI-HRMS (+) calcd for [C₁₈H₂₆NO₃]⁺ 304.1913, found 304.1924.

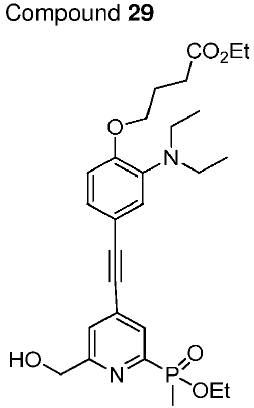

Compound **28** (528 mg, 1.74 mmol) and ethyl (6-(hydroxymethyl)-4-(bromopyridin-2-yl)(methyl)phosphinate **19** (486 mg, 1.65 mmol) were combined in anhydrous CH₃CN (10 mL) under argon. To this solution was added Pd₂Cl₂(allyl)₂ (70 mg, 0.191 mmol), P(^{t}Bu)₃ (0.06 mL, 0.25 mmol) and piperidine (0.43 mL, 4.70 mmol) in that order. The resulting mixture was stirred at 40°C under argon for 36 h before removing the solvent under reduced pressure. The residue was dissolved in CH₂Cl₂ (40 mL), washed with H₂O (3 x 40 mL) and dried over K₂CO₃ then the solvent was removed under reduced pressure. The crude product was purified by reverse phase HPLC (10 to 100% CH₃CN in H₂O over 10 min, *t*ᵣ = 12.2 min) to yield a pale orange oil (359 mg, 40%); ¹H-NMR (400 MHz, CDCl₃) *δ* 8.04 (d, ³*J*_{H-P} = 6.0, 1H), 7.47 (s, 1H), 7.13 (d, ³*J*_{H-H} = 8.4, 1H), 7.07 (s, 1H), 6.82 (d, ³*J*_{H-H} = 8.4, 1H), 4.81 (s, 2H), 4.22 - 3.78 (m, 6H), 3.17 (q, ³*J*_{H-H} = 6.9, 4H), 2.53 (t, ³*J*_{H-H} = 7.2, 2H), 2.22 - 2.11 (m, 2H), 1.78 (d, ²*J*_{H-P} = 15, 3H), 1.31 - 1.22 (m, 6H), 1.06 (t, ³*J*_{H-H} = 6.9, 6H); ¹³C-NMR (101 MHz, CDCl₃) *δ* 173.0, 160.4 (d, ³*J*_{C-P} = 19), 153.9, 153.1 (d, ¹*J*_{C-P} = 155), 140.0, 133.2 (d, ³*J*_{C-P} = 11), 128.3 (d, ²*J*_{C-P} = 22), 126.5, 124.8, 124.0 (d, ⁴*J*_{C-P} = 3), 113.6, 112.8, 97.1, 84.7, 67.4, 64.0, 61.2 (d, ²*J*_{C-P} = 6.2), 60.5, 45.6, 30.8, 24.6, 16.5 (d, ³*J*_{C-P} = 6.0), 14.2, 13.5 (d, ¹*J*_{C-P} = 105), 12.3; ³¹P{¹H}-NMR (162 MHz, CDCl₃) *δ* +39.4; ESI-LRMS (+) *m*/*z* 517 [M+H]⁺; ESI-HRMS (+) calcd for [C₂₇H₃₈N₂O₆P]⁺ 517.2468, found 517.2451.

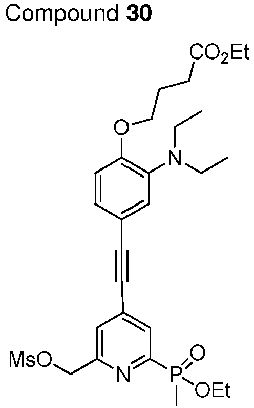

Compound **29** (250 mg, 0.484 mmol), methanesulfonic anhydride (126 mg, 0.72 mmol) and DIPEA (0.25 mL, 1.44 mmol) were combined in anhydrous THF (2 mL) under argon. The reaction mixture was stirred at RT for 1 h, monitoring the progress of the reaction by TLC. Following complete conversion, the solvent was removed under reduced pressure and the resulting crude residue was dissolved in CH₂Cl₂ (40 mL), washed with water (3 x 40 mL) and the organic layer was dried over K₂CO₃. Removal of solvent under reduced pressure afforded an orange oil (288 mg, quant.); ¹H-NMR (400 MHz, CDCl₃) *δ* 8.10 (d, ³*J*_{H-P} = 6.0, 1H), 7.64 (s, 1H), 7.15 (d, ³*J*_{H-H} = 8.4, 1H), 7.09 (s, 1H), 6.83 (d, ³*J*_{H-H} = 8.4, 1H), 5.37 (s, 2H), 4.19 - 3.82 (m, 6H), 3.18 (q, ³*J*_{H-H} = 7.0, 4H), 3.14 (s, 3H), 2.54 (t, ³*J*_{H-H} = 7.2, 2H), 2.22 - 2.13 (m, 2H), 1.78 (d, ²*J*_{H-P} = 15, 3H), 1.32 - 1.24 (m, 6H), 1.07 (t, ³*J*_{H-H} = 6.9, 6H); ESI-LRMS (+) *m*/*z* 595 [M+H]⁺; ESI-HRMS (+) calcd for [C₂₈H₄₀N₂O₈SP]⁺ 595.2242, found 595.2236; R_{f} = 0.4 (SiO₂, 5% CH₃OH in CH₂Cl₂).

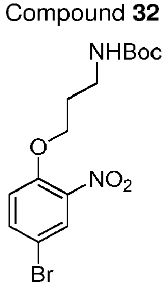

To a solution of 4-bromo-2-nitrophenol **22** (10 g, 45 mmol) in anhydrous CH₃CN (240 mL), under inert atmosphere, were added 3-(Boc-amino)propyl bromide **31** (14.1 g, 58.4 mmol), Cs₂CO₃ (22.2 g, 67.4 mmol) and NaI (1.7 g, 11.2 mmol). The reaction mixture was stirred at 70°C for 16 h, filtered and washed with CH₃CN. The mother liquors were concentrated under reduced pressure. Purification by chromatography over silica gel (Cyclohexane/EtOAc 8/2 then 7/3 then 6/4) led to compound **32** (15.9 g, 94%) as a yellow oil which solidified upon standing; ¹H NMR (CDCl₃, 400MHz) *δ* (ppm) 7.98 (d, *J* = 2.5 Hz, 1H), 7.62 (dd, *J* = 8.9 Hz, *J* = 2.5 Hz, 1H), 6.98 (d, *J* = 8.9Hz, 1H), 4.95 (br s, 1H), 4.15 (t, *J* = 5.9 Hz, 2H), 3.36-3.32 (m, 2H), 2.06-2.01 (m, 2H), 1.43 (s, 9H);¹³C NMR (CDCl₃, 100MHz) *δ* (ppm) 156.3, 151.6, 140.2, 137.1, 128.6, 116.2, 112.2, 79.4, 68.3, 38.0, 29.3, 28.5.

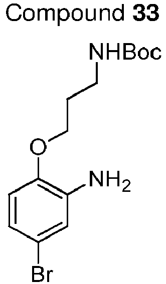

To a solution of compound **32** (15.9 g, 42.4 mmol) in dioxane (133 mL) and H₂O (26 mL) were added zinc (14.1 g, 211 mmol) and, at 0°C, NH₄Cl (11.4 g, 211 mmol). After 15 min at 0°C, the reaction mixture was stirred at 50°C for 20 h. It was then filtered over Celite^{®} and washed with EtOAc. The organic layer was successively washed with H₂O and brine, dried over Na₂SO₄, filtered and concentrated under reduced pressure to give crude compound **33** (14.4 g, 99%) as a brown oil; ¹H NMR (CDCl₃, 400MHz) *δ* (ppm) 6.84-6.81 (d, *J* = 2.3 Hz, 1H), 6.79-6.76 (dd, *J* = 2.3 Hz, *J* = 8.5 Hz, 1H), 6.62 (d, *J* = 8.5 Hz, 1H), 4.69 (br s, 1H), 4.02 (t, *J* = 6.0 Hz, 2H), 3.70 (br s, 2H), 3.34-3.31 (m, 2H), 2.01 -1.96 (m, 2H), 1.44 (s, 9H); ¹³C NMR (CDCl₃, 100MHz) *δ* (ppm) 156.1, 145.2, 138.1, 120.7, 117.6, 113.6, 113.0, 79.5, 66.3, 37.9, 29.8, 28.5; ESI-LRMS (+) *m*/*z* (M)⁺ 344.49.

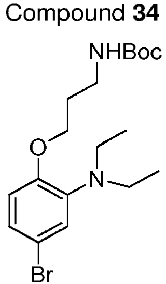

In a sealed flask, to a solution of compound **33** (7.3 g, 21.1 mmol) in CH₃CN (23 mL) was added K₂CO₃ (7.38 g, 52.9 mmol) and Etl (8.11 mL, 99.4 mmol). The reaction mixture was stirred at 70°C for 16 h, filtered over Büchner and washed with EtOAc. The mother liquors were washed with water, dried over Na₂SO₄, filtered and concentrated under reduced pressure. Purification by chromatography over silica gel (cyclohexane/EtOAc 8:2) led to compound **34** (7.04 g, 83%) as a slightly yellow oil; ¹H NMR (CDCl₃, 400MHz) *δ* (ppm) 7.07-7.03 (m, 2H), 6.70 (d, *J* = 8.9 Hz, 1H), 5.7 (br s, 1H), 4.03 (t, *J* = 5.8 Hz, 2H), 3.37-3.33 (m, 2H), 3.13 (q, *J* = 7.1 Hz, 4H), 2.00-1.95 (m, 2H), 1.45 (s, 9H), 1.01 (t, *J* = 7.1 Hz, 6H);¹³C NMR (CDCl₃, 100MHz) *δ* (ppm) 156.3, 152.7, 141.4, 125.4, 125.0, 114.6, 113.4, 79.0, 68.5, 46.2, 39.2, 29.6, 28.6, 12.0; ESI-LRMS (+) *m*/*z* (M+H)⁺ 403.0.

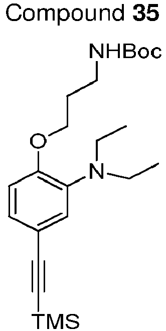

In a sealed flask, a solution of compound **34** (5.4 g, 13.5 mmol) in anhydrous THF (18.9 mL) was degassed with argon. Triethylamine (18.9 mL, 135 mmol), trimethylsilylacetylene (12.2 mL, 85.4 mmol), Cul (1.09 g, 5.73 mmol) and Pd(PPh₃)₂Cl₂ (2 g, 2.85 mmol) were then added and the mixture was degassed with argon and then stirred at 65°C for 16 h. The reaction mixture was filtered over Celite^{®}, washed with EtOAc and concentrated under reduced pressure. The residue was solubilized in EtOAc and the organic layer was successively washed with H₂O and brine, dried over Na₂SO₄, filtered and concentrated under reduced pressure. Purification by flash chromatography over silica gel (cyclohexane/EtOAc 10:0 to 8:2) led to compound **35** (6.4 g) with 80% purity. A second purification by flash chromatography over silica gel (CH₂Cl₂/EtOAc 100:0 to 95:5) led to compound **35** (3.5 g, 61%) as an orange oil; ¹H NMR (CDCl₃, 400MHz) *δ* (ppm) 7.12 (d, *J* = 8.3 Hz, 1H), 7.08 (s, 1H), 6.74 (d, *J* = 8.3 Hz, 1H), 5.96 (br s, 1H), 4.08-4.03 (t, *J* = 5.7 Hz, 2H), 3.37-3.32 (m, 2H), 3.15-3.08 (q, *J* = 7.0 Hz, 4H), 2.01-1.96 (m, 2H), 1.45 (s, 9H), 0.99 (t, *J* = 7.1 Hz, 6H), 0.24 (s, 9H); ¹³C NMR (CDCl₃, 100MHz) *δ* (ppm) 156.4, 154.4, 139.6, 127.6, 125.9, 115.6, 112.7, 105.8, 92.1, 79.0, 68.4,46.4, 39.3, 29.7, 28.7, 12.1, 0.2; ESI-LRMS (+) *m*/*z* (M+H)⁺ 419.15.

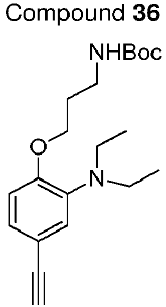

Triethylamine trihydrofluoride (1.1 mL, 6.75 mmol) was added to compound **35** (185 mg, 0.442 mmol) in anhydrous THF (5 mL) under argon. The solution was stirred at 30°C for 38 h before removing the solvent under reduced pressure. The residue was subsequently dissolved in CH₂Cl₂ (30 mL) and washed with water (3 x 30 mL). The combined aqueous layers were extracted with CH₂Cl₂ (2 x 30 mL) and the combined organic layers were dried over K₂CO₃ to yield a pale yellow oil (74 mg, 48%). The product was used in the next step without further purification; ¹H-NMR (600 MHz, CDCl₃) *δ* 7.13 (d, ³*J*_{H-H} = 8.2, 1H), 7.10 (s, 1H), 6.76 (d, ³*J*_{H-H} = 8.2, 1H), 4.06 (t, ³*J*_{H-H} = 5.7, 2H), 3.40 - 3.31 (m, 2H), 3.12 (q, ³*J*_{H-H} = 7.0, 4H), 2.98 (s, 1H), 2.02 - 1.95 (m, 2H), 1.45 (s, 9H), 0.99 (t, ³*J*_{H-H} = 7.0, 6H); ¹³C-NMR (151 MHz, CDCl₃) *δ* 156.4, 154.5, 139.6, 127.5, 125.9, 114.4, 112.7, 84.3, 79.0, 75.5, 68.4, 46.3, 39.3, 29.7, 28.6, 12.0; ESI-LRMS (+) *m*/*z* 347 [M+H]⁺; ESI-HRMS (+) calcd for [C₂₀H₃₁N₂O₃]⁺ 347.2353, found 347.2350.

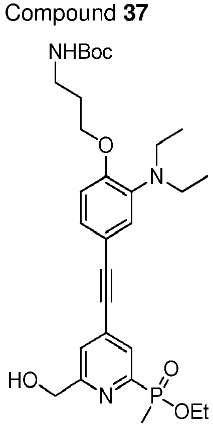

Compound **36** (172 mg, 0.496 mmol) and ethyl (6-(hydroxymethyl)-4-(bromopyridin-2-yl)(methyl)phosphinate **6** (139 mg, 0.473 mmol) were combined in anhydrous CH₃CN (3 mL) under argon. To this solution was added Pd₂Cl₂(allyl)₂ (20 mg, 0.055 mmol), P(tBu)₃ (0.02 mL, 0.082 mmol) and piperidine (0.12 mL, 1.22 mmol) in that order. The resulting mixture was stirred at 35°C under argon for 24 h before removing the solvent under reduced pressure. The residue was dissolved in CH₂Cl₂ (40 mL), washed with H₂O (3 x 40 mL) and dried over K₂CO₃. Removal of the solvent under reduced pressure afforded a brown oil which was purified by reverse phase HPLC (10 to 100% CH₃CN over 10 min, *t*ᵣ = 11.7 min) to give a pale yellow oil (64 mg, 24%); ¹H-NMR (700 MHz, CDCl₃) *δ* 8.03 (d, ³*J*_{H-P} = 6.0, 1H), 7.50 (s, 1H), 7.17 (d, ³*J*_{H-H} = 8.2, 1H), 7.12 (s, 1H), 6.81 (d, ³*J*_{H-H} = 8.2, 1H), 5.83 (br s, 1H), 4.80 (s, 2H), 4.14 - 4.06 & 3.89 - 3.82 (m, 2H), 4.08 (t, ³*J*_{H-H} = 5.8, 2H), 3.38 - 3.32 (m, 2H), 3.15 (q, ³*J*_{H-H} = 7.0, 4H), 2.03 - 1.97 (m, 2H), 1.77 (d, ²*J*_{H-P} = 15, 3H), 1.44 (s, 9H), 1.26 (t, ³*J*_{H-H} = 7.1, 3H), 1.01 (t, ³*J*_{H-H} = 7.0, 6H); ¹³C-NMR (176 MHz, CDCl₃) *δ* 160.8 (d, ³*J*_{C-P} = 19), 156.4, 155.0, 153.3 (d, ¹*J*_{C-P} = 155), 139.8, 133.2 (d, ³*J*_{C-P} = 11), 128.3 (d, ²*J*_{C-P} = 22), 127.5, 125.7, 124.1 (d, 4*J*_{C-P} = 3), 113.9, 112.8, 96.9, 85.0 (d, ⁴*J*_{C-P} = 2), 79.1, 68.4, 64.2, 61.3 (d, ²*J*_{C-P} = 7), 46.2, 39.2, 29.6, 28.6, 16.5 (d, ³*J*_{C-P} = 6), 13.5 (d, ¹*J*_{C-P} = 105), 12.1; ³¹P{¹H}-NMR (162 MHz, CDCl₃) *δ* +39.5; ESI-LRMS (+) *m*/*z* 560 [M+H]⁺; ESI-HRMS (+) calcd for [C₂₉H₄₃N₃O₆P]⁺ 560.2889, found 560.2879. 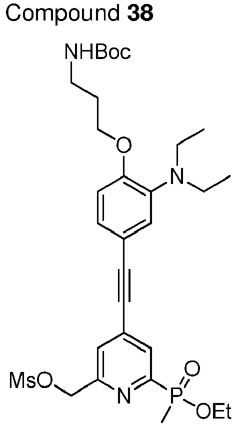 Compound **37** (40 mg, 0.072 mmol), methanesulfonic anhydride (25 mg, 0.143 mmol) and DIPEA (0.025 mL, 0.025 mmol) were combined in anhydrous THF (2 mL) under argon. The reaction mixture was stirred at RT for 90 min before removing the solvent under reduced pressure. The resulting residue was dissolved in CH₂Cl₂ (40 mL), washed with water (3 x 40 mL), and the combined aqueous layers were extracted with CH₂Cl₂ (2 x 40 mL). The combined organic layers were dried over K₂CO₃ and the solvent was removed under reduced pressure to give a yellow oil (45 mg, 100%); ¹H-NMR (400 MHz, CDCl₃) *δ* 8.09 (dd, ³*J*_{H-P} = 6.0, ⁴*J*_{H-H} = 1.4, 1H), 7.63 (s, 1H), 7.19 (dd, ³*J*_{H-H} = 8.2, ⁴*J*_{H-H} = 1.6, 1H), 7.14 (d, ⁴*J*_{H-H} = 1.6, 1H), 6.82 (d, ³*J*_{H-P} = 8.2, 1H), 5.86 (br s, 1H), 5.36 (s, 2H), 4.18 - 4.02 & 3.94 - 3.80 (m, 4H), 3.40 - 3.31 (m, 2H), 3.19 - 3.12 (m, 7H), 2.03 - 1.99 (m, 2H), 1.77 (d, ²*J*_{H-P} = 15, 3H), 1.44 (s, 9H), 1.27 (t, ³*J*_{H-H} = 7.1, 3H), 1.04 - 1.00 (m, 6H); ESI-LRMS (+) *m*/*z* 638 [M+H]⁺; ESI-HRMS (+) calcd for [C₃₀H₄₅N₃O₈SP]⁺ 638.2665, found 638.2676.

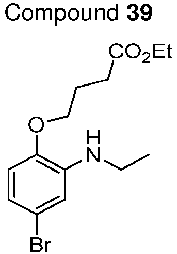

To compound **24** (3.50 g, 11.6 mmol) and K₂CO₃ (4.8 g, 34.5 mmol) in anhydrous CH₃CN under argon (3 mL) was added iodoethane (1.21 mL, 15.0 mmol). This mixture was heated to 55°C for 70 h and filtered to remove the inorganic salts, followed by removal of the solvent under reduced pressure. The resulting oil was purified by column chromatography (SiO₂, 100% CH₂Cl₂) to afford a pale yellow oil (1.65 g, 43%); ¹H-NMR (700 MHz, CDCl₃) *δ* 6.70 (dd, ³*J*_{H-H} = 8.3, 1H), 6.67 (s, 1H), 6.57 (d, ³*J*_{H-H} = 8.3, 1H), 4.15 (q, ³*J*_{H-H} = 7.1, 2H), 4.00 (t, ³*J*_{H-H} = 6.1, 2H), 3.13 (q, ³*J*_{H-H} = 7.2, 2H), 2.49 (t, ³*J*_{H-H} = 7.1, 2H), 2.18 - 2.11 (m, 2H), 1.29 (t, ³*J*_{H-H} = 7.2, 3H), 1.25 (t, ³*J*_{H-H} = 7.1, 3H); ¹³C-NMR (176 MHz, CDCl₃) *δ* 173.3, 145.0, 139.8, 118.4, 114.3, 112.7, 111.6, 67.6, 60.7, 38.2, 31.3, 24.7, 14.7, 14.4; ESI-LRMS (+) *m*/*z* 331 [M+H]⁺; ESI-HRMS (+) calcd for [C₁₄H₂₁NO₃Br]⁺ 330.0705, found 330.0714; R_{f} = 0.4 (SiO₂, neat CH₂Cl₂)

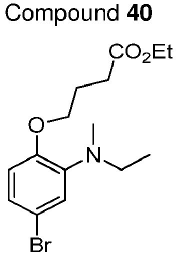

Compound **39** (939 mg, 2.84 mmol), paraformaldehyde (415 mg, 4.61 mmol) and a few drops of acetic acid were combined in anhydrous EtOH (25 mL). The mixture was stirred at RT under argon for 20 min, at which point NaBH₃CN was added (640 mg, 10 mmol) and the reaction stirred for a further 48 h. Following removal of the solvent under reduced pressure, the resulting residue was dissolved in CH₂Cl₂ (30 mL) and washed with a NaHCO₃ solution (1 x 30 mL) and water (3 x 30 mL). The organic layer was dried over K₂CO₃ and the solvent was removed under reduced pressure to yield a pale yellow oil (833 mg, 85%); ¹H-NMR (400 MHz, CDCl₃) *δ* 7.00 (dd, ³*J*_{H-H} = 8.3, ⁴*J*_{H-H} = 2.3, 1H), 6.98 (d, ⁴*J*_{H-H}= 2.3, 1H), 6.70 (d, ³*J*_{H-H} = 8.3, 1H), 4.16 (q, ³*J*_{H-H}= 7.1, 2H), 4.02 (t, ³*J*_{H-H}= 6.3, 2H), 3.13 (q, ³*J*_{H-H}= 7.2, 2H), 2.76 (s, 2H), 2.54 (t, ³*J*_{H-H}= 7.3, 2H), 2.17 - 2.10 (m, 2H), 1.27 (t, ³*J*_{H-H} = 7.1, 3H), 1.12 (t, ³*J*_{H-H} = 7.2, 3H); ¹³C-NMR (176 MHz, CDCl₃) *δ* 172.9, 150.6, 143.3, 123.9, 118.0, 113.8, 113.3, 67.4, 60.5, 49.2, 39.0, 30.8, 24.7, 14.2, 12.2; ESI-LRMS (+) m/z 344 [M+H]⁺. ESI-HRMS (+) calcd for [C₁₅H₂₃NO₃Br]⁺ 344.0861, found 344.0872.

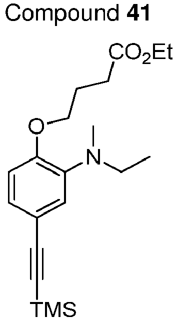

Compound **40** (1.47 g, 4.27 mmol) and Pd₂Cl₂(allyl)₂ (160 mg, 0.44 mmol) were combined in a vessel which was degassed then back-filled with argon in three cycles followed by the addition of anhydrous CH₃CN (8 mL). To this mixture was added P(*t*-Bu)₃ (0.16 mL, 0.66 mmol), trimethylsilylacetylene (1.2 mL, 8.66 mmol) and piperidine (1.0 mL, 10.1 mmol) in that order. The reaction mixture was stirred at 35°C for 19 h. The solvent was subsequently removed under reduced pressure and the residue dissolved in CH₂Cl₂ (50 mL). The solution was washed with H₂O (2 x 50 mL) and dried over K₂CO₃. Removal of the solvent under reduced pressure yielded a brown oil which was purified by column chromatography (SiO₂, 100% hexane to 7% EtOAc in hexane) to afford the expected compound as a pale orange oil (1.19 g, 77%); ¹H-NMR (600 MHz, CDCl₃) δ 7.05 (d, ³*J*_{H-H} = 8.2, 1H), 7.00 (s, 1H), 6.73 (d, ³*J*_{H-H} = 8.2, 1H), 4.14 (q, ³*J*_{H-H} = 7.1, 2H), 4.04 (t, ³*J*_{H-H} = 6.2, 2H), 3.09 (d, ³*J*_{H-H} = 7.0, 2H), 2.75 (s, 3H), 2.53 (t, ³*J*_{H-H} = 7.3, 2H), 2.20 - 2.13 (m, 2H), 1.25 (t, ³*J*_{H-H} = 7.1, 3H), 1.09 (t, ³*J*_{H-H} = 7.0, 3H), 0.24 (s, 9H); ¹³C-NMR (151 MHz, CDCl₃) *δ* 173.2, 152.3, 141.7, 126.4, 122.7, 115.4, 112.1, 105.9, 92.0, 67.3, 60.6, 49.5, 39.3, 31.0, 24.8, 14.4, 12.3, 0.2; ²⁹Si-NMR (139 MHz, CDCl₃) *δ* -18.3; ESI-LRMS (+) *m*/*z* 362 [M+H]⁺; ESI-HRMS (+) calcd for [C₂₆H₃₂NO₃Si]⁺ 362.2151, found 362.2162; R_{f} = 0.2 (SiO₂, 10% EtOAc in hexane).

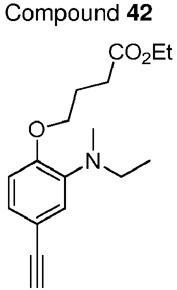

Triethylamine trihydrofluoride (3.6 mL, 22.0 mmol) was added to compound **41** (530 mg, 1.47 mmol) in anhydrous THF (5 mL) under argon. The solution was stirred at 30°C for 17 h before removing the solvent under reduced pressure. The residue was subsequently dissolved in CH₂Cl₂ (30 mL) and washed with water (3 x 30 mL). The combined aqueous layers were extracted with CH₂Cl₂ (2 x 30 mL) and the combined organic layers were dried over K₂CO₃ to yield a yellow oil (410 mg, 97%). The product was used in the next step without further purification; ¹H-NMR (600 MHz, CDCl₃) *δ* 7.07 (dd, ³*J*_{H-H} = 8.3, ⁴*J*_{H-H} = 2.0, 1H), 7.02 (d, ⁴*J*_{H-H} = 2.0, 1H), 6.75 (d, ³*J*_{H-H} = 8.3, 1H), 4.14 (q, ³*J*_{H-H} = 7.1, 2H), 4.04 (t, ³*J*_{H-H} = 6.3, 2H), 3.10 (q, ³*J*_{H-H} = 7.2, 2H), 2.97 (s, 1H), 2.75 (s, 3H), 2.53 (t, ³*J*_{H-H} = 7.3, 2H), 2.21 - 2.14 (m, 2H), 1.25 (t, ³*J*_{H-H} = 7.1, 3H), 1.10 (t, ³*J*_{H-H} = 7.2, 3H); ¹³C-NMR (151 MHz, CDCl₃) *δ* 173.2, 152.5, 141.8, 126.3, 122.8, 114.3, 112.2, 84.4, 75.4, 67.3, 60.6, 49.4, 39.2, 31.0, 24.8, 14.4, 12.4; ESI-LRMS (+) *m*/*z* 290 [M+H]⁺; ESI-HRMS (+) calcd for [C₁₇H₂₄NO₃]⁺ 290.1756, found 290.1752.

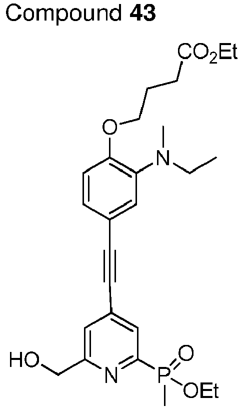

Compound **42** (396 mg, 1.37 mmol) and ethyl (6-(hydroxymethyl)-4-(bromopyridin-2-yl)(methyl)phosphinate **19** (362 mg, 1.23 mmol) were combined in anhydrous CH₃CN (10 mL) under argon. To this solution was added Pd₂Cl₂(allyl)₂ (50 mg, 0.137 mmol), P(^{t}Bu)₃ (0.05 mL, 0.21 mmol) and piperidine (0.4 mL, 4.37 mmol) in that order. The resulting mixture was stirred at 40°C under argon for 36 h before removing the solvent under reduced pressure. The residue was dissolved in CH₂Cl₂ (40 mL), washed with H₂O (3 x 40 mL) and dried over K₂CO₃ and the solvent was removed under reduced pressure to give an orange oil which was purified by reverse phase HPLC (10 to 100% CH₃CN over 10 min, *t*ᵣ = 13.2 min) to afford a pale orange oil (185 mg, 30%); ¹H-NMR (600 MHz, CDCl₃) *δ* 8.02 (d, ³*J*_{H-P} = 6.0, 1H), 7.47 (s, 1H), 7.12 (dd, ³*J*_{H-H} = 8.3, ⁴*J*_{H-H} = 2.0, 1H), 7.05 (d, ⁴*J*_{H-H} = 2.0, 1H), 6.80 (d, ³*J*_{H-H} = 8.3, 1H), 4.80 (s, 2H), 4.14 (q, ³*J*_{H-H} = 7.2, 2H), 4.12 - 4.08 & 3.90 - 3.83 (m, 2H), 4.07 (t, ³*J*_{H-H} = 6.4, 2H), 3.12 (q, ³*J*_{H-H} = 7.0, 2H), 2.78 (s, 3H), 2.53 (t, ³*J*_{H-H} = 7.3, 2H), 2.21 - 2.15 (m, 2H), 1.77 (d, ²*J*_{H-P} = 15, 3H), 1.27 (t, ³*J*_{H-H} = 7.0, 3H), 1.25 (t, ³*J*_{H-H} = 7.2, 3H), 1.12 (t, ³*J*_{H-H} = 7.0, 3H); ¹³C-NMR (151 MHz, CDCl₃) *δ* 173.1, 160.7 (d, ³*J*_{C-P} = 19), 153.3 (d, ¹*J*_{C-P} = 155), 153.1, 142.1, 133.3 (d, ³*J*_{C-P} = 11), 128.3 (d, ²*J*_{C-P} = 22), 126.4, 124.1 (d, ⁴*J*_{C-P} = 3), 122.6, 113.9, 112.3, 97.1, 84.9, 67.4, 64.2, 61.3 (d, ²*J*_{C-P} = 6), 60.6, 49.4, 39.2, 31.0, 24.7, 16.6 (d, ³*J*_{C-P} = 6), 14.3, 13.6 (d, ¹J_{C-P} = 104), 12.4; ³¹P{¹H}-NMR (162 MHz, CDCl₃) *δ* +39.5; ESI-LRMS (+) *m*/*z* 503 [M+H]⁺; ESI-HRMS (+) calcd for [C₂₆H₃₆N₂O₆P]⁺ 503.2311, found 503.2297.

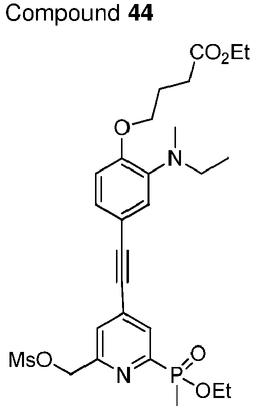

Compound **43** (160 mg, 0.318 mmol), methanesulfonic anhydride (111 mg, 0.637 mmol) and DIPEA (0.11 mL, 0.632 mmol) were combined in anhydrous THF (4 mL) under argon. The reaction mixture was stirred at RT for 1 h at which point the solvent was removed under reduced pressure. The resulting crude residue was dissolved in CH₂Cl₂ (40 mL), washed with water (3 x 40 mL) and the combined aqueous layers were extracted with CH₂Cl₂ (3 x 40 mL). The combined organic layers were dried over K₂CO₃ and the solvent was removed under reduced pressure to afford an orange oil (167 mg, 90%); ¹H-NMR (400 MHz, CDCl₃) *δ* 8.10 (d, ³*J*_{H-P} = 6.0, 1H), 7.64 (s, 1H), 7.15 (d, ³*J*_{H-H} = 8.4, 1H), 7.09 (s, 1H), 6.83 (d, ³*J*_{H-H} = 8.4, 1H), 5.37 (s, 2H), 4.19 - 3.82 (m, 6H), 3.18 (q, ³*J*_{H-H} = 7.0, 4H), 3.14 (s, 3H), 2.54 (t, ³*J*_{H-H} = 7.2, 2H), 2.22 - 2.13 (m, 2H), 1.78 (d, ²*J*_{H-P} = 15, 3H), 1.32 - 1.24 (m, 6H), 1.07 (t, ³*J*_{H-H} = 6.9, 6H); ESI-LRMS (+) *m*/*z* 595 [M+H]⁺; ESI-HRMS (+) calcd for [C₂₇H₃₈N₂O₈SP]⁺ 581.2087, found 581.2099; R_{f} = 0.4 (SiO₂, 5% CH₃OH in CH₂Cl₂).

Compounds **45a-d** are commercially available.

Compound **46a** was prepared according to the procedure described in Angew Chem Int Ed 2018, 57 5110 (see supplementary material) for the formation of N-Boc piperazine derivative.

Compound **46b** was prepared according to the procedure described in WO 2005/075410 for the formation of N-methyl piperazine derivative (see example 5).

Compound **46c** was prepared according to the procedure described in Journal Heterocyclic chemistry 2013, 50, 995.

Compound **46d** was prepared as the procedure described in Organic Biomolecular & Chemistry 2010, 8, 4077 (see supplementary material).

Compounds **47a-d** were prepared as described for compound **4.**

Compounds **48a-d** were prepared as described for compound **5.**

Compounds **49a-d** were prepared as described for compound **7.**

Compounds **50a-d** were prepared as described for compound **8.**

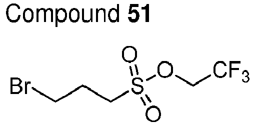

Oxalyl chloride (0.75 mL, 8.75 mmol) was added dropwise to a mixture of sodium salt of 3-bromopropanesulfonic acid (1 g, 4.44 mmol), anhydrous CH₃CN (4 mL) and anhydrous DMF (0.1 mL) cooled to 0°C under argon. The mixture was stirred at 0°C for 2 h before removing the solvent under reduced pressure (bath temperature <20°C). The resulting residue was dried under high vacuum. To this residue under argon was added anhydrous CH₃CN (4 mL) and 2,2,2-trifluoroethanol (9 mL, 125 mmol). Triethylamine (3 mL, 21.5 mmol) was added dropwise with cooling to 0°C. The mixture was stirred at 0°C for 5 h before removing the solvent under reduced pressure. Ethyl acetate (40 mL) and H₂O (40 mL) were added to the crude residue before washing the organic layer with H₂O (3 x 40 mL). The organic layer was dried over Na₂SO₄ and the solvent was removed under reduced pressure to yield a pale orange oil (1.1 g, 87%); ¹H-NMR (600 MHz, CDCl₃) *δ* 4.53 (q, 2H, ³*J*_{H-F} = 8.0), 3.54 (t, 2H, ³*J*_{H-H} = 6.2), 3.45 - 3.41 (m, 2H), 2.47 - 2.42 (m, 2H); ¹³C-NMR (151 MHz, CDCl₃) *δ* 122.0 (q, ¹*J*_{C-F} = 280), 64.0 (q, ²*J*_{C-F} = 40), 49.9, 30.1, 26.7; ¹⁹F-NMR (151 MHz, CDCl₃ *δ* -74.0.

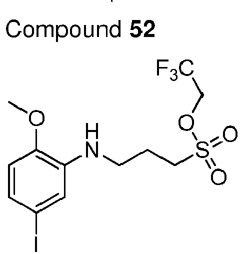

5-lodo-o-anisidine (245 mg, 0.984 mmol), compound **51** (1.1 g, 3.86 mmol), K₂CO₃ (425 mg, 3.08 mmol) and Nal (40 mg, 0.27 mmol) were combined in anhydrous CH₃CN (5 mL) under argon. The resulting mixture was heated to 78°C for 72 h before removing the solvent under reduced pressure. DCM (40 mL) and H₂O (40 mL) were added to the crude residue before washing the organic layer with H₂O (2 x 40 mL). The organic layer was dried over K₂CO₃ and the solvent was removed under reduced pressure to give a crude product as a pale brown residue which was purified by column chromatography (SiO₂, 100% hexane to 20% EtOAc in hexane) to yield a pale yellow oil (283 mg, 63%); ¹H-NMR (400 MHz, CDCl₃) *δ* 6.99 (dd, ³*J*_{H-H} = 8.3, ⁴*J*_{H-H} = 2.0, 1H), 6.82 (d, ⁴*J*_{H-H} = 2.0, 1H), 6.50 (d, ³*J*_{H-H} = 8.3, 1H), 4.53 (q, ³*J*_{H-F} = 8.0, 2H), 3.86 (s, 3H), 3.39 - 3.29 (m, 4H), 2.26 - 2.17 (m, 2H); ¹³C-NMR (101 MHz, CDCl₃) *δ* 146.9, 138.9, 126.0, 122.2 (q, ¹*J*_{C-F} = 280), 118.3, 111.5, 84.1, 63.9 (q, ²*J*_{C-F} = 40), 55.7, 49.1, 41.4, 23.5; ¹⁹F-NMR (376 MHz, CDCl₃) *δ* - 73.9; ESI-LRMS (+) *m*/*z* 454 [M+H]⁺; ESI-HRMS (+) calcd for [C₁₂H₁₆NO₄SF₃l]⁺ 453.9797, found 453.9792; R_{f} (SiO₂, 20% EtOAc in hexane) = 0.3.

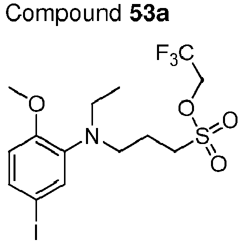

Compound **52** (310 mg, 0.684 mmol) and acetaldehyde (0.46 mL, 8.16 mmol) were combined in anhydrous EtOH (3 mL) under argon with two drops of glacial acetic acid. The resulting mixture was stirred at RT for 15 min before adding NaBH₃CN (170 mg, 2.71 mmol). The mixture was once more stirred at RT under argon for 16 h, after which the solvent was removed under reduced pressure to give a yellow residue. DCM (40 mL) and H₂O (40 mL) were added to this residue and the organic layer was washed with H₂O (3 x 40 mL). The combined aqueous layers were extracted with DCM (1 x 40 mL) and the combined organic layers were dried over K₂CO₃. Removal of the solvent under reduced pressure yielded a pale yellow oil (247 mg, 75%); ¹H-NMR (400 MHz, CDCl₃) *δ* 7.29 (dd, ³*J*_{H-H} = 8.4, ⁴*J*_{H-H} = 2.1, 1H), 7.18 (d, ⁴*J*_{H-H} = 2.1, 1H), 6.60 (d, ³*J*_{H-H} = 8.4, 1H), 4.46 (q, ³*J*_{H-F} = 8.0, 2H), 3.79 (s, 3H), 3.37 - 3.30 (m, 2H), 3.15 (t, ³*J*_{H-H} = 6.6, 2H), 3.07 (q, ³*J*_{H-H} = 7.1, 2H), 2.06 - 1.97 (m, 2H), 1.00 (t, ³*J*_{H-H} = 7.1, 3H); ¹³C-NMR (101 MHz, CDCl₃) *δ* 154.1, 140.2, 132.3, 130.9, 122.0 (q, ¹*J*_{C-F} = 280), 113.7, 82.9, 63.7 (q, ²*J*_{C-F} = 40), 55.4, 49.8, 49.0, 46.6, 21.5, 12.1; ¹⁹F-NMR (376 MHz, CDCl₃) *δ* -74.0; ESI-LRMS (+) *m*/*z* 482 [M+H]⁺; ESI-HRMS (+) calcd for [C₁₄H₂₀NO₄SF₃l]⁺ 482.0110, found 482.0106.

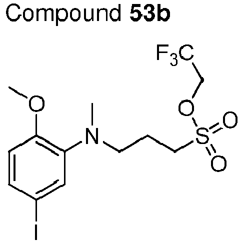

Compound **52** (269 mg, 0.594 mmol) and paraformaldehyde (800 mg, 8.89 mmol) were combined in anhydrous EtOH (8 mL) under argon with two drops of glacial acetic acid. The resulting mixture was stirred at RT for 15 min before adding NaBH₃CN (300 mg, 4.77 mmol). The mixture was once more stirred at RT under argon for 18 h, after which the solvent was removed under reduced pressure to give a yellow residue. DCM (40 mL) and H₂O (40 mL) were added to this residue and the organic layer was washed with H₂O (3 x 40 mL). The combined aqueous layers were extracted with DCM (2 x 40 mL) and the combined organic layers were dried over K₂CO₃. Removal of the solvent under reduced pressure yielded a pale yellow oil (249 mg, 90%); ¹H-NMR (400 MHz, CDCl₃) *δ* 7.27 (dd, ³*J*_{H-H} = 8.3, ⁴*J*_{H-H} = 2.0, 1H), 7.17 (d, ⁴*J*_{H-H} = 2.0, 1H), 6.59 (d, ³*J*_{H-H} = 8.3, 1H), 4.50 (q, ³*J*_{H-F} = 8.0, 2H), 3.81 (s, 3H), 3.44 - 3.37 (m, 2H), 3.11 (t, ³*J*_{H-H} = 6.7, 2H), 2.72 (s, 3H), 2.18 - 2.07 (m, 2H); ¹³C-NMR (176 MHz, CDCl₃) *δ* 152.6, 143.0, 131.8, 128.4, 122.2 (q, ¹*J*_{C-F} = 280), 113.5, 83.4, 63.8 (q, ²*J*_{C-F} = 40), 55.5, 53.1, 49.1, 39.5, 21.6; ¹⁹F-NMR (376 MHz, CDCl₃) *δ* -73.9; ESI-LRMS (+) *m*/*z* 468 [M+H]⁺; ESI-HRMS (+) calcd for [C₁₃H₁₈NO₄SF₃l]⁺ 467.9953, found 467.9952.

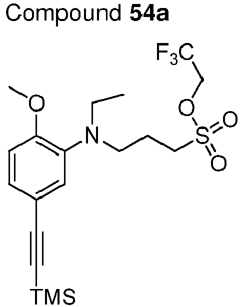

Compound **53a** (176 mg, 0.366 mmol) and trimethylsilylacetylene (0.1 mL, 0.72 mmol) were combined in anhydrous CH₃CN (3 mL) under argon. To this solution was added Pd₂Cl₂(allyl)₂ (14 mg, 0.038 mmol), P(^{t}Bu)₃ (0.015 mL, 0.062 mmol) and piperidine (0.09 mL, 0.91 mmol) in that order. The resulting mixture was stirred at 35°C under argon for 20 h before removing the solvent under reduced pressure to give a crude product as a brown residue which was purified by column chromatography (SiO₂, 100% hexane to 20% EtOAc in hexane) to yield a pale orange oil (151 mg, 91%); ¹H-NMR (400 MHz, CDCl₃) *δ* 7.16 (dd, ³*J*_{H-H} = 8.4, ⁴*J*_{H-H} = 2.0, 1H), 7.06 (d, ⁴*J*_{H-H} = 2.0, 1H), 6.77 (d, ³*J*_{H-H} = 8.4, 1H), 4.45 (q, ³*J*_{H-F} = 8.0, 2H), 3.83 (s, 3H), 3.38 - 3.30 (m, 2H), 3.16 (t, ³*J*_{H-H} = 6.5, 2H), 3.08 (q, ³*J*_{H-H} = 7.2, 2H), 2.06 - 1.94 (m, 2H), 0.99 (t, ³*J*_{H-H} = 7.2, 3H), 0.24 (s, 9H); ¹³C-NMR (101 MHz, CDCl₃) *δ* 154.9, 138.2, 128.3, 126.1, 122.0 (q, ¹*J*_{C-F}= 280), 115.3, 111.5, 105.3, 92.4, 63.8 (q, ²*J*_{C-F} = 40), 55.5, 49.9, 49.1, 46.9, 21.6, 12.2, 0.2; ¹⁹F-NMR (376 MHz, CDCl₃) *δ* -74.0; ESI-LRMS (+) *m*/*z* 452 [M+H]⁺; ESI-HRMS (+) calcd for [C₁₉H₂₉NO₄F₃SSi]⁺ 452.1539, found 452.1545; R_{f} (SiO₂, 20% EtOAc in hexane) = 0.3.

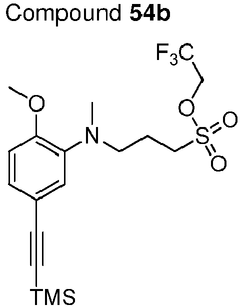

Compound **53b** (240 mg, 0.514 mmol) and trimethylsilylacetylene (0.14 mL, 1.01 mmol) were combined in anhydrous CH₃CN (2.5 mL) under argon. To this solution was added Pd₂Cl₂(allyl)₂ (20 mg, 0.055 mmol), P(*^{t}*Bu)₃ (0.013 mL, 0.054 mmol) and piperidine (0.13 mL, 1.32 mmol) in that order. The resulting mixture was stirred at 35°C under argon for 16 h before removing the solvent under reduced pressure to give a crude product as a brown residue that was purified by column chromatography (SiO₂, 100% hexane to 20% EtOAc in hexane) to afford a pale orange oil (157 mg, 70%); ¹H-NMR (700 MHz, CDCl₃) *δ* 7.13 (dd, ³*J*_{H-H} = 8.3, ⁴*J*_{H-H} = 1.8, 1H), 7.05 (d, ⁴*J*_{H-H} = 1.8, 1H), 6.76 (d, ³*J*_{H-H} = 8.3, 1H), 4.49 (q, ³*J*_{H-F} = 8.0, 2H), 3.84 (s, 3H), 3.42 - 3.38 (m, 2H), 3.10 (t, ³*J*_{H-H} = 6.7, 2H), 2.73 (s, 3H), 2.14 - 2.08 (m, 2H), 0.24 (s, 9H); ¹³C-NMR (151 MHz, CDCl₃) *δ* 153.2, 141.0, 127.5, 123.3, 122.2 (q, ¹*J*_{C-F} = 280), 115.6, 111.2, 105.4, 92.4, 63.8 (q, ²*J*_{C-F} = 40), 55.5, 53.1, 49.1, 39.6, 21.5, 0.2; ¹⁹F-NMR (376 MHz, CDCl₃) *δ* -74.0; ESI-LRMS (+) *m*/*z* 438 [M+H]⁺; ESI-HRMS (+) calcd for [C₁₈H₂₇NO₄F₃SSi]⁺ 438.1382, found 438.1377; R_{f} (SiO₂, 20% EtOAc in hexane) = 0.3.

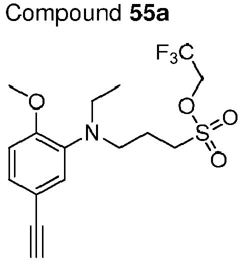

Triethylamine trihydrofluoride (3 mL, 6.75 mmol) was added to compound **54a** (341 mg, 0.755 mmol) in anhydrous THF (5 mL) under argon. The solution was stirred at 30°C for 72 h before removing the solvent under reduced pressure. The residue was dissolved in CH₂Cl₂ (30 mL) and washed with water (4 × 30 mL). The combined aqueous layers were extracted with CH₂Cl₂ (1 × 30 mL) and the combined organic layers were dried over K₂CO₃ to yield after solvent evaporation a pale brown oil (207 mg, 72%). This product was used in the next step without further purification; ¹H-NMR (400 MHz, CDCl₃) *δ* 7.18 (dd, ³*J*_{H-H} = 8.4, ⁴*J*_{H-H} = 2.0, 1H), 7.08 (d, ⁴*J*_{H-H} = 2.0, 1H), 6.80 (d, ³*J*_{H-H} = 8.4, 1H), 4.46 (q, ³*J*_{H-F} = 8, 2H), 3.84 (s, 3H), 3.38 - 3.31 (m, 2H), 3.17 (t, ³*J*_{H-H} = 6.6, 2H), 3.09 (q, ³*J*_{H-H} = 7.1, 2H), 2.99 (s, 1H), 2.05 - 1.95 (m, 2H), 0.99 (t, ³*J*_{H-H} = 7.1, 3H), ¹³C-NMR (101 MHz, CDCl₃) *δ* 155.0, 138.3, 128.2, 126.1, 122.2 (q, ¹*J*_{C-F} = 280), 114.2, 111.6, 83.9, 75.8, 63.8 (q, ²*J*_{C-F} = 40), 55.5, 49.9, 49.1, 46.7, 21.6, 12.2; ¹⁹F-NMR (376 MHz, CDCl₃) *δ* -74.0; ESI-LRMS (+) *m*/*z* 380 [M+H]⁺; ESI-HRMS (+) calcd for [C₁₆H₂₁NO₄SF₃]⁺ 380.1143, found 380.1155.

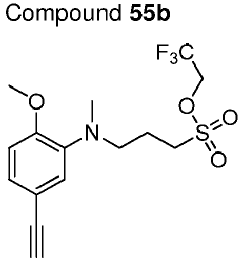

Triethylamine trihydrofluoride (1.1 mL, 6.75 mmol) was added to compound **54b** (195 mg, 0.446 mmol) in anhydrous THF (3 mL) under argon. The solution was stirred at 30°C for 48 h before removing the solvent under reduced pressure. The residue was dissolved in CH₂Cl₂ (30 mL) and washed with water (3 × 30 mL). The combined aqueous layers were extracted with CH₂Cl₂ (2 × 30 mL) and the combined organic layers dried over K₂CO₃ to give a pale brown oil (160 mg, 98%). This product was used in the next step without further purification; ¹H-NMR (400 MHz, CDCl₃) *δ* 7.15 (dd, ³*J*_{H-H} = 8.3, ⁴*J*_{H-H} = 1.9, 1H), 7.07 (d, ⁴*J*_{H-H} = 1.9, 1H), 6.78 (d, ³*J*_{H-H} = 8.3, 1H), 4.50 (q, ³*J*_{H-F} = 8.0, 2H), 3.85 (s, 3H), 3.43 - 3.37 (m, 2H), 3.10 (t, ³*J*_{H-H} = 6.6, 2H) 2.99 (s, 1H), 2.73 (s, 3H), 2.17 - 2.07 (m, 2H); ¹³C-NMR (101 MHz, CDCl₃) *δ* 153.3, 141.1, 127.4, 123.2, 122.2 (q, ¹*J*_{C-F} = 280), 114.4, 111.2, 83.9, 75.8, 63.8 (q, ²*J*_{C-F} = 40), 55.5, 53.0, 49.0, 39.4, 21.5; ¹⁹F-NMR (376 MHz, CDCl₃) *δ* -74.0; ESI-LRMS (+) *m*/*z* 366 [M+H]⁺; ESI-HRMS (+) calcd for [C₁₅H₁₉NO₄SF₃]⁺ 366.0987, found 366.0986.

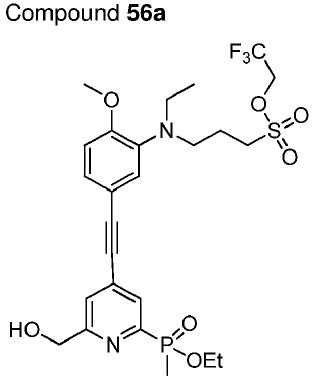

Compound **55a** (207 mg, 0.546 mmol) and ethyl (6-(hydroxymethyl)-4-(bromopyridin-2-yl)(methyl)phosphinate **6** (152 mg, 0.517 mmol) were combined in anhydrous CH₃CN (5 mL) under argon. To this solution was added Pd₂Cl₂(allyl)₂ (20 mg, 0.055 mmol), P(^{t}Bu)₃ (0.02 mL, 0.082 mmol) and piperidine (0.11 mL, 1.11 mmol) in that order. The resulting mixture was stirred at 35°C under argon for 18 h before removing the solvent under reduced pressure. The residue was dissolved in CH₂Cl₂ (40 mL), washed with H₂O (3 × 40 mL) and the combined aqueous layers were extracted with CH₂Cl₂ (2 x 40 mL). The combined organic layers were dried over K₂CO₃ and removal of the solvent under reduced pressure afforded an orange oil which was purified by reverse phase HPLC (10 to 100% CH₃CN over 10 min, *t*ᵣ = 11.3 min) to yield a pale yellow oil (111 mg, 36%); ¹H-NMR (400 MHz, CDCl₃) *δ* 8.02 (d, ³*J*_{H-P} = 6.0, 1H), 7.51 (s, 1H), 7.21 (dd, ³*J*_{H-H} = 8.4, ⁴*J*_{H-H} = 2.0, 1H), 7.11 (d, ⁴*J*_{H-H} = 2.0, 1H), 6.84 (d, ³*J*_{H-H} = 8.4, 1H), 4.81 (s, 2H), 4.46 (q, ³*J*_{H-F} = 8, 2H), 4.16 - 4.05 & 3.91 - 3.79 (m, 2H), 3.86 (s, 3H), 3.38 - 3.31 (m, 2H), 3.19 (t, ³*J*_{H-H} = 6.6, 2H), 3.11 (q, ³*J*_{H-H} = 7.1, 2H), 2.08 - 1.99 (m, 2H), 1.77 (d, ²*J*_{H-P} = 15, 3H), 1.31 - 1.14 (t, ³*J*_{H-H} = 7.0, 3H), 1.01 (t, ³*J*_{H-H} = 7.1, 3H); ¹³C-NMR (101 MHz, CDCl₃) *δ* 161.0 (d, ³*J*_{C-P} = 19), 155.6, 153.2 (d, ¹*J*_{C-P} = 155), 138.6, 133.1 (d, ³*J*_{C-P} = 11), 128.2 (d, ²*J*_{C-P} = 22), 128.1, 125.8, 124.1 (d, ⁴*J*_{C-P} = 3), 122.1 (q, ¹*J*_{C-F} = 280), 113.8, 111.8, 96.4, 85.2 (d, ⁴*J*_{C-P} = 2), 64.2, 63.7 (q, ²*J*_{C-F} = 40), 61.3 (d, ²*J*_{C-P} = 6), 55.6, 49.8, 49.1, 46.7, 21.6, 16.5 (d, ³*J*_{C-P} = 6), 13.5 (d, ¹*J*_{C-P} = 104) 12.2; ¹⁹F-NMR (376 MHz, CDCl₃) *δ* -74.0; ³¹P-NMR (162 MHz, CDCl₃) *δ* +39.5; ESI-LRMS (+) *m*/*z* 593 [M+H]⁺; ESI-HRMS (+) calcd for [C₂₆H₃₃N₂O₇SF₃P]⁺ 593.1698, found 593.1709.

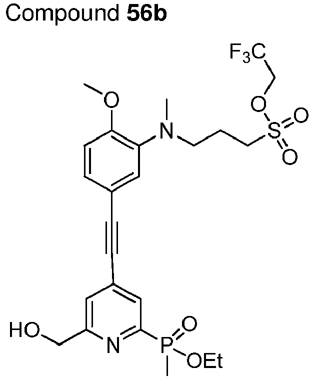

Compound **55b** (160 mg, 0.438 mmol) and ethyl (6-(hydroxymethyl)-4-(bromopyridin-2-yl)(methyl)phosphinate **6** (116 mg, 0.395 mmol) were combined in anhydrous CH₃CN (3 mL) under argon. To this solution was added Pd₂Cl₂(allyl)₂ (16 mg, 0.044 mmol), P(^{t}Bu)₃ (0.016 mL, 0.066 mmol) and piperidine (0.09 mL, 0.92 mmol) in that order. The resulting mixture was stirred at 35°C under argon for 16 h before removing the solvent under reduced pressure. The residue was dissolved in CH₂Cl₂ (40 mL), washed with H₂O (3 x 40 mL) and the combined aqueous layers extracted with CH₂Cl₂ (2 × 40 mL). The combined organic layers were dried over K₂CO₃ and removal of the solvent under reduced pressure afforded a brown oil which was purified by reverse phase HPLC (10 to 100% CH₃CN over 10 min, *t*ᵣ = 10.8 min) to afford a pale yellow oil (119 mg, 52%); ¹H-NMR (700 MHz, CDCl₃) *δ* 8.00 (d, ³*J*_{H-P} = 6.0, 1H), 7.50 (s, 1H), 7.18 (dd, ³*J*_{H-H} = 8.4, ⁴*J*_{H-H} = 2.0, 1H), 7.09 (d, ⁴*J*_{H-H} = 2.0, 1H), 6.82 (d, ³*J*_{H-H} = 8.4, 1H), 4.80 (s, 2H), 4.49 (q, ³*J*_{H-F} = 8.0, 2H), 4.13 - 4.06 & 3.91 - 3.82 (m, 2H), 3.86 (s, 3H), 3.42 - 3.38 (m, 2H), 3.12 (t, ³*J*_{H-H} = 6.3, 2H), 2.75 (s, 3H), 2.16 - 2.09 (m, 2H), 1.76 (d, ²*J*_{H-P} = 15, 3H), 1.26 (t, ³*J*_{H-H} = 7.1, 3H); ¹³C-NMR (101 MHz, CDCl₃) *δ* 161.0 (d, ³*J*_{C-P} = 19), 153.9, 153.3 (d, ¹*J*_{C-P} = 155), 141.4, 133.1 (d, ³*J*_{C-P} = 11), 128.2 (d, ²*J*_{C-P} = 22), 127.5, 124.1 (d, ⁴*J*_{C-P} = 3), 123.0, 122.2 (q, ¹*J*_{C-F} = 280), 114.0, 111.5, 96.4, 85.2 (d, ⁴*J*_{C-P} = 2), 64.2, 63.8 (q, ²*J*_{C-F} = 40), 61.3 (d, ²*J*_{C-P} = 7), 55.5, 53.0, 49.0, 39.4, 21.5, 16.5 (d, ³*J*_{C-P} = 6), 13.5 (d, ¹*J*_{C-P} = 104); ¹⁹F-NMR (376 MHz, CDCl₃) *δ* -73.9; ³¹P{¹H}-NMR (162 MHz, CDCl₃) *δ* +39.6; ESI-LRMS (+) *m*/*z* 579 [M+H]⁺; ESI-HRMS (+) calcd for [C₂₄H₃₁N₂O₇SF₃P]⁺ 579.1542, found 579.1543.

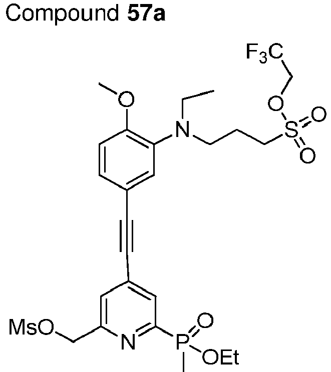

Compound **56a** (110 mg, 0.186 mmol), methanesulfonic anhydride (65 mg, 0.373 mmol) and DIPEA (0.065 mL, 0.373 mmol) were combined in anhydrous THF (3 mL) under argon. The reaction mixture was stirred at RT for 2 h before removing the solvent under reduced pressure. The resulting residue was dissolved in CH₂Cl₂ (40 mL), washed with water (3 x 40 mL) and the organic layer was dried over K₂CO₃. Removal of the solvent under reduced pressure afforded a yellow oil (125 mg, 100%); ¹H-NMR (400 MHz, CDCl₃) *δ* 8.09 (dd, ³*J*_{H-P} = 6.0, ⁴*J*_{H-H} = 1.3, 1H), 7.63 (s, 1H), 7.24 (dd, ³*J*_{H-H} = 8.4, ⁴*J*_{H-H} = 1.9, 1H), 7.13 (d, ⁴*J*_{H-H} = 1.9, 1H), 6.86 (d, ³*J*_{H-H} = 8.4, 1H), 5.36 (s, 2H), 4.46 (q, ³*J*_{H-F} = 8.0, 2H), 4.18 - 4.06 & 3.93 - 3.83 (m, 2H), 3.87 (s, 3H), 3.38 - 3.32 (m, 2H), 3.20 (t, ³*J*_{H-H} = 6.4, 2H), 3.15 - 3.09 (m, 5H), 2.09 - 1.99 (m, 2H), 1.77 (d, ²*J*_{H-P} = 15), 1.27 (t, ³*J*_{H-H} = 7.1, 3H), 1.02 (t, ³*J*_{H-H} = 7.0, 3H); ESI-LRMS (+) *m*/*z* 671 [M+H]⁺; ESI-HRMS (+) calcd for [C₂₆H₃₅N₂O₉S₂F₃P]⁺ 671.1474, found 671.1483.

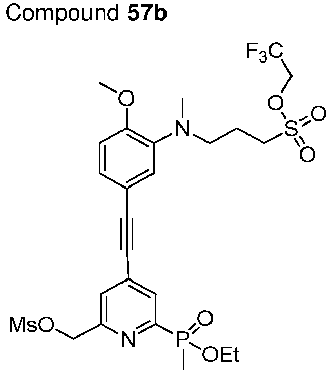

Compound **56b** (119 mg, 0.206 mmol), methanesulfonic anhydride (72 mg, 0.413 mmol) and DIPEA (0.072 mL, 0.413 mmol) were combined in anhydrous THF (3 mL) under argon. The reaction mixture was stirred at RT for 2 h before removing the solvent under reduced pressure. The resulting residue was dissolved in CH₂Cl₂ (40 mL), washed with water (3 x 40 mL) and the organic layer was dried over K₂CO₃. Removal of the solvent under reduced pressure afforded a clear colourless oil (88 mg, 65%); ¹H-NMR (400 MHz, CDCl₃) *δ* 8.08 (d, ³*J*_{H-P} = 6.0, 1H), 7.63 (s, 1H), 7.21 (dd, ³*J*_{H-H} = 8.3, ⁴*J*_{H-H} = 2.0, 1H), 7.11 (d, ⁴*J*_{H-H} = 2.0, 1H), 6.84 (d, ³*J*_{H-H} = 8.3, 1H), 5.36 (s, 2H), 4.50 (q, ³*J*_{H-F} = 8.0, 2H), 4.17 - 4.08 & 3.93 - 3.83 (m, 2H), 3.87 (s, 3H), 3.44 - 3.38 (m, 2H), 3.17 - 3.11 (m, 5H), 2.77 (s, 3H), 2.18 - 2.09 (m, 2H), 1.76 (d, ²*J*_{H-P} = 15, 3H), 1.27 (t, ³*J*_{H-H} = 7.1, 3H).

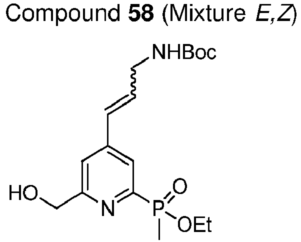

Compound 6 (74 mg, 0.25 mmol), *tert*-butyl N-allylcarbamate (155 mg, 0.99 mmol), palladium(II) acetate (11 mg, 0.05 mmol) and triphenylphosphine (20 mg, 0.08 mmol) were combined in toluene (2 mL) under an argon atmosphere. The reaction mixture was degassed by bubbling argon through the solution for 15 min. Triethylamine (0.3 mL, 2.2 mmol) was added and the mixture was heated to 80°C under argon for 18 h. After cooling, the solvent was removed under reduced pressure and the resulting residue was dissolved in CH₂Cl₂ (30 mL), washed with H₂O (4 × 30 mL) and dried over MgSO₄. Removal of the solvent under reduced pressure gave a crude product which was purified by reverse phase HPLC (10 to 100% CH₃CN in H₂O over 10 min, *t*ᵣ = 9.1 min) to yield a yellow oil as an *E*/*Z* isomeric mixture (93 mg, 63%); ¹H-NMR (400 MHz, CDCl₃) *δ* 8.03 - 7.91 (2 x d, 1H), 7.45 - 7.28 (2 x s, 1H), 6.61 - 6.44 (m, 2H), 4.83 - 4.77 (2 x s, 2H), 4.14 - 4.03 & 3.89 - 3.77 (m, 2H), 3.97 - 3.93 (m, 2H), 1.80 - 1.74 (2 x d, 3H), 1.48 - 1.40 (2 x t, 3H); ³¹P{¹H}-NMR (298 K, 162 MHz, CDCl₃) *δ* +40.5, +40.4; ESI-LRMS (+) *m*/*z* 371 [M+H]⁺; ESI-HRMS (+) calcd for [C₁₇H₂₈N₂O₅P]⁺ 371.1736, found 371.1746.

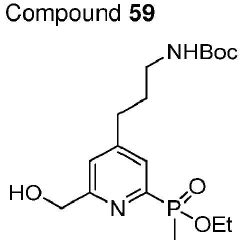

In a vessel, the *E*/*Z* isomeric mixture of compound **58** (210 mg, 0.57 mmol) was dissolved in EtOH (60 mL) to which Pd/C (Pd content 10%, 10 mg) was added. The vessel was then loaded onto a Parr hydrogenator (pressure 40 bar H₂) and the reaction mixture was stirred for 8 h. After this time, the catalyst was removed by filtration and the solvent removed under reduced pressure to yield a pale yellow oil (211 mg, quant.); ¹H-NMR (600 MHz, CDCl₃) *δ* 7.82 (d, ³*J*_{H-P} = 6.0, 1H), 7.24 (s, 1H), 4.78 (s, 2H), 4.66 (br s, 1H), 4.12 - 3.79 (m, 2H), 3.18 - 3.11 (m, 2H), 2.73 - 2.68 (m, 2H), 1.87 - 1.81 (m, 2H), 1.76 (d, ²*J*_{H-P} = 15, 3H), 1.43 (s, 9H), 1.26 (t, ³*J*_{H-H} = 7.0, 3H); ¹³C-NMR (151 MHz, CDCl₃) *δ* 160.5 (d, ³*J*_{C-P} = 19), 156.1, 153.0 (d, ¹*J*_{C-P} = 156), 152.2 (d, ³*J*_{C-P} = 10), 127.0 (d, ²*J*_{C-P} = 21), 123.0, 79.5, 64.1, 61.2 (d, ²*J*_{C-P} = 6), 40.0, 32.6, 30.8, 28.5, 16.6 (d, ³*J*_{C-P} = 6), 13.7 (d, ¹*J*_{C-P} = 104); ³¹P{¹H}-NMR (243 MHz, CDCl₃) *δ* +39.9; ESI-LRMS (+) *m*/*z* 373 [M+H]⁺; ESI-HRMS (+) calcd for [C₁₇H₃₀N₂O₅P]⁺ 373.1892, found 373.1880; R_{f} = 0.4 (SiO₂, 5% CH₃OH in CH₂Cl₂).

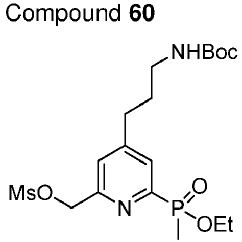

Compound **59** (25 mg, 0.067 mmol), methanesulfonic anhydride (20 mg, 0.115 mmol) and DIPEA (0.03 mL, 0.172 mmol) were combined in anhydrous THF (0.5 mL) under argon. The reaction mixture was stirred at RT for 1 h, with monitoring by TLC. Following complete conversion, the solvent was removed under reduced pressure and the resulting crude residue was dissolved in CH₂Cl₂ (40 mL), washed with water (3 × 40 mL) and the organic layer dried over K₂CO₃. Removal of the solvent under reduced pressure afforded a clear oil (30 mg, quant.); ¹H-NMR (400 MHz, CDCl₃) *δ* 7.86 (d, ³*J*_{H-P} = 6.0, 1H), 7.41 (s, 1H), 5.33 (s, 2H), 4.66 (br s, 1H), 4.15 - 3.77 (m, 2H), 3.19 - 3.09 (m, 5H), 2.76 - 2.67 (m, 2H), 1.89 - 1.79 (m, 2H), 1.74 (d, ²*J*_{H-P} = 15, 3H), 1.42 (s, 9H), 1.25 (t, ³*J*_{H-H} = 7.1, 3H); ESI-LRMS (+) *m*/*z* 373 [M+H]⁺; ESI-HRMS (+) calcd for [C₁₈H₃₂N₂O₇PS]⁺ 451.1668, found 451.1666; R_{f} = 0.5 (SiO₂, 5% CH₃OH in CH₂Cl₂).

Compounds **61**, **62** and **63** were prepared according to the procedure described in WO2018/229408 for the carboxylate derivatives.

Compound **64** was synthesized as described for compound **13.**

Complex **65** was synthesized as described for complex **14.**

Compound **66** was synthesized as described for compound **11.**

Compound **67** was synthesized as described for compound **12.**

Compound **68** was synthesized as described for compound **13.**

Complex **69** was synthesized as described for complex **14.**

Complex **70** was synthesized as described for complex **78a-b.**

Complex **71** was synthesized as described for complex **99a-b.**

Compound **72** was synthesized as described for compound **13.**

Complex **73a** was synthesized as described for complex **14.**

Complex **73b** was synthesized as described for complex **78a-b.**

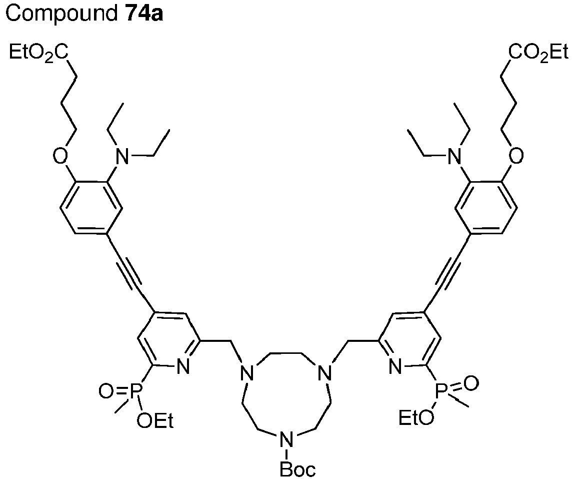

1-*tert*-Butoxycarbonyl-1,4,7-triazacyclononane dihydrochloride (66 mg, 0.218 mmol), mesylate **30** (288 mg, 0.484 mmol) and K₂CO₃ (122 mg, 0.833 mmol) were combined in anhydrous CH₃CN (2.5 mL) under argon. The resulting mixture was heated to 70°C for 16 h before cooling to RT. The solution was separated from the inorganic salts by filtration, and the solvent removed under reduced pressure to give the crude product as a viscous residue. This was purified by reverse phase HPLC (10 to 100% CH₃CN in H₂O over 10 min, *t*ᵣ = 17.5 min) to afford an orange oil (160 mg, 27%); ¹H-NMR (700 MHz, CDCl₃) *δ* 8.01 - 7.97 (m, 2H), 7.65 (s, 1H), 7.56 (s, 1H), 7.11 (d, 2H), 7.05 (s, 2H), 6.80 (d, 2H), 4.13 (q, 4H), 4.11 - 4.06 & 3.88 - 3.80 (m, 4H), 4.05 (t, 4H), 3.94 (s, 2H), 3.93 (s, 2H), 3.41 - 3.31 (m, 4H), 3.15 (q, 8H), 3.12 - 3.04 (m, 4H), 2.74 - 2.62 (m, 4H), 2.51 (t, 4H), 2.18 - 2.11 (m, 4H), 1.76 (d, ²*J*_{H-P} = 15), 1.75 (d, ²*J*_{H-P} = 15), 1.47 (s, 9H), 1.26 - 1.22 (m, 12H), 1.04 (t, 12H); ¹³C-NMR (176 MHz, CDCl₃) *δ* 173.1, 161.7 (d, ³*J*_{C-P} = 20), 161.4 (d, ³*J*_{C-P} = 20), 155.7, 154.1, 153.9 (d, ¹*J*_{C-P} = 157), 153.6 (d, ¹*J*_{C-P} = 157), 140.1, 132.8 (app. t, ³*J*_{C-P} = 11), 127.9 (app. t, ²*J*_{C-P} = 21), 126.5, 126.4 (d, ⁴*J*_{C-P} = 3), 126.2 (d, ⁴*J*_{C-P} = 3), 124.8, 114.0, 113.8, 112.8, 96.5, 96.3, 85.2, 85.1, 79.5, 67.5, 62.9, 62.6, 61.1 (d, ²*J*_{C-P} = 6.4), 61.0 (d, ²*J*_{C-P} = 6.4), 60.6, 56.1, 55.0, 54.7, 54.0, 50.0, 49.7, 45.7, 30.9, 28.8, 24.7, 16.6 (d), 14.3, 13.4 (d, ¹*J*_{C-P} = 104), 13.3 (d, ¹*J*_{C-P} = 104), 12.4; ³¹P{¹H}-NMR (283 MHz, CDCl₃) *δ* +40.1, +40.0; ESI-LRMS (+) *m*/*z* 1226 [M+H]⁺; ESI-HRMS (+) calcd for [C₆₅H₉₄N₇O₁₂P₂]⁺ 1226.644, found 1226.646.

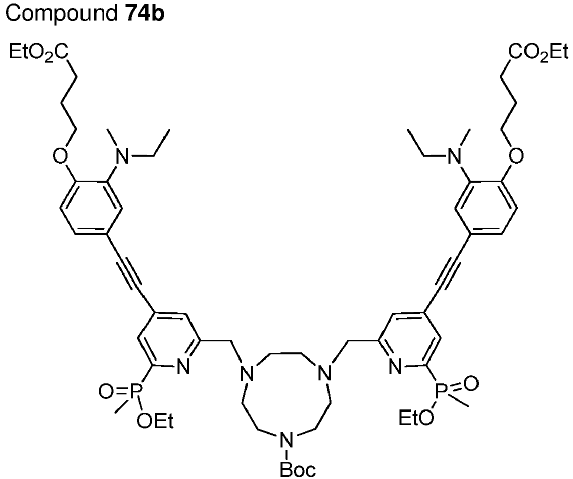

1-*tert*-Butoxycarbonyl-1,4,7-triazacyclononane dihydrochloride (38 mg, 0.126 mmol), mesylate **44** (167 mg, 0.288 mmol) and K₂CO₃ (104 mg, 0.753 mmol) were combined in anhydrous CH₃CN (4 mL) under argon. The resulting mixture was heated to 60°C for 48 h, before cooling to RT. The solution was separated from the inorganic salts by filtration, and the solvent removed under reduced pressure to yield the crude product. This was purified by reverse phase HPLC (10 to 100% CH₃CN in H₂O over 10 min, *t*ᵣ = 12.2 min) to afford a pale orange oil (110 mg, 73%); ¹H-NMR (400 MHz, CDCl₃) *δ* 8.00 (d, 2H), 7.65 (s, 1H), 7.59 (s, 1H), 7.12 (dd, 2H), 7.06 (s, 2H), 6.80 (d, 2H), 4.18 - 3.78 (m, 16H), 3.47 - 3.32 (m, 4H), 3.21 - 3.06 (m, 8H), 2.83 - 2.72 (m, 10H), 2.53 (t, 4H), 2.23 - 2.14 (m, 4H), 1.76 (d, 6H), 1.48 (s, 9H), 1.28- 1.22 (m, 12H), 1.11 (t, 6H); ³¹P{¹H}-NMR (162 MHz, CDCl₃) *δ* +40.2, +40.1; ESI-LRMS (+) *m*/*z* 400 [M+3H]³⁺, 600 [M+2H]²⁺, 1198 [M+H]⁺; ESI-HRMS (+) calcd for [C₆₃H₉₀N₇O₁₂P₂]⁺ 1198.612, found 1198.607;

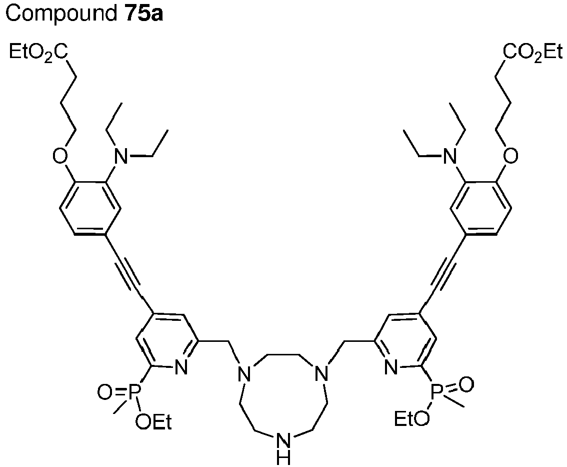

A solution of compound **74a** (19 mg, 0.016 mmol) in trifluoroacetic acid and CH₂Cl₂ was prepared (10% v/v, 3 mL total) and stirred under argon for 40 min. Immediately following this, the solvent was removed under reduced pressure. Additional CH₂Cl₂ (∼50 mL) was added and the solvent was removed under reduced pressure. This procedure was repeated 3 times. The residue was dissolved in CH₂Cl₂ (30 mL), washed with an aqueous Na₂CO₃ solution (4%, pH = 11, 3 × 30 mL) and dried over K₂CO₃. Removal of the solvent under reduced pressure yielded an orange oil (17 mg, quant.); ¹H-NMR (400 MHz, CDCl₃) *δ* 7.95 (dd, ³*J*_{H-P} = 6.0, ⁴*J*_{H-H} = 1.3, 2H), 7.38 (app. s, 2H), 7.12 (dd, ³*J*_{H-H} = 8.4, ⁴*J*_{H-H} = 2.0, 2H), 7.06 (d, ⁴*J*_{H-H} = 2.0, 2H), 6.81 (d, ³*J*_{H-H} = 8.4, 2H), 4.23 - 4.10 & 3.96 - 3.86 (m, 8H), 4.05 (t, ³*J*_{H-H} = 6.4, 4H), 3.99 (s, 4H), 3.31 - 3.24 (m, 4H), 3.16 (q, ³*J*_{H-H} = 7.1, 8H), 3.13 - 3.07 (m, 4H), 2.73 (br s, 4H), 2.52 (t, ³*J*_{H-H} = 7.2, 4H), 2.20 - 2.11 (m, 4H), 1.77 (d, ²*J*_{H-P} = 15, 6H), 1.31 - 1.22 (m, 12H), 1.05 (t, ³*J*_{H-H} = 7.1, 12H); ¹³C-NMR (101 MHz, CDCl₃) *δ* 173.2, 159.4 (d, ³*J*_{C-P} = 20), 154.6 (d, ¹*J*_{C-P} = 157), 154.2, 140.2, 133.4 (d, ³*J*_{C-P} = 12), 127.9 (d, ²*J*_{C-P} = 22), 126.7, 126.3, 124.8, 113.5, 112.8, 97.5, 84.7, 67.5, 61.3 (d, ²*J*_{C-P} = 6), 60.7, 60.0, 53.9, 51.6, 50.1, 49.4, 49.2, 45.7, 44.8, 31.0, 24.7, 16.7 (d, ³*J*_{C-P} = 6), 14.4, 13.8 (d, ¹*J*_{C-P} = 103), 12.5; ³¹P{¹H}-NMR (162 MHz, CDCl₃) *δ* +39.2; ESI-LRMS (+) *m*/*z* 1127 [M+H]⁺; ESI-HRMS (+) calcd for [C₆₀H₈₆N₇O₁₀P₂]⁺ 1126.592, found 1126.592.

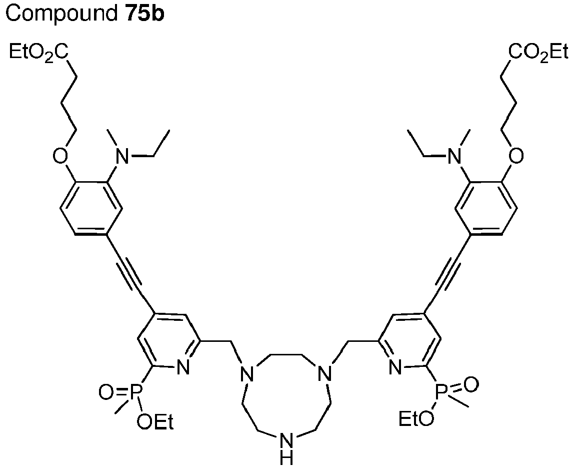

A solution of compound **74b** (110 mg, 0.092 mmol) in trifluoroacetic acid and CH₂Cl₂ was prepared (10% v/v, 3 mL total) and stirred under argon for 60 min. The solvent was removed under reduced pressure, additional CH₂Cl₂ (∼50 mL) was added and the solvent removed under reduced pressure. This process was repeated 4 times. The residue was dissolved in CH₂Cl₂ (30 mL), washed with an aqueous Na₂CO₃ solution (4%, pH = 11, 3 x 30 mL) and dried over K₂CO₃. Removal of the solvent under reduced pressure yielded an orange oil (111 mg, quant.); ¹H-NMR (700 MHz, CDCl₃) *δ* 7.89 (d, ³*J*_{H-P} = 6.0, 2H), 7.77 (s, 2H), 7.64 (dd, ³*J*_{H-H} = 8.5, ⁴*J*_{H-H} = 1.3, 2H), 7.57 (s, 2H), 7.13 (d, ³*J*_{H-H} = 8.5, 2H), 4.39 (s, 4H), 4.22 (t, ³*J*_{H-H} = 6.5, 4H), 4.15 - 4.07 & 3.97 - 3.90 (m, 8H), 3.68 (q, ³*J*_{H-H} = 7.2, 4H), 3.64 - 3.51 (m, 8H), 3.43 - 3.35 (m, 4H), 3.29 (s, 6H), 2.51 (t, ³*J*_{H-H} = 6.8, 4H), 2.21 - 2.16 (m, 4H), 1.75 (d, ²*J*_{H-P} = 15, 6H), 1.27 (td, , ³*J*_{H-H} = 7.1, ⁴*J*_{H-P} = 3.0, 6H), 1.23 (t, ³*J*_{H-P} = 7.1, 6H), 1.18 (t, ³*J*_{H-H} = 7.2, 6H); ¹³C-NMR (176 MHz, CDCl₃) *δ* 173.1, 156.2 (d, ³*J*_{C-P} = 21), 153.3 (d, ¹*J*_{C-P} = 160), 152.5, 135.6, 128.2, 128.1 (d, ²*J*_{C-P} = 20), 127.7, 127.1 (d, ⁴*J*_{C-P} = 4), 115.4, 114.2, 94.6, 86.2 (d, ⁴*J*_{C-P} = 2.6), 69.1, 62.6 (d, ²*J*_{C-P} = 7.0), 61.0, 59.7, 54.1, 53.6, 51.5, 50.0, 44.2, 30.5, 24.0, 16.3 (d, ³*J*_{C-P} = 6.2), 14.2, 13.4 (d, ¹*J*_{C-P} = 102), 10.3; ³¹P-NMR (162 MHz, CDCl₃) *δ* +40.8; ESI-LRMS (+) *m*/*z* 1098 [M+H]⁺; ESI-HRMS (+) calcd for [C₅₈H₈₂N₇O₁₀P₂]⁺ 1098.5598, found 1098.5573.

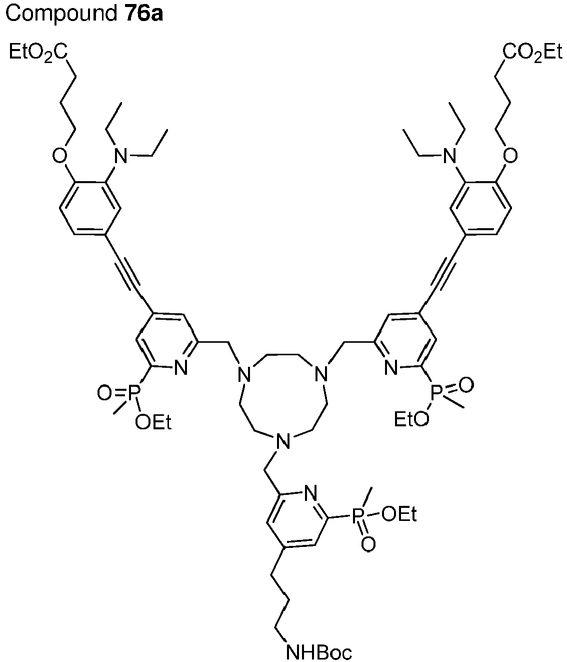

Compound **75a** (43 mg, 0.038 mmol), compound **60** (45 mg, 0.10 mmol) and K₂CO₃ (30 mg, 0.22 mmol) were combined in anhydrous CH₃CN (2 mL) under argon and heated to 70°C for 18 h. After this time, LCMS analysis confirmed the complete transformation of the macrocycle. The reaction mixture was allowed to cool before separating it from the inorganic salts by filtration. Removal of the solvent under reduced pressure afforded an orange solid (88 mg) which was used directly in the next step without further purification; ESI-LRMS (+) *m*/*z* 1481 [M+H]⁺; ESI-HRMS (+) calcd for [C₇₇H₁₁₃N₉O₁₄P₃]⁺ 1480.762, found 1480.760;

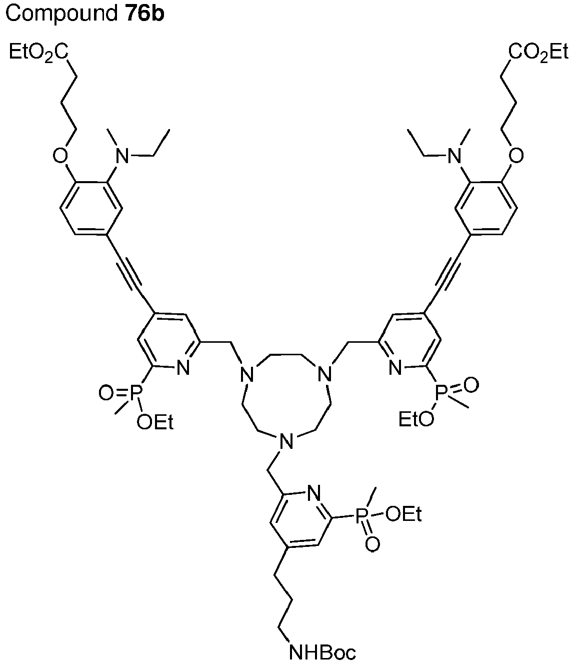

Compound **75b** (111 mg, 0.0919 mmol), compound **60** (59 mg, 0.130 mmol) and K₂CO₃ (45 mg, 0.33 mmol) were combined in anhydrous CH₃CN (3 mL) under argon and heated to 65°C for 16 h. After this time, LCMS analysis confirmed the complete transformation of the macrocycle. The reaction mixture was allowed to cool before separation of the solution from the inorganic salts by filtration. Removal of the solvent under reduced pressure afforded an orange oil (165 mg) which was used directly in the next step without further purification; ESI-LRMS (+) *m*/*z* 1453 [M+H]⁺; ESI-HRMS (+) calcd for [C₇₅H₁₀₉N₉O₁₄P₃]⁺ 1452.731, found 1452.729.

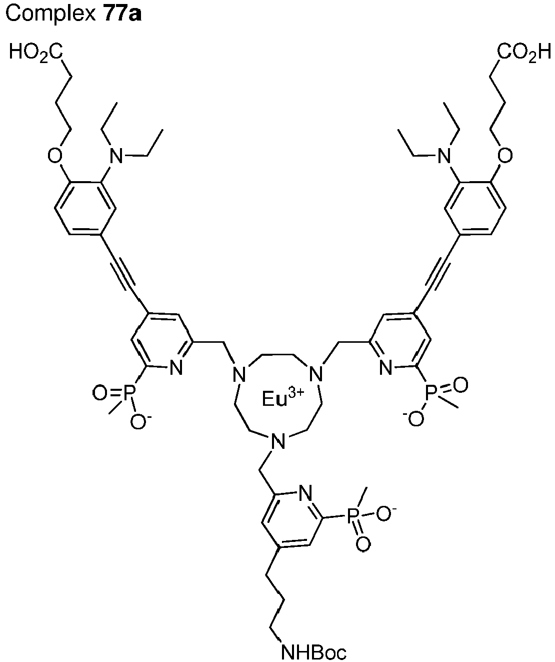

The crude ligand **76a** (88 mg) from the previous step was dissolved in a mixture of CH₃OH/H₂O (4:1, 2.5 mL total) and the pH was adjusted to 12 using an aqueous NaOH solution. The solution was heated to 60°C for 1.5 h. After this time, LCMS analysis confirmed complete hydrolysis of the phosphinate and chromophore head ester groups. After cooling and adjusting the pH to 7 using hydrochloric acid (0.1 M), EuCl₃.6H₂O (42 mg, 0.115 mmol) was added and the reaction mixture was heated to 60°C for 18 h. The reaction mixture was purified by reverse phase HPLC (10 to 100% CH₃CN in H₂O over 10 min, *t*ᵣ = 8.5 min) to yield a yellow solid (22 mg, 39% over three steps); ESI-LRMS (+) *m*/*z* 1490 [M+H]⁺, 746 [M+2H]²⁺; ESI-HRMS (+) calcd for [C₆₇H₉₁¹⁵¹EuN₉O₁₄P₃]²⁺ 745.7561, found 745.7562; *τ*_{H2O} (ms) = 0.27 (pH = 8), 0.39 (pH = 7), 0.53 (pH = 6), 0.95 (pH = 5), 0.97 (pH = 4).

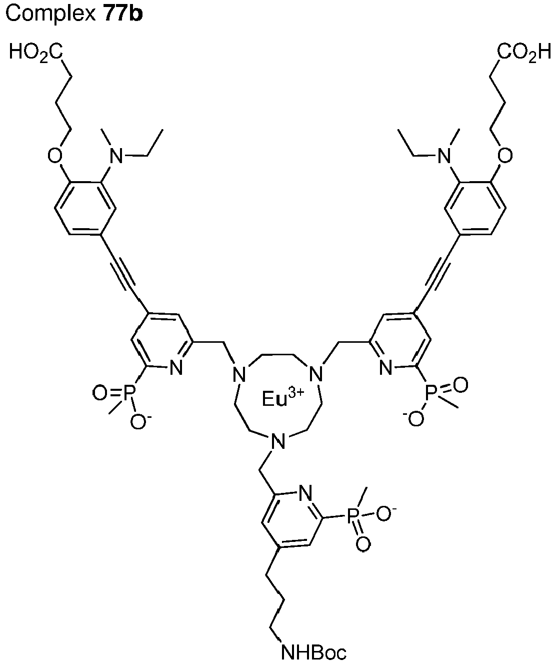

The crude ligand **76b** (165 mg) from the previous step was dissolved in a mixture of CH₃OH/H₂O (1:1, 2 mL total) and the pH was adjusted to 12 using an aqueous NaOH solution. The solution was heated to 60°C for 1.5 h. After this time, LCMS analysis confirmed complete hydrolysis of the phosphinate and chromophore head ester groups. After cooling and adjusting the pH to 7 using hydrochloric acid (0.1 M), EuCl₃.6H₂O (34 mg, 0.093 mmol) was added and the reaction mixture was heated to 60°C for 17 h. The reaction mixture was purified by reverse phase HPLC (10 to 100% CH₃CN in H₂O over 10 min, *t*ᵣ = 8.7 min) to yield a yellow solid (50 mg, 37% over three steps); ESI-LRMS (+) *m*/*z* 1462 [M+H]⁺, 732 [M+2H]²⁺; ESI-HRMS (+) calcd for [C₆₅H₈₆¹⁵¹EuN₉O₁₄P₃]⁺ 1462.473, found 1462.468.

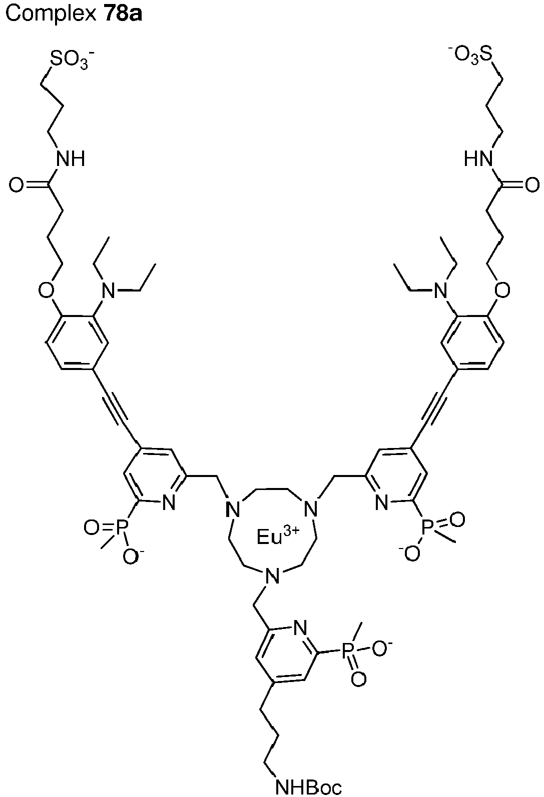

Complex **77a** (3.9 mg, 2.6 µmol) and DIPEA (6 µL, 34 µmol) were combined in anhydrous DMSO (0.4 mL) under argon. To this solution was added HATU (5 mg, 13 µmol), homotaurine (2 mg, 14 µmol) and water (40 µL). The mixture was stirred at RT for 19 h. Following dilution with water, the reaction mixture was purified by reverse phase HPLC (10 to 100% CH₃OH in H₂O over 10 min, *t*ᵣ = 11.6 min) yielding a yellow solid (4 mg, 89%); ESI-LRMS (-) *m*/*z* 1730 [M-H]⁻, 865 [M-2H]²⁻; ESI-HRMS (+) calcd for [C₇₃H₁₀₁¹⁵¹EuN₁₁O₁₈P₃S₂]²⁻ 864.7601, found 864.7610; *τ*_{H2O} (ms) = 0.25 (pH 8), 0.34 (pH 7), 0.70 (pH 6), 0.98 (pH 5), 1.04 (pH 4); *ε*_{330 nm} = 39000 L M⁻¹ cm⁻¹; *Φ*_{pH 8} = 0.3%, *Φ*_{pH 4} = 16% (*λ*_{exc} = 330 nm).

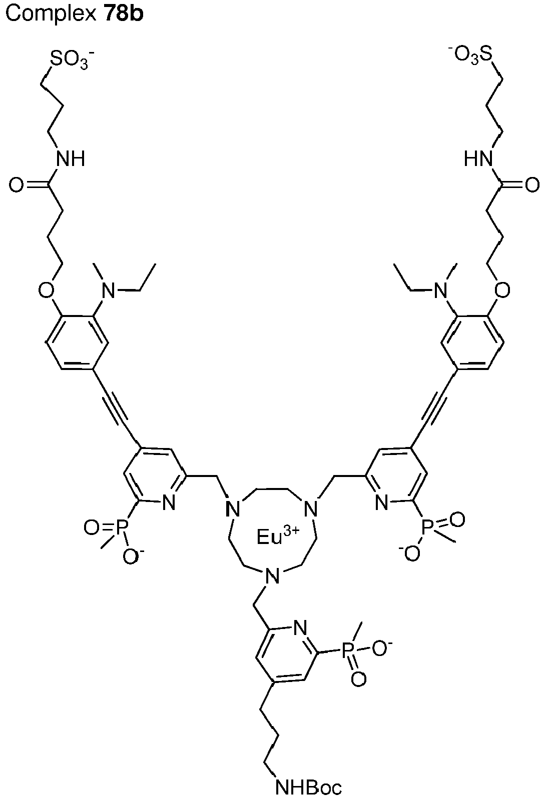

Complex **77b** (20 mg, 13.7 µmol) and DIPEA (15 µL, 85 µmol) were combined in anhydrous DMSO (0.5 mL) under argon. To this solution was added HATU (12 mg, 31 µmol), homotaurine (4.5 mg, 31 µmol) and water (50 µL), and the mixture was stirred at RT for 16 h. Following dilution with water, the reaction mixture was purified by reverse phase HPLC (10 to 100% CH₃OH in H₂O over 10 min, *t*ᵣ = 10.6 min) to yield a yellow solid (9 mg, 38%); ESI-LRMS (-) *m*/*z* 1701 [M-H]⁻, 850 [M-2H]²⁻ ESI-HRMS (+) calcd for [C₇₁H₉₉¹⁵¹EuN₁₁O₁₈P₃S₂]²⁺ 852.7530 found 852.7601; *τ*_{H2O} (ms) = 0.30 (pH 8), 0.29 (pH 7), 0.42 (pH 6), 0.77 (pH 5), 1.05 (pH 4); *ε*_{330 nm} = 39000 L M⁻¹ cm⁻¹; *Φ*_{pH 8} = 0.01%, *Φ*_{pH 4} = 14.5% (*λ*_{exc} = 330 nm).

Compound **80** was synthesized as described for compound **11.**

Compound **81** was synthesized as described for compound **12.**

Compound **82** was synthesized as described for compound **17.**

Complex **83** was synthesized as described for complex **18.**

Complex **84** was functionalized using the method described in WO 2004/113275.

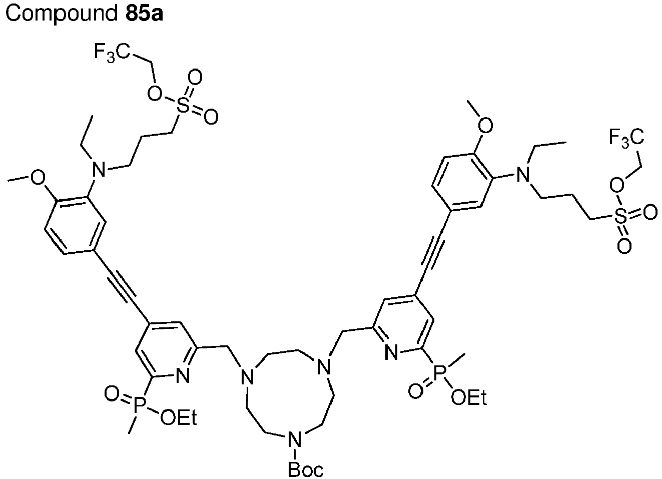

The dihydrochloride salt of 1-(*tert*-butoxycarbonyl)-1,4,7-triazacyclononane **9** (24 mg, 0.0794 mmol), mesylate **57a** (125 mg, 0.186 mmol) and K₂CO₃ (44 mg, 0.32 mmol) were combined in anhydrous CH₃CN (3 mL) under argon. The resulting mixture was heated to 65°C for 16 h before separating the crude solution from the inorganic salts and removing the solvent under reduced pressure to yield an orange oil which was purified by reverse phase HPLC (10 to 100% CH₃CN in H₂O with 0.1% formic acid over 10 min, *t*ᵣ = 13.5 min) to afford a pale yellow oil (88 mg, 80%); ¹H-NMR (700 MHz, CDCl₃) *δ* 8.01 (s, 2H), 7.66 (s, 1H), 7.61 (s, 1H), 7.22 (d, 2H), 7.12 (s, 2H), 6.84 (d, 2H), 4.46 (q, 4H), 4.17 - 3.77 (m, 14H), 3.46 - 3.31 (m, 8H), 3.25 - 3.04 (m, 12H), 2.87 - 2.65 (m, 4H), 2.11 - 1.98 (m, 4H), 1.77 (d, 6H), 1.48 (s, 9H), 1.25 (t, 6H), 1.01 (t, 6H); ¹³C-NMR (101 MHz, CDCl₃) *δ* 161.5 (d), 155.6 (d), 155.5, 153.1, 138.6, 132.7 (d), 128.1, 127.9 (d), 126.5 (d), 125.8, 122.1 (q), 113.9 (d), 111.8, 96.1, 85.4, 79.7, 63.8 (q) 62.5, 61.1 (d), 55.7, 55.6, 54.8, 54.4, 53.7, 49.8, 49.4, 49.1, 46.7, 46.0, 28.7, 21.6, 16.6 (d), 13.4 (2 × d), 12.2; ¹⁹F-NMR (376 MHz, CDCl₃) *δ* -74.0; ³¹P-NMR (162 MHz, CDCl₃) *δ* +40.1 (s); ESI-LRMS (+) *m*/*z* 1379 [M+H]⁺; ESI-HRMS (+) calcd for [C₆₁H₈₄F₆N₇O₁₄P₂S₂]⁺ 1378.490, found 1378.489.

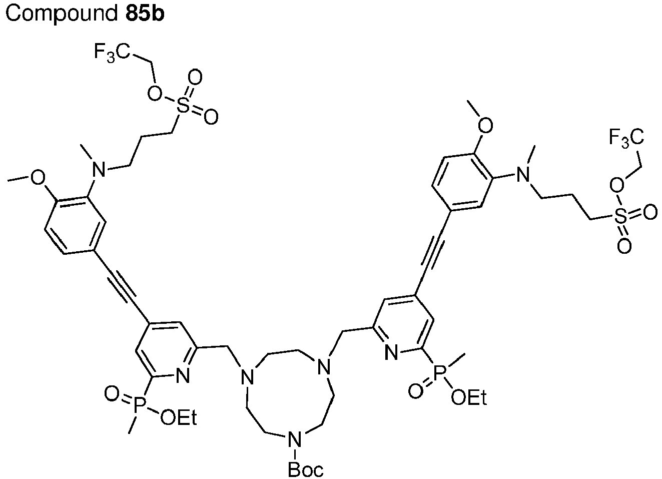

The dihydrochloride salt of 1-(*tert*-butoxycarbonyl)-1,4,7-triazacyclononane **9** (18 mg, 0.06 mmol), mesylate **57b** (88 mg, 0.134 mmol) and K₂CO₃ (35 mg, 0.25 mmol) were combined in anhydrous CH₃CN (3 mL) under argon. The resulting mixture was heated to 65°C for 18 h before separating the crude solution from the inorganic salts and removing the solvent under reduced pressure to yield an orange oil which was purified by reverse phase HPLC (10 to 100% CH₃CN in H₂O over 10 min, *t*ᵣ = 14.2 min) to afford a pale yellow oil (58 mg, 72%); ¹H-NMR (700 MHz, CDCl₃) *δ* 8.02 - 7.98 (m, 2H), 7.65 (s, 1H), 7.58 (s, 1H), 7.20 (d, 2H), 7.11 (s, 2H), 6.83 (d, 2H), 4.50 (q, 4H), 4.15 - 4.06 & 3.89 - 3.81 (m, 10H), 3.95 (2 × s, 4H), 3.44 - 3.32 (m, 8H), 3.16 - 3.05 (m, 8H), 2.76 (s, 6H), 2.73 - 2.65 (m, 4H), 2.17 - 2.10 (m, 4H), 1.76 (2 × d, 6H), 1.48 (s, 9H), 1.28 - 1.22 (m, 6H); ¹⁹F-NMR (376 MHz, CDCl₃) *δ* -73.9; ³¹P-NMR (162 MHz, CDCl₃) *δ* +40.1 (s); ESI-LRMS (+) *m*/*z* 1350 [M+H]⁺; ESI-HRMS (+) calcd for [C₅₉H₈₀F₆N₇O₁₄P₂S₂]⁺ 1350.458, found 1350.455.

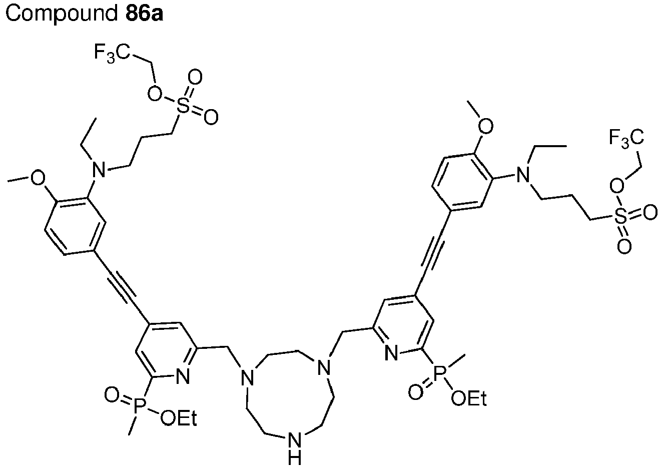

A solution of compound **85a** (88 mg, 0.064 mmol) in trifluoroacetic acid and CH₂Cl₂ was prepared (10% v/v, 3 mL total) and stirred under argon for 60 min. Immediately following this, the solvent was removed under reduced pressure. Additional CH₂Cl₂ (∼50 mL) was added and the solvent was removed under reduced pressure. This procedure was repeated 3 times. Drying under high vacuum for several hours yielded an orange oil (88 mg, quant.); ¹H-NMR (400 MHz, CD₃OD) *δ* 8.00 (d, ⁴*J*_{H-H} = 1.9, 2H), 7.98 (dd, ³*J*_{H-P} = 6.0, ⁴*J*_{H-H} = 1.1, 2H), 7.82 (dd, ³*J*_{H-H} = 8.8, ⁴*J*_{H-H} = 1.9, 2H), 7.75 - 7.74 (m, 2H), 7.42 (d, ³*J*_{H-H} = 8.8, 2H), 5.20 - 5.08 (m, 12H), 4.70 (q, ³*J*_{H-F} = 8.3, 4H), 4.43 (s, 4H), 4.16 - 3.90 (m, 10H), 3.88 - 3.80 (m, 4H), 3.75 (q, ³*J*_{H-H} = 7.1, 4H), 3.49 (t, ³*J*_{H-H} = 7.2, 4H), 2.04 - 1.94 (m, 4H), 1.80 (d, ²*J*_{H-P} = 15, 6H), 1.30 (t, ³*J*_{H-H} = 7.0, 6H), 1.13 (t, ³*J*_{H-H} = 7.1, 6H); ¹³C-NMR (101 MHz, CD₃OD) *δ* 161.3 (d, ³*J*_{C-P} = 20), 155.5, 155.1 (d, ¹*J*_{C-P} = 157), 137.0, 134.2 (d, ³*J*_{C-P} = 12), 128.9 (d, ⁴*J*_{C-P} = 2.5), 128.8 (d, ²*J*_{C-P} = 22), 127.8, 126.1, 123.8 (q, ¹*J*_{C-F} = 280), 116.8, 115.0, 94.9, 87.2 (d, ⁴*J*_{C-P} = 2.2), 65.7 (q, ²*J*_{C-F} = 38), 63.1 (d, ²*J*_{C-P} = 6.5), 60.6, 57.7, 56.6, 55.3, 52.4, 51.0, 48.0, 45.5, 32.7, 26.7, 23.7, 20.8, 16.8 (d, ³*J*_{C-P} = 6.2), 13.7 (d, ¹*J*_{C-P} = 103), 10.4; ¹⁹F-NMR (376 MHz, CD₃OD) *δ* -73.5 (-SO₂CH₂CF₃), -74.9 (TFA salt); ³¹P-NMR (162 MHz, CD₃OD) *δ* +40.8; ESI-LRMS (+) *m*/*z* 1279 [M+H]⁺; ESI-HRMS (+) calcd for [C₅₆H₇₆F₆N₇O₁₂P₂S₂]⁺ 1278.437, found 1278.440.

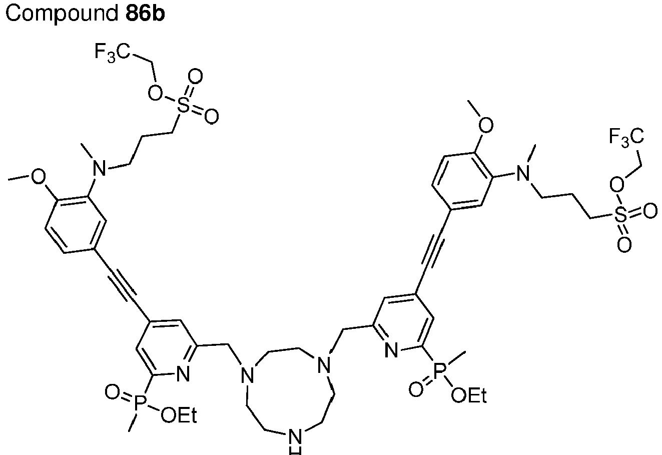

A solution of compound **85b** (29 mg, 0.021 mmol) in trifluoroacetic acid and CH₂Cl₂ was prepared (10% v/v, 3 mL total) and stirred under argon for 60 min. Immediately following this, the solvent was removed under reduced pressure. Additional CH₂Cl₂ (∼50 mL) was added and the solvent was removed under reduced pressure. This procedure was repeated 3 times. Drying under high vacuum for several hours yielded an orange oil (29 mg, quant.); ¹H-NMR (400 MHz, CDCl₃) *δ* 7.87 (d, ³*J*_{H-P} = 6.0, 2H), 7.57 - 7.43 (m, 6H), 7.00 (d, ³*J*_{H-H} = 8.5, 2H), 4.51 (q, ³*J*_{H-F} = 7.9, 4H), 4.27 - 4.09 & 4.03 - 3.87 (m, 14H), 3.67 - 3.53 (m, 6H), 3.47 - 3.26 (m, 12H), 3.13 - 3.03 (m, 8H), 2.14 - 2.05 (m, 4H), 1.77 (d, ²*J*_{H-P} = 15, 6H), 1.31 (t, ³*J*_{H-H} = 7.4, 6H); ¹⁹F-NMR (376 MHz, CDCl₃) *δ* -74.6 (-SO₂CH₂CF₃), -76.3 (TFA salt); ³¹P-NMR (162 MHz, CDCl₃) *δ* +40.3; ESI-LRMS (+) *m*/*z* 1250 [M+H]⁺; ESI-HRMS (+) calcd for [C₅₄H₇₂N₇F₆O₁₂P₂S₂]⁺ 1250.406, found 1250.408.

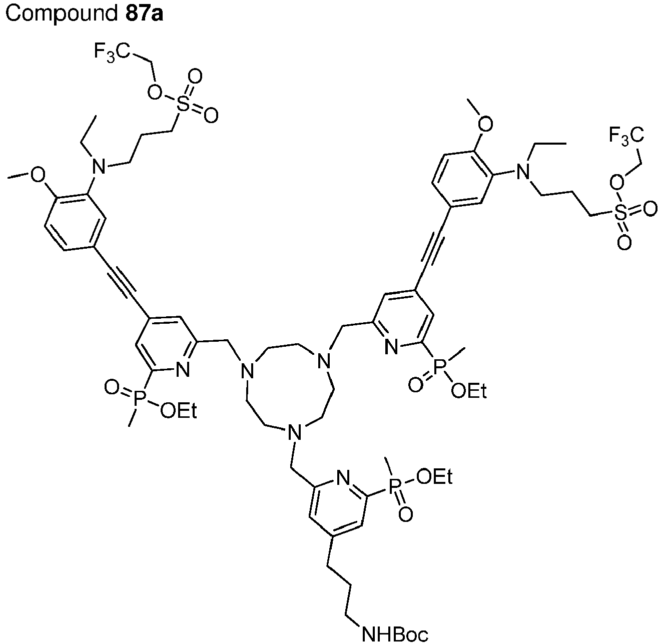

Compound **86a** (44 mg, 0.032 mmol), compound **60** (30 mg, 0.067 mmol) and K₂CO₃ (30 mg, 0.22 mmol) were combined in anhydrous CH₃CN (2 mL) under argon and heated to 60°C for 15 h. After this time, LC-MS analysis confirmed the complete transformation of the macrocycle. The reaction mixture was allowed to cool before separation of the solution from the inorganic salts by filtration. Removal of the solvent under reduced pressure afforded a yellow oil (72 mg) which was used directly in the next step without further purification; ¹H-NMR (400 MHz, CDCl₃) *δ* 7.96 (d, ³*J*_{H-P} = 6.2, 1H), 7.78 (d, ³*J*_{H-P} = 6.2, 2H), 7.42 (s, 1H), 7.24 (s, 2H), 7.19 (dd, ³*J*_{H-H} = 8.4, ⁴*J*_{H-H} = 2.0, 2H), 7.09 (d, ⁴*J*_{H-H} = 2.0, 2H), 6.82 (d, ³*J*_{H-H} = 8.4, 2H), 4.76 (s, 6H) 4.68 (br s, 1H), 4.45 (q, ³*J*_{H-F} = 8.0, 4H), 4.12 - 3.76 (m, 16H), 3.37 - 3.30 (m, 4H), 3.20 - 3.05 (m, 10H), 2.94 - 2.82 (m, 8H), 2.72 - 2.64 (m, 2H), 1.83 - 1.79 (m, 4H), 1.73 (d, ²*J*_{H-P} = 15, 9H), 1.41 (s, 9H), 1.26 - 1.20 (m, 9H), 0.99 (t, ³*J*_{H-H} = 7.1, 6H); ¹⁹F-NMR (376 MHz, CDCl₃) *δ* -74.0; ³¹P-NMR (162 MHz, CDCl₃) *δ* +40.0; ESI-LRMS (+) *m*/*z* 1633 [M+H]⁺, 817 [M+2H]²⁺; ESI-HRMS (+) calcd for [C₇₃H₁₀₄F₆N₉O₁₆P₃S₂]²⁺ 816.8080, found 816.8085.

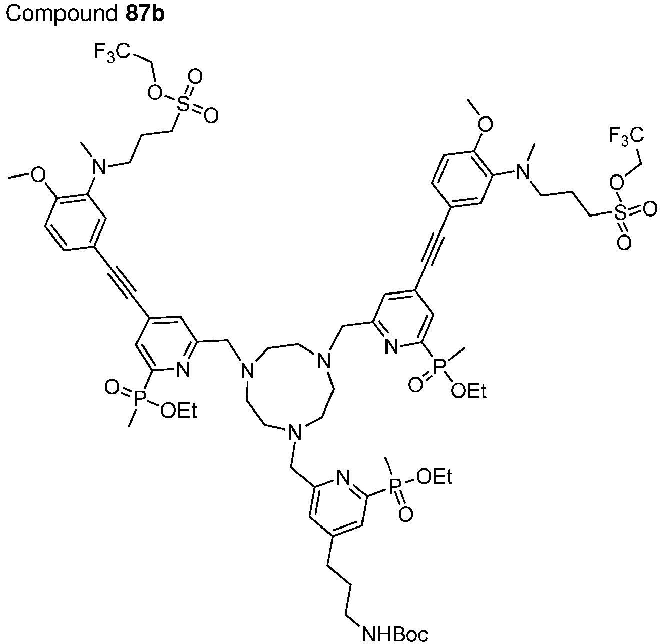

Compound **86b** (29 mg, 0.021 mmol), compound **60** (19 mg, 0.042 mmol) and K₂CO₃ (20 mg, 0.15 mmol) were combined in anhydrous CH₃CN (2 mL) under argon and heated to 60°C for 15 h. After this time, LCMS analysis confirmed the complete transformation of the macrocycle. The reaction mixture was allowed to cool before separation of the solution from the inorganic salts by filtration. Removal of the solvent under reduced pressure afforded a yellow oil (47 mg) which was used directly in the next step without further purification; ¹H-NMR (400 MHz, CDCl₃) *δ* 7.99 (d, ³*J*_{H-P} = 6.0, 1H), 7.80 (dd, ³*J*_{H-P} = 6.0, ⁴*J*_{H-H} = 1.4, 2H), 7.44 - 7.42 (m, 1H), 7.25 - 7.22 (m, 2H), 7.22 - 7.16 (m, 2H), 7.09 (d, ⁴*J*_{H-H} = 1.8, 2H), 6.83 (d, ³*J*_{H-H} = 8.4, 2H), 4.77 (s, 6H), 4.50 (q, ³*J*_{H-F} = 8.0, 4H), 4.13 - 3.76 (m, 16H), 3.43 - 3.38 (m, 2H), 3.17 - 3.09 (m, 14H), 2.75 (s, 6H), 2.71 - 2.66 (m, 2H), 1.85 - 1.80 (m, 4H), 1.74 (d, ²*J*_{H-P} = 15, 9H), 1.42 (s, 9H), 1.25 (t, ³*J*_{H-H} = 7.1, 9H); ¹⁹F-NMR (376 MHz, CDCl₃) *δ* -73.9; ³¹P-NMR (162 MHz, CDCl₃) δ +40.0; ESI-LRMS (+) *m*/*z* 1605 [M+H]⁺; ESI-HRMS (+) calcd for [C₇₁H₉₉F₆N₉O₁₆P₃S₂]⁺ 1604.577, found 1604.577.

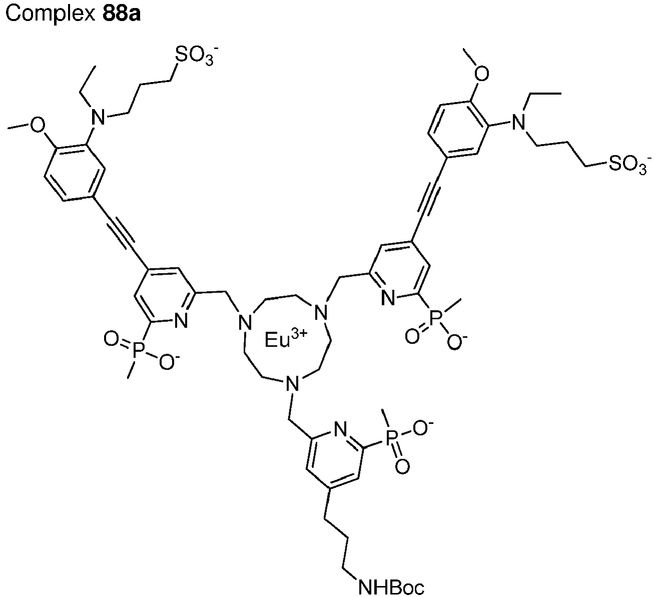

The crude ligand **87a** (57 mg) was dissolved in a mixture of CH₃OH/aqueous NaOH solution (0.1 M, 3:1, 2 mL total). The solution was heated to 70°C for 24 h. After this time, LCMS analysis confirmed complete hydrolysis of the phosphinate and sulfonate ester groups. After cooling and adjusting the pH to 6 using dilute hydrochloric acid (0.1 M), EuCl₃.6H₂O (14 mg, 0.038 mmol) was added and the reaction mixture was heated to 75°C for 24 h. The reaction mixture was purified by reverse phase HPLC (10 to 100% CH₃CN in H₂O over 10 min, *t*ᵣ = 7.9 min) to yield a pale yellow solid (13 mg, 27% over two steps); *T*_{H2O} (ms) = 0.31 (pH 9), 0.31 (pH 8), 0.29 (pH 7), 0.41 (pH 6), 0.76 (pH 5), 1.07 (pH 4); *ε*_{332 nm} = 35000 M⁻¹ cm⁻¹; *Φ*_{pH 8} = 0.06%, *Φ*_{pH 4} = 13.4% (*λ*_{exc} = 332 nm).

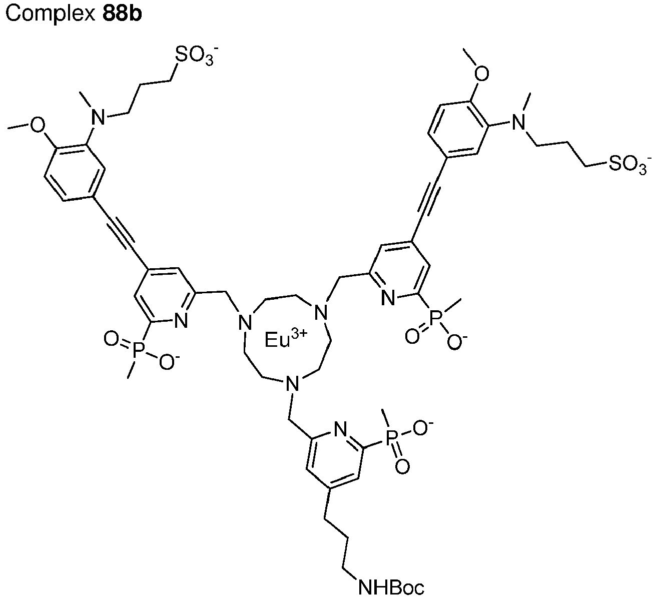

The crude ligand **87b** (23 mg) was dissolved in a mixture of CH₃OH/aqueous NaOH solution (0.1 M, 3:1, 2 mL total). The solution was heated to 70°C for 48 h. After this time, LCMS analysis confirmed complete hydrolysis of the phosphinate and sulfonate ester groups. After cooling and adjusting the pH to 5.5 using hydrochloric acid (0.1 M), EuCl₃.6H₂O (8 mg, 0.022 mmol) was added and the reaction mixture was heated to 75°C for 24 h. The reaction mixture was purified by reverse phase HPLC (10 to 100% CH₃CN in H₂O over 10 min, *t*ᵣ = 7.9 min) to yield a dark yellow solid (1.8 mg, 11% over two steps); *T*_{H2O} (ms) = 0.30 (pH 6.5), 0.31 (pH 6), 0.40 (pH 5), 0.83 (pH 4), 1.01 (pH 3). *ε*_{336 nm} = 35000 M⁻¹ cm⁻¹; *Φ*_{pH 6} = 0.06%, *Φ*_{pH 3} = 13% (*λ*_{exc} = 336 nm).

Compound **89** was synthesized as described for compound **92.**

Compound **90** was synthesized as described for compound **93.**

Compound **91** was synthesized as described for compound **78a.**

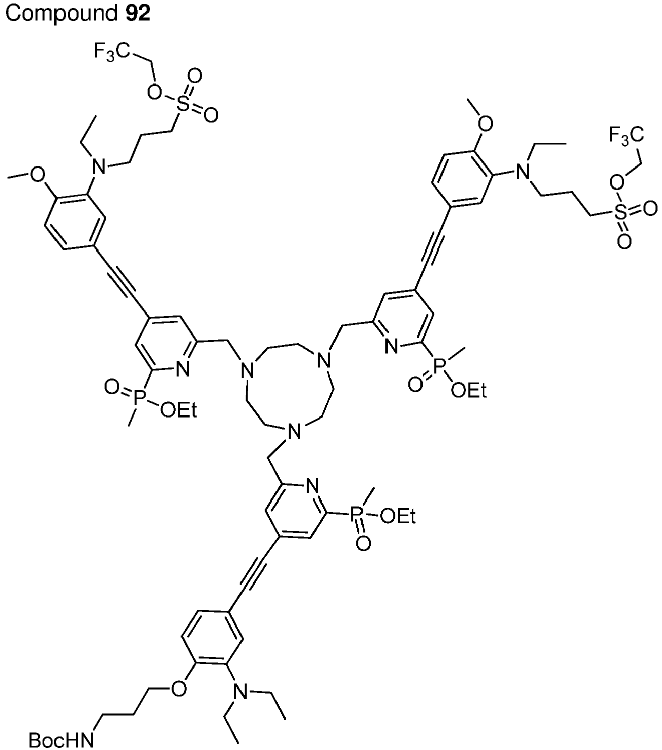

Compound **85a** (44 mg, 0.032 mmol), compound **38** (31 mg, 0.049 mmol) and K₂CO₃ (30 mg, 0.22 mmol) were combined in anhydrous CH₃CN (2 mL) under argon and heated to 60°C for 14 h. After this time, LCMS analysis confirmed the complete transformation of the macrocycle **85a.** The reaction mixture was allowed to cool before separation of the solution from the inorganic salts by filtration. Removal of the solvent under reduced pressure afforded a yellow oil (47 mg) which was used directly in the next step without further purification; ¹H-NMR (400 MHz, CD₃OD) *δ* 7.91 - 7.82 (m, 5H), 7.30 - 7.16 (m, 7H), 6.99 - 6.93 (m, 3H), 4.84 (s, 6H), 4.64 (q, ³*J*_{H-F} = 8.1, 4H), 4.14 - 3.84 (m, 22H), 3.43 - 3.37 (m, 2H), 3.30 - 3.24 (m, 2H), 3.19 - 3.07 (m, 8H), 2.91 - 2.83 (m, 8H), 2.02 - 1.89 (m, 6H), 1.77 (d, ²*J*_{H-P} = 15, 9H), 1.44 (s, 9H), 1.26 - 1.21 (m, 9H), 1.03 - 0.98 (m, 12H); ¹⁹F-NMR (376 MHz, CD₃OD) *δ* -76.8; ³¹P-NMR (162 MHz, CD₃OD) *δ* + 41.8; ESI-LRMS (+) *m*/*z* 1821 [M+H]⁺, 911 [M+2H]²⁺; ESI-HRMS (+) calcd for [C₈₅H₁₁₇F₆N₁₀O₁₇P₃S₂]²⁺ 910.8594, found 910.8554.

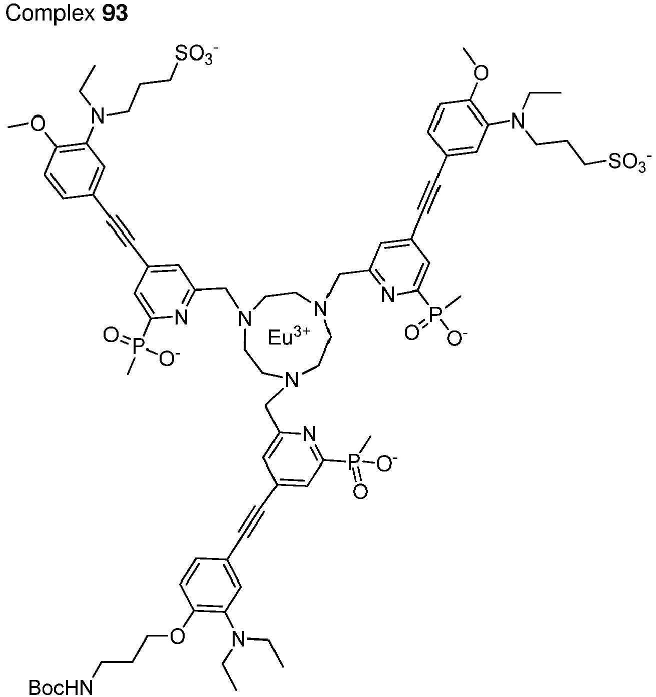

The crude ligand **92** (38 mg) was dissolved in a mixture of CH₃OH/aqueous NaOH solution (0.1M, 3:1, 2 mL total). The solution was heated to 70°C for 24 h. After this time, LCMS analysis confirmed complete hydrolysis of the phosphinate and sulfonate ester groups. After cooling and adjusting the pH to 6.5 using hydrochloric acid (0.1 M), EuCl₃.6H₂O (14 mg, 0.038 mmol) was added and the reaction mixture was heated to 75°C for 24 h. The reaction mixture was purified by reverse phase HPLC (10 to 100% CH₃CN in H₂O over 10 min, *t*ᵣ = 8.8 min) to yield a pale yellow solid (11 mg, 25% over two steps); *^{T}*_{H2O} (ms) = 0.20 (pH 9), 0.21 (pH 8), 0.22 (pH 7), 0.35 (pH 6), 0.69 (pH 5), 0.98 (pH 4), 0.98 (pH 4), 1.01 (pH 3.5). *ε*₃₃₂ nm = 75000 M⁻¹ cm⁻¹; *Φ*_{pH 8} = 0.01%, *Φ*_{pH 4} = 15.9% (*λ*_{exc} = 332 nm).

Compound **94** was obtained according to the procedure described in US9,981,967.

Compound **95** was prepared as described for compound **20.**

Complex **96** was prepared as described for complex **21.**

Complex **97** was prepared as described for complex **78a.**

Complex **98** was prepared as described for complex **99a.**

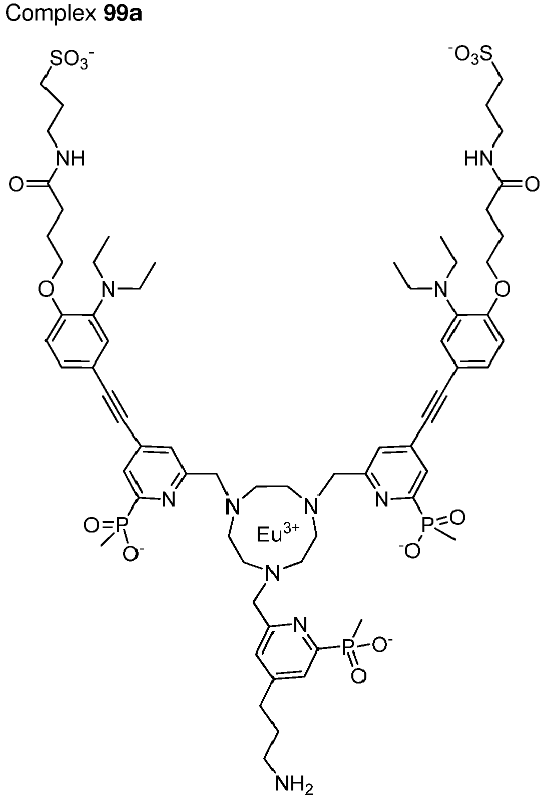

To complex **78a** (6.93 mg, 4 µmol) was added trifluoroacetic acid (200 µL). The mixture was stirred at RT for 1 h and then purified by preparative HPLC (Column Waters Xbridge C₁₈, 5 µm, 20 x 100 mm - A / H₂O 25 mM TEAAc pH 7 B / CH₃CN t = 0 min 2% B - t = 18 min 40% B - 20 mL.min⁻¹) to afford a yellow solid (3.5 µmol, 87%). ESI-LRMS (-) *m*/*z* 814.70 [M-2H]²⁻.

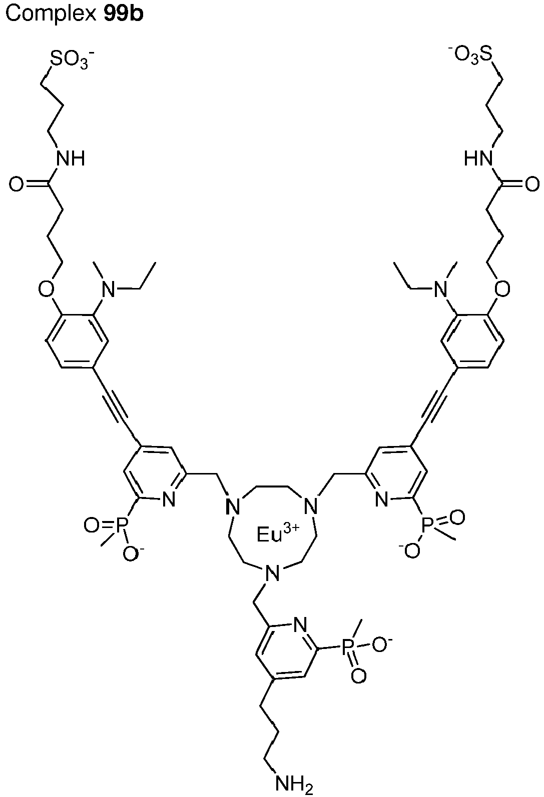

To complex **78b** (9 mg, 5283 nmol) was added trifluoroacetic acid (1 mL). The mixture was stirred at RT for 1 h and then purified by preparative HPLC (Column Waters Xbridge C₁₈, 5 µm, 20 x 100 mm - A / H₂O 25 mM TEAAc pH 7 B / CH₃CN t = 0 min 2% B - t = 18 min 40% B - 20 mL.min⁻¹) to give a yellow solid (4256 nmol, 81%). ESI-LRMS (-) m/z 800.83 [M-2H]²⁻.

### BG-MB-NHS (structure scheme 26)

The compound was obtained as described in Inorganic Chemistry-2014-53-1854.

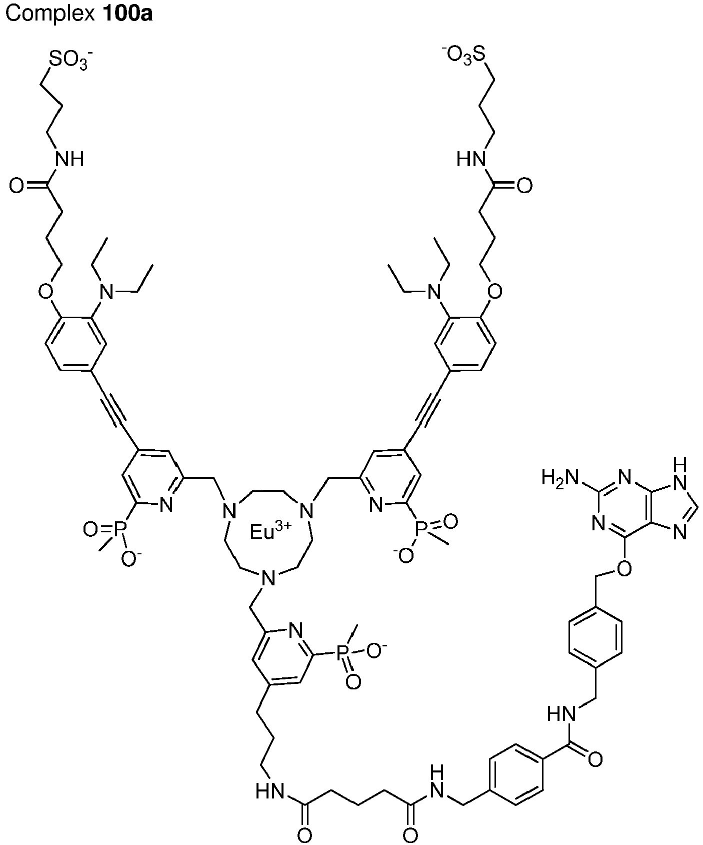

A solution of **BG-MB-NHS** (0.615 mg, 1 µmol) in dry DMSO (100 µL) was added to complex **99a** (1.403 mg, 860 nmol). To the resulting solution was added DIPEA (0.5 µl, 2862 nmol) and the mixture was stirred at RT for 1 h and purified by preparative HPLC (Column Waters Xbridge C₁₈, 5 µm, 20 x 100 mm - A / H₂O 25 mM TEAAc pH 7 B / CH₃CN t = 0 min 2% B - t = 18 min 40% B - 20 mL.min⁻¹) to give a white powder (370 nmol, 43%). ESI-LRMS (-) *m*/*z* 1064.89 [M-2H]²⁻.

### Complex 100b

A solution of **BG-MB-NHS** (0.738 mg, 1.20 µmol) in dry DMSO (430 µL) was added to complex **99b** (1.924 mg, 1.2 µmol). To the resulting solution was added DIPEA (0.5 µl, 2862 nmol) and the mixture was stirred at RT for 1 h and purified by preparative HPLC (Column Waters Xbridge C₁₈, 5 µm, 20 x 100 mm - A / H₂O 25 mM TEAAc pH 7 B / CH₃CN t = 0 min 2% B - t = 18 min 40% B - 20 mL.min⁻¹) to give a white powder (0,728 µmol, 60% yield). ESI-LRMS (-) *m*/*z* 1051.09 [M+H]²⁻.

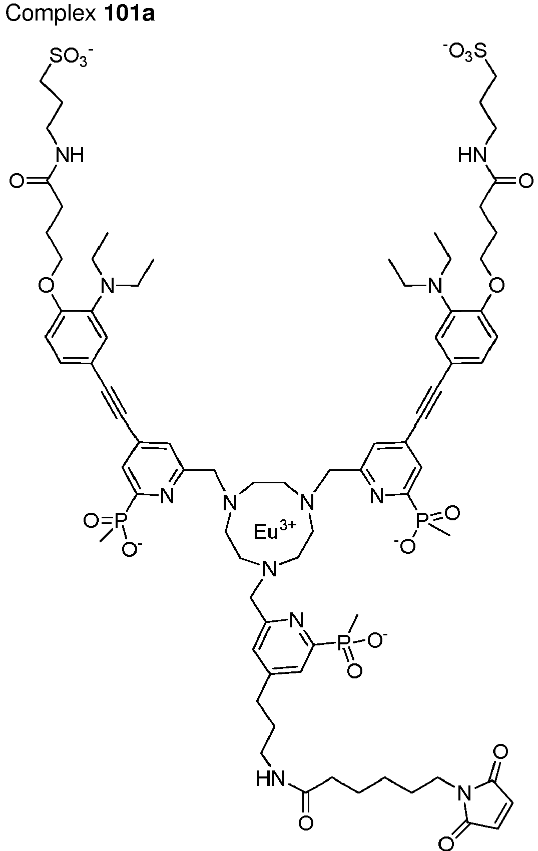

To a solution of complex **99a** (1.403 mg, 860 nmol) in dry DMSO (100 µL) was added DIPEA (0.150 µl, 860 nmol) and 6-maleimidohexanoic acid N-hydroxysuccinimide ester (0.271 mg, 860 nmol) in anhydrous DMSO (5 µL). The mixture was stirred at RT for 1 h and purified by preparative HPLC (Column Waters Xbridge C₁₈, 5 µm, 20 x 100 mm - A / H₂O 25 mM TEAAc pH 5 B / CH₃CN t = 0 min 2% B - t = 18 min 40% B - 20 mL.min⁻¹) to give a yellow powder (761 nmol, 89% yield). ESI-LRMS (-) *m*/*z* 911.75 [M+H]²⁻.

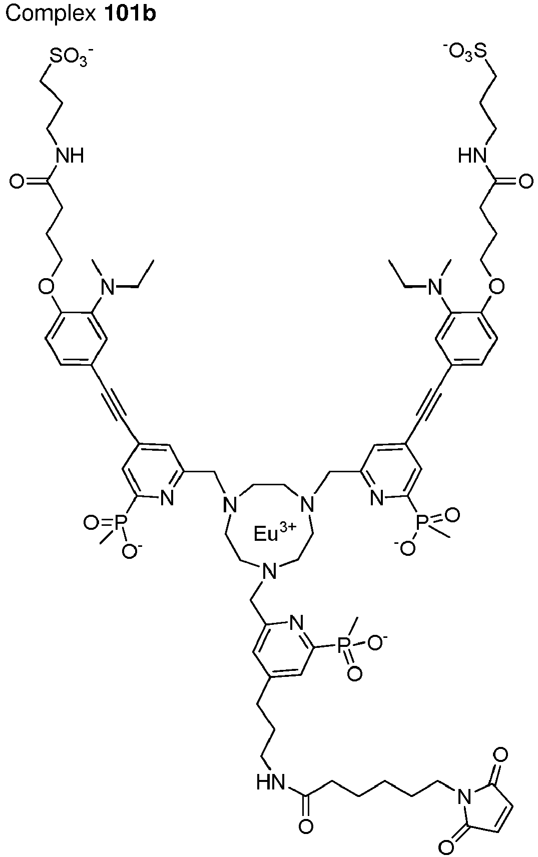

To a solution of complex **99b** (1.283 mg, 800 nmol) in dry DMSO (100 µL) was added DIPEA (0.140 µl, 800 nmol) and 6-maleimidohexanoic acid N-hydroxysuccinimide ester (0.252 mg, 800 nmol) in anhydrous DMSO (5 µL). The mixture was stirred at RT for 1 h and purified by preparative HPLC (Column Waters Xbridge C₁₈, 5 µm, 20 x 100 mm - A / H₂O 25 mM TEAAc pH 5 B / CH₃CN t = 0 min 2% B - t = 18 min 40% B - 20 mL.min⁻¹) to give a yellow powder (726 nmol, 91% yield). ESI-LRMS (-) *m*/*z* 898.2 [M+H]²⁻. Compound **102** was synthesized following the procedure described in Helvetica Chimica Acta, 1993, 76, 877.

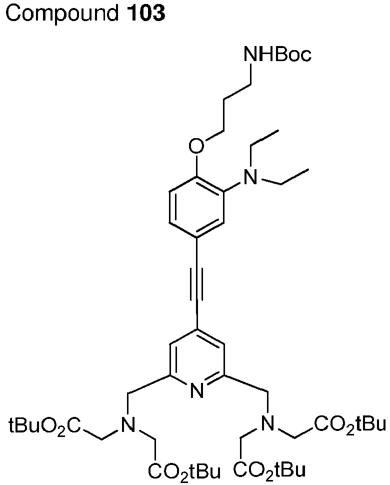

Compound **36** (74 mg, 0.214 mmol) and compound **102** (140 mg, 0.195 mmol) were combined in anhydrous CH₃CN (2.5 mL) under argon. To this solution was added Pd₂Cl₂(allyl)₂ (10 mg, 0.027 mmol), P(^{t}Bu)₃ (0.01 mL, 0.041 mmol) and piperidine (0.06 mL, 0.66 mmol) in that order. The resulting mixture was stirred at 40°C under argon for 17 h before removing the solvent under reduced pressure. The crude product was purified by reverse phase HPLC (10 to 100% CH₃CN in H₂O, *t*ᵣ = 19.8 min) to yield a pale orange oil (73 mg, 40%); ¹H-NMR (700 MHz, CDCl₃) *δ* 7.59 (s, 2H), 7.18 - 7.11 (m, 2H), 6.81 (d, ³*J*_{H-H} = 8.2, 1H), 4.09 (t, ³*J*_{H-H} = 5.4, 2H), 4.01 (s, 4H), 3.49 (s, 8H), 3.40 - 3.34 (m, 2H), 3.15 (q, ³*J*_{H-H} = 6.7, 4H), 2.03 - 1.98 (m, 2H), 1.46 (s, 36H), 1.45 (s, 9H), 1.02 (t, ³*J*_{H-H} = 6.7, 6H); ¹³C-NMR (176 MHz, CDCl₃) *δ* 170.7, 159.1, 156.4, 154.6, 139.6, 133.1, 127.3, 125.8, 122.8, 114.9, 112.8, 94.0, 86.4, 81.2, 79.0, 68.5, 59.9, 56.0, 46.4, 39.3, 29.7, 28.7, 28.3, 12.0; ESI-LRMS (+) *m*/*z* 939 [M+H]⁺; ESI-HRMS (+) calcd for [C₅₁H₈₀N₅O₁₁]⁺ 938.5854, found 938.5851.

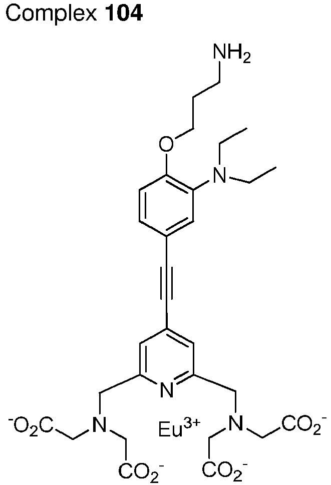

A solution of complex **102** (36 mg, 0.038 mmol) in trifluoroacetic acid and CH₂Cl₂ (20% v/v, 4 mL total) was prepared. The solution was stirred at RT for 6 h before removing the solvent under reduced pressure to give an orange residue. This residue was purified by reverse phase HPLC (10 to 100% CH₃CN in H₂O over 10 min, *t*ᵣ = 1.2 min) to afford the free amine which was dissolved in H₂O (3 mL), the pH was adjusted to 6.5 before addition of EuCl₃.6H₂O (14 mg, 0.038 mmol). The mixture was heated to 60°C for 24 h before separating the solution from the inorganic salts by filtration. The solution was purified by reverse phase HPLC (10 to 100% CH₃CN in H₂O over 10 min, *t*ᵣ = 1.7 min) to yield a yellow solid (27 mg, 93% over two steps); ESI-LRMS (+) *m*/*z* 763 [M+H]⁺; ESI-HRMS (+) calcd for [C₃₀H₃₆¹⁵¹EuN₅O₉]⁺ 763.1729, found 763.1731; *T*_{H2O} (ms) = 0.30 (pH 8), 0.32 (pH 7), 0.35 (pH 6), 0.37 (pH 5), 0.38 (pH 4); *ε*_{330 nm} = 12000 M⁻¹ cm⁻¹; *Φ*_{pH 8} = 0.17%, *Φ*_{pH 4} = 3.2% (*λ*_{exc} = 330 nm).

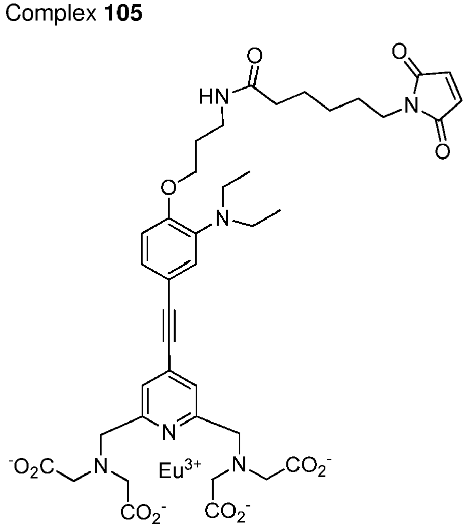

To a solution of complex **104** (0.655 mg, 860 nmol) in dry DMSO (100 µL) was added DIPEA (0.150 µL, 860 nmol) and 6-maleimidohexanoic acid N-hydroxysuccinimide ester (0.271 mg, 860 nmol) in anhydrous DMSO (5 µL). The mixture was stirred at RT for 1 h and purified by preparative HPLC (Column Waters Xbridge C₁₈, 5 µm, 20 x 100 mm - A / H₂O 25 mM TEAAc pH 6 B / CH₃CN t = 0 min 5% B - t = 19 min 40% B - 20 mL.min⁻¹) to give a yellow powder. ESI-LRMS (-) *m*/*z* 956.85 [M+H]⁻.

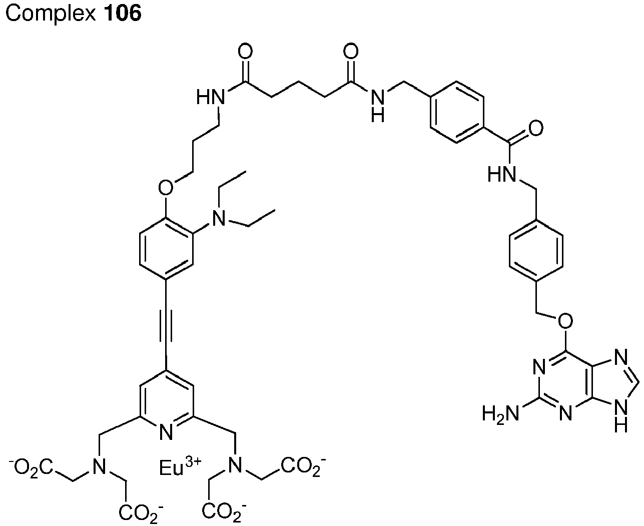

A solution of **BG-MB-NHS** (783 µg, 1274 nmol) in dry DMSO (430 µL) was added to complex **104** (970 µg, 1274 nmol). To the resulting solution was added DIPEA (0.5 µL, 2862 nmol) and the mixture was stirred at RT for 1 h and purified by preparative HPLC (Column Waters Xbridge C₁₈, 5 µm, 20 x 100 mm - A / H₂O 25 mM TEAAc pH 6 B / CH₃CN t = 0 min 5% B - t = 19 min 40% B - 20 mL.min⁻¹) to give a white powder (0.728 µmol, 60% yield). ESI-LRMS (-) *m*/*z* 1262.06 [M+H]⁻.

The properties of representative complexes of the invention (complexes 14, 18, 21, 78a, 78b, 88a, 88b, 93, 104) were investigated. The presence of the Boc- group was not predicted to alter these properties. The experiments were thus carried out with the Boc group for the functionalized complexes. The absorption and emission spectra were recorded in a pH gradient (pH 4 to pH 10).

### 1) Photophysical properties

Photophysical properties are summarised in Tables 1 & 2.

**Table 1: Photophysical properties of some europium complexes**

| **Complex** | **14** | **18** | **21** | **78a** | **78b** | **88a** | **88b** | **93** | **104** |
|---|---|---|---|---|---|---|---|---|---|
| *λ*_{exc} (nm) | 331 | 328 | 331 | 330 | 330 | 332 | 336 | 332 | 330 |
| ε (M⁻¹.cm⁻¹) | 11450 | 35000 | 45000 | 39000 | 39000 | 35000 | 35000 | 75000 | 12000 |
| Q.Y. pH 8 | 0.5 | 0.2 | 0.1 | 0.3 | 0.01 | 0.06 | 0.06 | 0.01 | 0.17 |
| Q.Y. pH 4 | 16.8 | 17.6 | 17 | 16 | 14.5 | 13.4 | 13 | 15.9 | 3.2 |

Traditionally, molar extinction coefficients, *ε*, are calculated using the Beer-Lambert equation and involve measuring the absorbance of a series of solutions of a complex at different known concentrations. A plot of absorbance at a particular wavelength against concentration allows determination of *ε* from the gradient of the linear correlation. The quantum yield (QY) of the complexes increases as the pH decreases. For complexes **78b, 88a, 88b** and **93,** the quantum yield at pH 8 is approaching 0 meaning that the complexes are therefore hardly emissive at physiological pH and conversely, these complexes are very bright at pH 4.

Rather unexpectedly, an isosbestic point was observed at 332 nm. This could be useful as excitation at, or near, this wavelength would allow equal excitation of both the unprotonated and protonated complex, which exist in a pH-dependent equilibrium in solution. An isosbestic point was not observed for Complex **14.**

**Table 2: Excited state lifetimes of complexes at different pH (ms)**

| **Complex** | **14** | **18** | **21** | **78a** | **78b** | **88a** | **88b** | **93** | **104** |
|---|---|---|---|---|---|---|---|---|---|
| pH 9 | 0.5 | 0.34 | 0.24 | - | - | 0.31 | - | 0.2 | |
| pH 8 | 0.53 | 0.34 | 0.25 | 0.25 | 0.3 | 0.31 | - | 0.21 | 0.3 |
| pH 7 | 0.74 | 0.47 | 0.32 | 0.34 | 0.29 | 0.29 | 0.3 | 0.22 | 0.32 |
| pH 6 | 1.06 | 0.78 | 0.59 | 0.7 | 0.42 | 0.41 | 0.31 | 0.35 | 0.35 |
| pH 5 | 1.15 | 0.96 | 0.83 | 0.98 | 0.77 | 0.76 | 0.4 | 0.69 | 0.37 |
| pH 4 | 1.16 | 1 | 0.84 | 1.04 | 1.05 | 1.07 | 0.83 | 0.98 | 0.38 |
| pH 3 | - | - | - | - | - | - | 1.01 | 1.01 | - |

### 2) Emission of the complexes

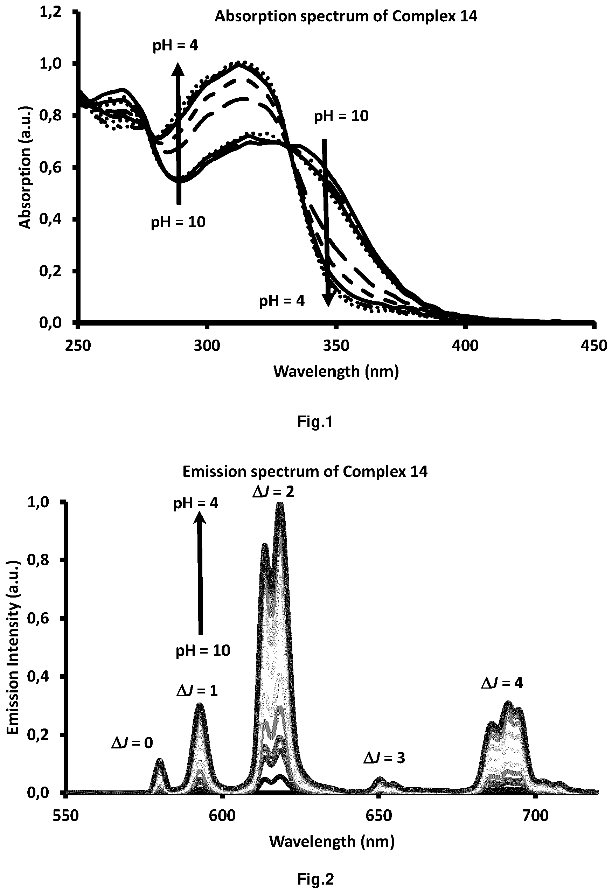

For all the emission spectra, an impressive 'switch-on' of emission intensity is observed in response to the lowering of pH over the physiological range (Nat. Rev. Mol. Cell. Biol. 2010, 11, 50). The Δ*J* = 2 band ((Δ*J* assignment showed in figure 2) is clearly the most intense and therefore the most suitable reference band in the emission spectrum, especially in the event of excitation at higher wavelengths. The change in emission intensity with pH occurs for all the complexes of the invention. The exchange of one extended chromophore for a single pyridine and installation of lengthy head groups at the periphery of the chromophore had no impact on the form of the europium emission spectra. Indeed the significant modifications which have been made to the complexes do not impact the immediate coordination environment around the europium(III) center which remains effectively unperturbed.

As the most emissive band, the maximum wavelength of the Δ*J* = 2 band (Δ*J* assignment showed in figure 2) was chosen as the wavelength of emission to be monitored.

### 3) pKₐ Values of the complexes

Table 3 shows the p*K*ₐ value determined for the complexes of the present invention.

**Table 3: pKₐ values of complexes (295 K, I = 0.1 M NaCl)**

| **Complex** | **14** | **18** | **21** | **78a** | **78b** | **88a** | **88b** | **93** | **104** |
|---|---|---|---|---|---|---|---|---|---|
| p*K*ₐ | 6.75 | 6.3 | 6.21 | 6.20 | 5.34 | 5.2 | 4.32 | 5.19 | 6.35 |

The experimentally measured excited-state lifetime dependence on the pH has a very similar profile irrespective of the number of chromophores incorporated in the europium complexes.

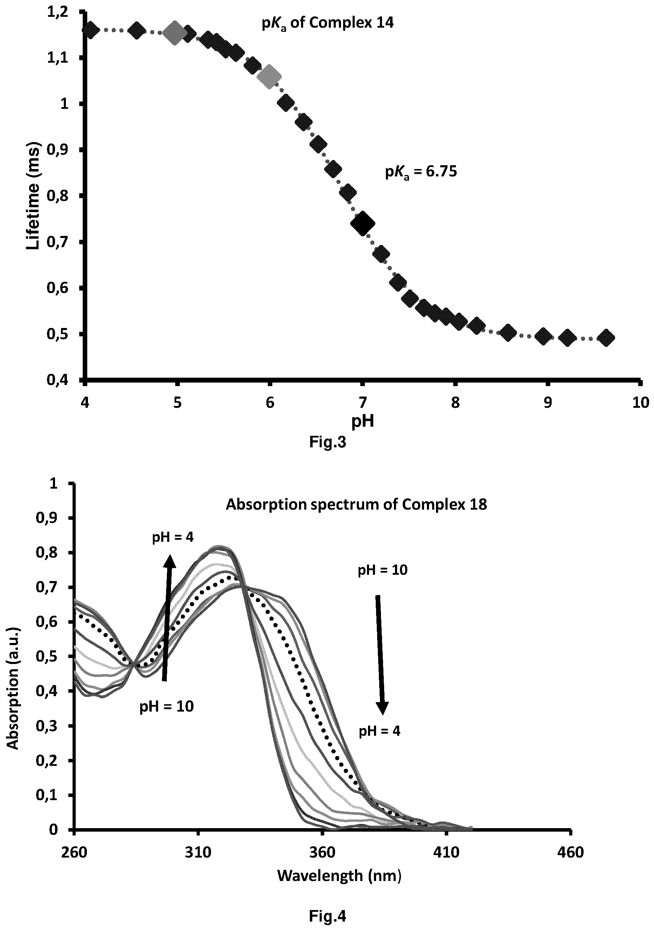

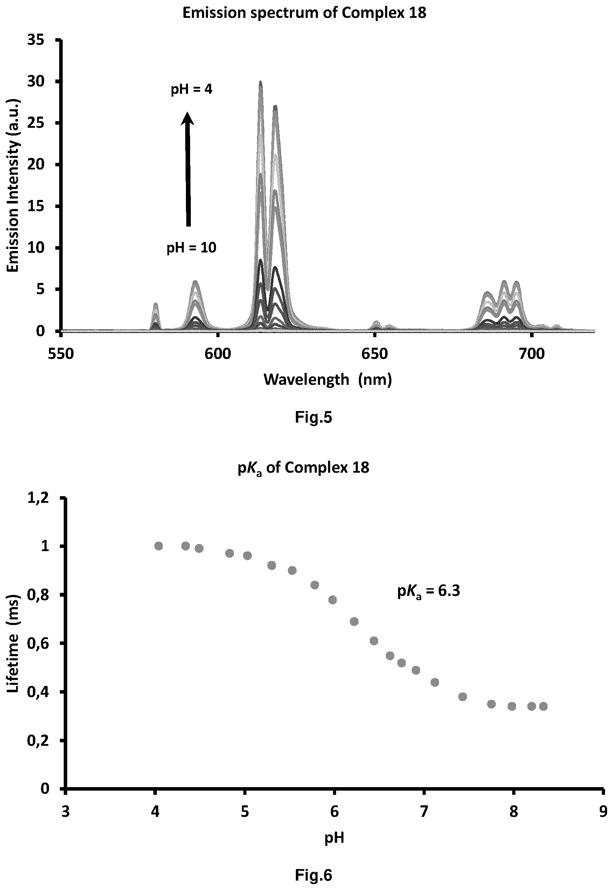

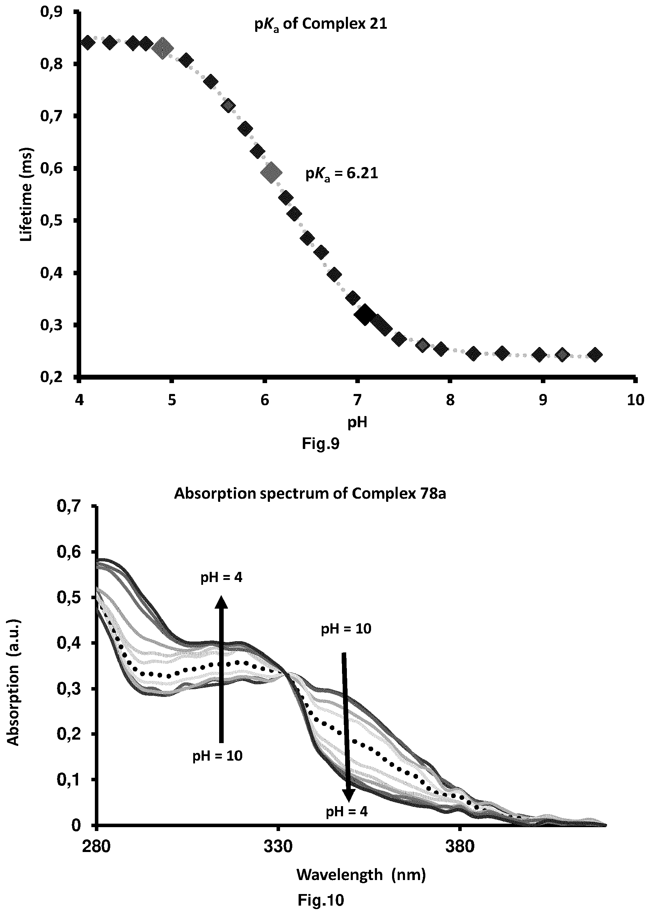

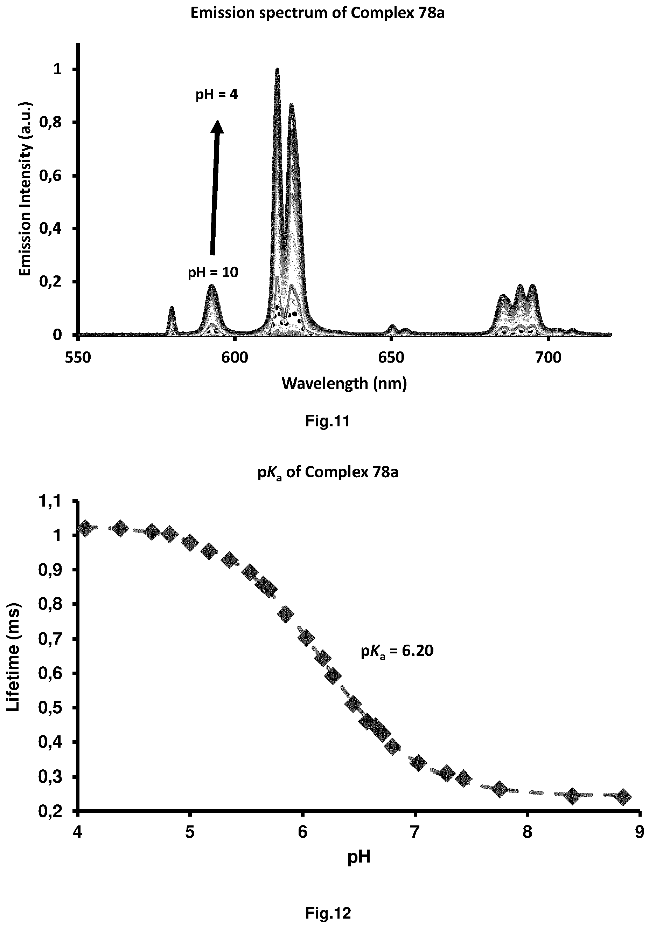

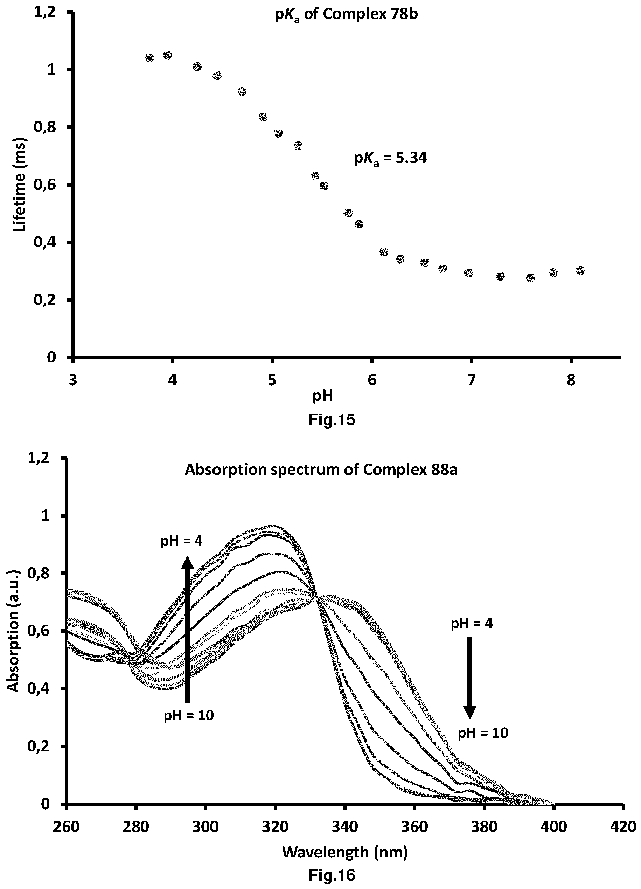

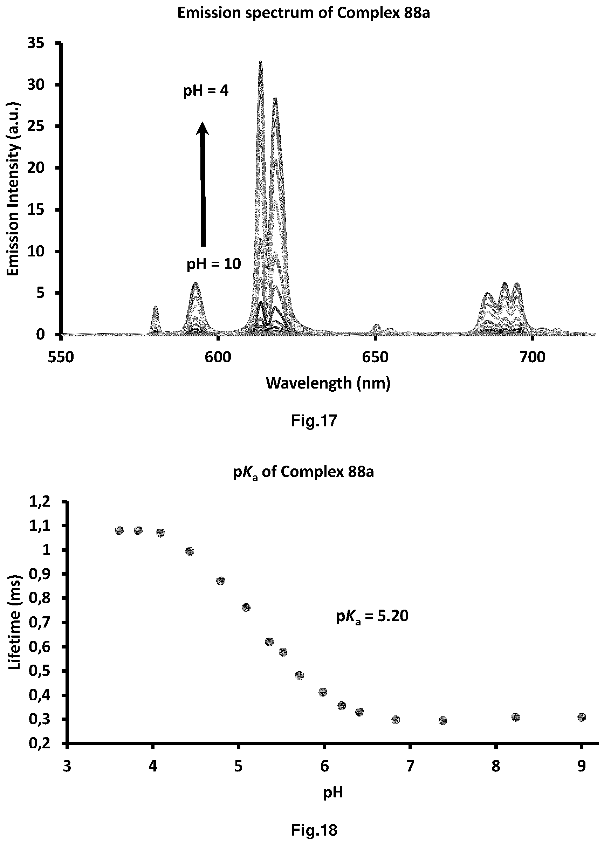

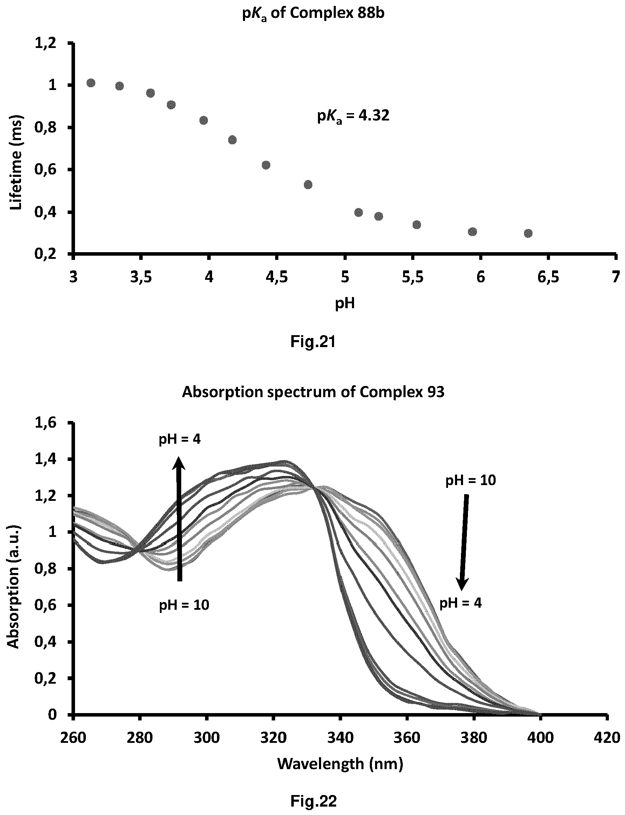

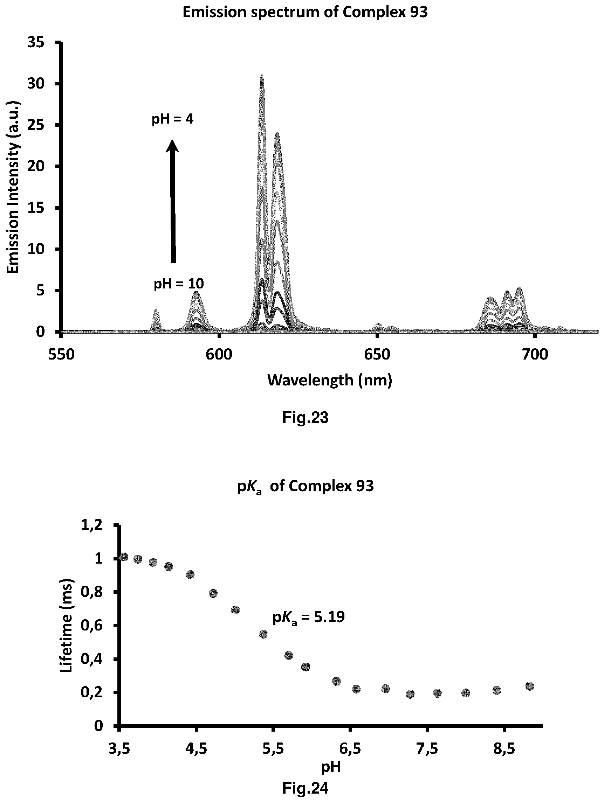

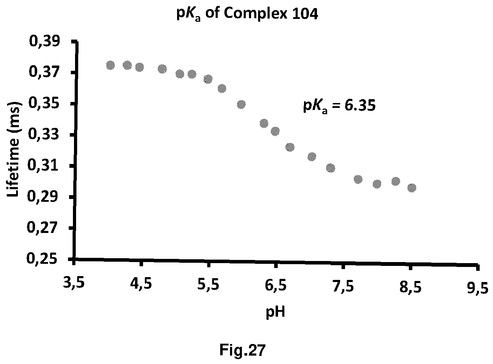

The variance in the emission lifetime with pH is shown in Figures **3****,** **6****,** **9****,** **12****,** **15****,** **18****,** **21****,** **24** and **27****.** An emission lifetime value at each pH was obtained by plotting the decay of the emission lifetime over time and fitting the curve to a simple exponential decay model. The p*K*ₐ values of the complexes were determined for the reversible protonation. Protonation of one chromophore has no impact on the p*K*ₐ of the other chromophore in the complex (complexes **18** and **21**).

The chemical modifications on the chromophore involve some changes of the p*K*ₐ values (4.32 to 6.75). One can fine-tune this p*K*ₐ value to target specifically a desired value. This result is highly desirable, since one can envisage monitoring pH organelle variations inside the cell, e.g. in lysosomes, endosomes, trans Golgi network.

### 4) Time-gated experiments

A benefit of the long-lived lifetime of emission for the lanthanides is the possibility of performing time-gated measurements. Further to this, as a result of the 'switch-on' nature of complex **18**, the unprotonated and protonated forms differ in emission lifetime. These properties allow the unique opportunity to control, to some degree, the apparent p*K*ₐ value as well as the relative degree of 'switching-on' of the probe by changing the time window. This aspect is illustrated in practice using appropriate buffers of different pH (in the presence of 0.1 M NaCl) in Figure 28.

By measuring the emission intensity of the hypersensitive Δ*J* = 2 band at different pH with a longer delay time after excitation (Figure 28 top), a shallower curve was obtained (a lower apparent p*K*ₐ).

This concept was demonstrated with three different time windows (Figure 28 bottom): 60 - 460, 1000 - 2000 and 1500 - 2500 µs.

In addition to minor tuning of the apparent p*K*ₐ, these time-gating results also demonstrate the possibility of changing the magnitude of the on/off ratio of the probe by changing the time-window.

A summary of the ratios of emission intensity (at *λ*ₑₘ = 613.5 nm) at pH 4 (sensor on) and pH 8 (sensor off), where the emission signal was measured between different time windows, for complex **18** is presented in Table 4.

**Table 4**

| | Time Window | | |
|---|---|---|---|
| | 60 - 460 µs | 1000 - 2000 µs | 1500 - 2500 µs |
| pH 4 / pH 8 | 32 | 227 | 465 |

The extent of the switch-on response of the complex may be quantified, for comparison purposes, as the ratio of the emission intensity at the maximum of the Δ*J* = 2 band at pH 4, when the sensor is fully switched 'on', and pH 8, when the sensor is effectively 'off'. These values are summarized in Table 4.

The relative switch-on response increases dramatically, as a longer time window is used. It was noted that the aqueous solubility of complexes **78a-b, 88a-b** and **93** is significantly higher than that of complexes **14, 18** and **21**, as a result of the presence of the hydrophilic sulfonate groups at the periphery of the complex.

## Claims

1. A compound of formula (I): 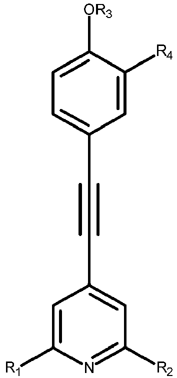 wherein:
R₁ is -CO₂H, -PO(OH)R₅ or -CH₂N(CH₂CO₂H)₂;
R₂ is -CH₂OH, -CH₂OSO₂CH₃, Cl, Br or -CH₂N(CH₂CO₂H)₂;
R₃ is a (C₁-C₆)alkyl optionally substituted with a group -L₁-E or with a group G;
R₄ is -(CH₂)ₘ-NR₆R₇;
R₅ is a (C₁-C₄)alkyl, preferably a methyl; a phenyl optionally substituted with a group -SO₃⁻, the latter preferably being in the meta or para position; or a benzyl;
R₆ is H or a (C₁-C₄)alkyl;
R₇ is a (C₁-C₆)alkyl optionally substituted with a group -L₁-E;
or R₆ and R₇, together with the nitrogen atom to which they are bonded, form a piperidine, a morpholine, or a piperazine optionally N-protected by a *tert*-butoxycarbonyl group or optionally N-substituted with R₈;
R₈ is a (C₁-C₆)alkyl optionally substituted with a group -L₁-E;
L₁ is a direct bond, -CONH-(CH₂)ₙ- or -NHCO-(CH₂)ₙ-;
E is -SO₃H, -SO₂(OCH₂CF₃), -N⁺Alk₁Alk₂Alk₃, a carbohydrate residue, or a sulfo-betaine;
G is a carboxyl group optionally protected in the form of an ester, an amino group optionally protected by a *tert*-butoxycarbonyl group, a succinimidyl ester, a haloacetamido group, a hydrazine group, an isothiocyanato group, or a maleimido group;
Alk₁, Alk₂, and Alk₃ each independently represent a (C₁-C₆)alkyl, preferably a (C₁-C₄)alkyl;
m is 0, 1, 2 or 3;
n is 0, 1, 2 or 3.

2. The compound of claim 1, wherein R₁ is -CO₂H or -PO(OH)R₅ where R₅ is a (C₁-C₄)alkyl, preferably a methyl, and R₂ is -CH₂OH or -CH₂OSO₂CH₃.

3. The compound of claim 1, wherein, R₁ and R₂ are each -CH₂N(CH₂COOH)₂.

4. A compound of formula (II) or (III): 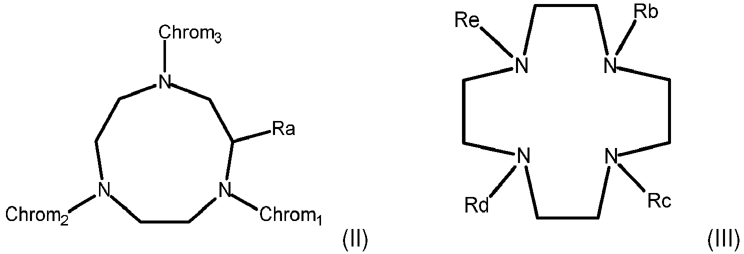 wherein:
Rₐ is absent or is -CH₂NH₂;
one of R_{b}, R_{c}, R_{d} and Rₑ is a group of formula (IV) and the others are each independently selected from -CH₂COOR_{f} and -CH₂PO(OH)R_{g};
R_{f} is H, (C₁-C₄)alkyl, -NHCH(Rₕ)-(C₁-C₄)alkyl or -NHCH(Rₕ)-C(O)ORⱼ;
R_{g} is (C₁-C₄)alkyl;
Rₕ is (C₁-C₄)alkyl or phenyl;
Rⱼ is H or (C₁-C₄)alkyl;
Chrom₁, Chrom₂ and Chrom₃ are each independently selected from a group of formula (IV) and a group of formula (V): 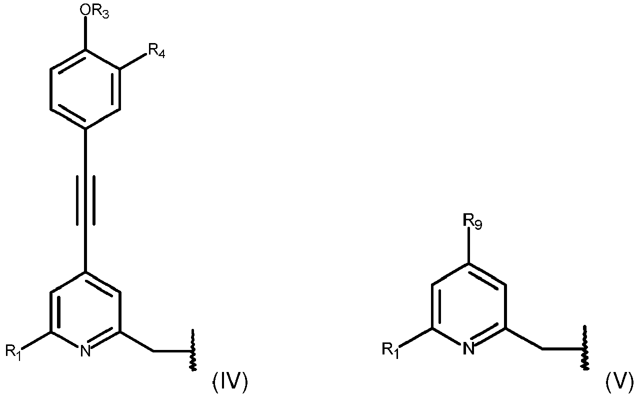 wherein:
each R₁ is -CO₂H or -PO(OH)R₅;
R₃ is a (C₁-C₆)alkyl optionally substituted with a group -L₁-E or with a group G;
R₄ is -(CH₂)ₘ-NR₆R₇;
R₅ is a (C₁-C₄)alkyl, preferably a methyl; a phenyl optionally substituted with a group -SO₃⁻, the latter preferably being in the meta or para position; or a benzyl;
R₆ is H or a (C₁-C₄)alkyl;
R₇ is a (C₁-C₆)alkyl optionally substituted with a group -L₁-E;
or R₆ and R₇, together with the nitrogen atom to which they are bonded, form a piperidine, a morpholine, or a piperazine optionally N-protected by a *tert*-butoxycarbonyl group or optionally N-substituted with a group R₈;
R₈ is a (C₁-C₆)alkyl optionally substituted with a group -L₁-E;
R₉ is a (C₁-C₆)alkyl optionally substituted with a group -L₁-E or with a group G;
L₁ is a direct bond; -CONH-(CH₂)ₙ- or -NHCO-(CH₂)ₙ;
E is -SO₃H, -SO₂(OCH₂CF₃), -N⁺Alk₁Alk₂Alk₃, a carbohydrate residue, or a sulfo-betaine;
G is a carboxyl group optionally protected in the form of an ester, an amino group optionally protected by a *tert*-butoxycarbonyl group, a succinimidyl ester, a haloacetamido group, a hydrazine group, an isothiocyanato group, or a maleimido group;
Alk₁, Alk₂, and Alk₃ each independently represent a (C₁-C₆)alkyl;
m is 0, 1, 2 or 3;
n is 0, 1, 2 or 3;
provided that the compound of formula (II) comprises at least one group of formula (IV).

5. The compound of claim 4, which is a compound of formula (II).

6. The compound of claim 5, which comprises only one group of formula (IV).

7. The compound of claim 5, which comprises two groups of formula (IV).

8. The compound of claim 5, which comprises three groups of formula (IV).

9. The compound of any of claims 5-8, wherein each R₁ is a group -PO(OH)R₅ where R₅ is a (C₁-C₄)alkyl, preferably a methyl.

10. The compound of any preceding claim, wherein R₄ is -NR₆R₇ where R₆ and R₇ are each independently a (C₁-C₄)alkyl, or R₆ and R₇, together with the nitrogen atom to which they are bonded, form a piperazine optionally N-protected by a *tert*-butoxycarbonyl group or optionally N-substituted with a group R₈.

11. The compound of any preceding claim, wherein L₁ is a direct bond or -CONH-(CH₂)ₙ-.

12. The compound of any preceding claim, wherein E is -SO₃H or -SO₂(OCH₂CF₃).

13. The compound of any preceding claim, wherein G is a carboxyl group optionally protected in the form of an ester or an amino group optionally protected by a *tert-*butoxycarbonyl group.

14. An europium complex comprising an europium(III) ion and a compound of formula (I), (II) or (III) as defined in any of claims 3-13.

15. The complex of claim 14, which has the following structure:

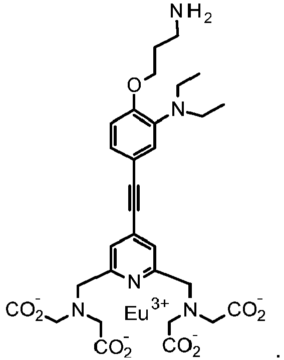

16. The complex of claim 14, which has the following structure: 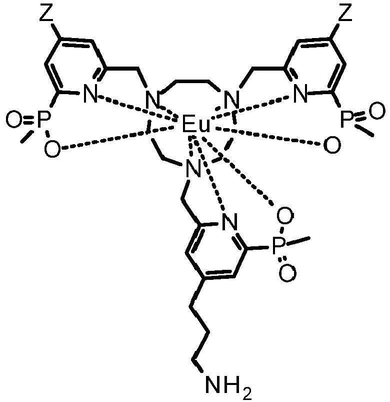 where Z is: 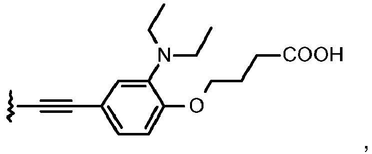 or

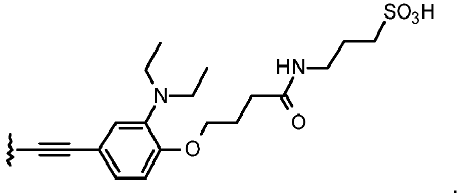

17. A conjugate obtained by reaction between (i) an europium complex as defined in any of claims 14-16, said complex bearing a group G, and (ii) a molecule of interest.

18. The conjugate of claim 17, wherein the molecule of interest is an amino acid, a peptide, a protein, an antibody, a sugar, a carbohydrate chain, a nucleoside, a nucleotide, an oligonucleotide, or an enzyme substrate.
